# EUROPÄISCHE PATENTSCHRIFT

(11) **EP 0 510 032 B1**
(45) Veröffentlichungstag und Bekanntmachung des Hinweises auf die Patenterteilung: **13.11.1996**
(21) Anmeldenummer: 91901751.7
(22) Anmeldetag: 24.12.1990
(51) Int. Cl.: C07D 401/12, C07D 401/14, C07D 417/14, C07D 213/71, A01N 47/36

(54) **PYRIDYLSULFONYLHARNSTOFFE ALS HERBIZIDE UND PFLANZENWACHSTUMSREGULATOREN, VERFAHREN ZU IHRER HERSTELLUNG UND IHRE VERWENDUNG**
USE OF PYRIDYL SULPHONYL UREAS AS HERBICIDES AND PLANT GROWTH REGULATORS, PROCESS FOR PRODUCING THEM AND THEIR USE
EMPLOI DE CARBAMIDES DE PYRIDYLSULFONYLE COMME HERBICIDES ET REGULATEURS DE CROISSANCE VEGETALE, PROCEDE DE PRODUCTION ET UTILISATION

(30) Priorität: 10.01.1990 DE 4000503; 27.09.1990 DE 4030577
(43) Veröffentlichungstag der Anmeldung: 28.10.1992
(73) Patentinhaber: Hoechst Schering AgrEvo GmbH, 13342 Berlin (DE)
(72) Erfinder: KEHNE, Heinz, D-6238 Hofheim am Taunus (DE); WILLMS, Lothar, D-5416 Hillscheid (DE); ORT, Oswald, D-6233 Kelkheim (Taunus) (DE); BAUER, Klaus, D-6470 Hanau (DE); BIERINGER, Hermann, D-6239 Eppstein/Taunus (DE)
(86) Internationale Anmeldenummer: EP9002308
(87) Internationale Veröffentlichungsnummer: WO9110660

(56) Entgegenhaltungen:
- EP-A- 0 178 101
- EP-A- 0 314 505
- WO-A-88/04297
- US-A- 4 435 206
- US-A- 4 487 626

## Beschreibung

Es ist bekannt, daß einige 2-Pyridylsulfonylharnstoffe herbizide und pflanzenwachstumsregulierende Eigenschaften besitzen; vergleiche EP-A-13 480, EP-A-272 855, EP-A-84 224, US-PS 4 421 550, EP-A-103 543 (US-A-4 579 583) US-PS 4 487 626, EP-A-125 864, WO 88/04297.

Es wurde nun gefunden, daß 2-Pyridylsulfonylharnstoffe mit speziellen Resten in 3-Position des Pyridylrestes besonders gut als Herbizide und Wachstumsregulatoren geeignet sind.

Gegenstand der vorliegenden Erfindung sind Verbindungen der Formel (I) oder deren Salze, 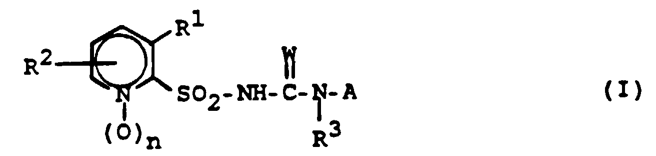 worin
- R¹: -OSO₂NR⁴R⁵, -NR⁶R⁷ oder Iod,
- R²: H, (C₁-C₄)Alkyl, vorzugsweise (C₁-C₃)Alkyl, (C₁-C₃)Haloalkyl, Halogen, NO₂, CN, (C₁-C₃)Alkoxy, (C₁-C₃)Haloalkoxy, (C₁-C₃)Alkylthio, (C₁-C₃)Alkoxy-(C₁-C₃)alkyl, (C₁-C₃)Alkoxycarbonyl, (C₁-C₃)Alkylamino, Di((C₁-C₃)alkyl)amino, (C₁-C₃)-Alkylsulfinyl, (C₁-C₃)Alkylsulfonyl, SO₂NR^{a}R^{b} oder C(O)NR^{a}R^{b},
- R^{a}, R^{b}: unabhängig voneinander H, (C₁-C₃)Alkyl, (C₃-C₄)-Alkenyl, Propargyl, oder zusammen -(CH₂)₄-, -(CH₂)₅- oder -CH₂CH₂OCH₂CH₂-
- R³: H oder CH₃,
- R⁴: H, (C₁-C₃)Alkyl, (C₃-C₄)Alkenyl, (C₁-C₃)Alkoxy oder (C₃-C₄)Alkinyl, vorzugsweise Propargyl, und
- R⁵: H, (C₁-C₃)Alkyl, (C₃-C₄)Alkenyl oder (C₃-C₄)Alkinyl, vorzugsweise Propargyl, oder
- R⁴ und R⁵: zusammen -(CH₂)₄-, -(CH₂)₅- oder -CH₂CH₂OCH₂CH₂-,
- R⁶: H, (C₁-C₈)Alkyl, das unsubstituiert oder durch ein oder mehrere Reste aus der Gruppe Halogen, (C₁-C₄)Alkoxy, (C₁-C₄)Alkylthio, (C₁-C₄)Alkylsulfinyl, (C₁-C₄)-Alkylsulfonyl, (C₁-C₄-Alkoxy)carbonyl und CN substituiert ist, (C₃-C₆)Alkenyl, das unsubstituiert oder durch ein oder mehrere Halogenatome substituiert ist, (C₃-C₆)-Alkinyl, das unsubstituiert oder durch ein oder mehrere Halogenatome substituiert ist, (C₁-C₄)Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, (C₁-C₄)Alkyl und (C₁-C₄)Alkoxy substituiert ist, (C₁-C₄)Alkoxy oder (C₁-C₄-Alkyl)carbonyl, das unsubstituiert oder durch ein oder mehrere Halogenatome substituiert ist,
- R⁷: (C₁-C₄)Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, (C₁-C₄)Alkyl und (C₁-C₄)Alkoxy substituiert ist, oder Di-[(C₁-C₄)alkyl]aminosulfonyl oder
- R⁶ und R⁷: gemeinsam eine Kette der Formel -(CH₂)ₘ-SO₂-, wobei die Kette noch durch 1 bis 4 (C₁-C₃)Alkylreste substituiert sein kann und m 3 oder 4 bedeutet,
- n: Null oder 1,
- W: O oder S,
- A: ein Rest der Formel 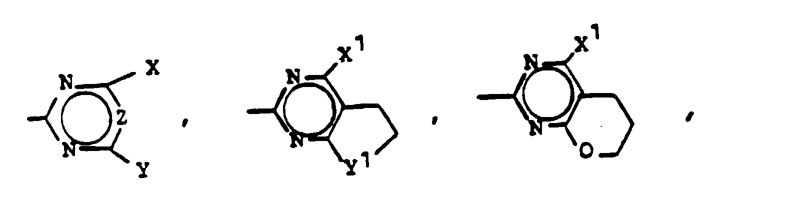 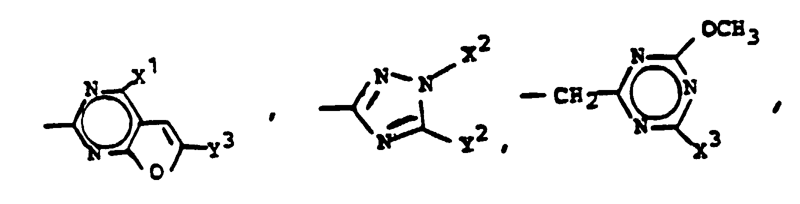 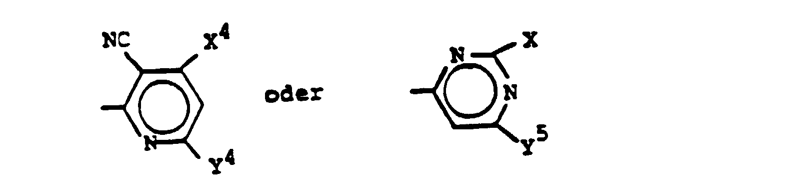
- X: H, Halogen, (C₁-C₃)Alkyl, (C₁-C₃)Alkoxy, wobei die beiden letztgenannten Reste unsubstituiert oder ein- oder mehrfach durch Halogen oder einfach durch (C₁-C₃)Alkoxy substituiert sind,
- Y: H, (C₁-C₃)Alkyl, (C₁-C₃)Alkoxy oder (C₁-C₃)Alkylthio, wobei die vorgenannten alkylhaltigen Reste unsubstituiert oder ein-oder mehrfach durch Halogen oder ein- oder zweifach durch (C₁-C₃)Alkoxy oder (C₁-C₃)Alkylthio substituiert sind, ferner einen Rest der Formel NR⁸R⁹, (C₃-C₆)-Cycloalkyl, (C₂-C₄)Alkenyl, (C₂-C₄)Alkinyl, (C₃-C₄)-Alkenyloxy oder (C₃-C₄)Alkinyloxy,
- Z: CH oder N,
- R⁸ und R⁹: unabhängig voneinander H, (C₁-C₃)Alkyl oder (C₃-C₄)Alkenyl,
- X¹: CH₃, OCH₃, OC₂H₅ oder OCF₂H,
- Y¹: -O- oder -CH₂-,
- X²: CH₃, C₂H₅ oder CH₂CF₃,
- Y²: OCH₃, OC₂H₃, SCH₅, SC₂H₅, CH₃ oder C₂H₅,
- X³: CH₃ oder OCH₃,
- Y³: H oder CH₃,
- X⁴: CH₃, OCH₃, OC₂H₅, CH₂OC₃ oder Cl,
- Y⁴: CH₃, OCH₃, OC₂H₅ oder Cl,
- Y⁵: CH₃, C₂H₅, OCH₃ oder Cl
bedeuten.

In der Formel (I) können Alkyl-, Alkoxy-, Haloalkyl-, Alkylamino- und Alkylthioreste sowie die entsprechenden ungesättigten und/oder substituierten Reste jeweils geradkettig oder verzweigt sein. Alkylreste, auch in den zusammengesetzten Bedeutungen wie Alkoxy, Haloalkyl usw., bedeuten Methyl, Ethyl, n- oder i-Propyl, Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metall-, insbesondere Alkali- oder Erdalkalisalze, oder auch Ammoniumsalze oder Salze mit organischen Aminen. Ebenso kann Salzbildung durch Anlagerung einer starken Säure, an den Pyridinteil der Verbindung der Formel (I) erfolgen. Geeignete Säuren hierfür sind HCl, HBr, H₂SO₄ oder HNO₃.

Bevorzugte Verbindungen der Formel (I) oder deren Salze sind solche, bei denen n= Null, W= O und A einen Rest der Formel 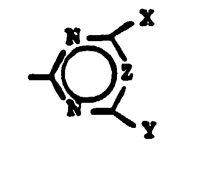 bedeuten worin X, Y und Z wie oben beschrieben, definiert sind.

Bevorzugte Verbindungen der Formel I oder deren Salze sind auch solche, in denen
- R², R^{a}, R^{b}, n, W und A: wie oben definiert sind und
- R⁴, R⁵: unabhängig voneinander (C₁-C₃)Alkyl, Allyl oder Propargyl oder
- R⁴, R⁵: zusammen -(CH₂)₄- , -(CH₂)₅- oder -CH₂CH₂OCH₂CH₂-,
- R⁶: H, (C₁-C₄)Alkyl, das unsubstituiert oder durch ein oder mehrere Halogenatome oder durch einen Rest aus der Gruppe (C₁-C₃)Alkoxy, (C₁-C₃)Alkylthio, (C₁-C₃)-Alkylsulfonyl, (C₁-C₄)Alkoxycarbonyl und CN substituiert ist, (C₃-C₄)Alkenyl, (C₃-C₄)Alkinyl, (C₁-C₄)-Alkylsulfonyl, Phenylsulfonyl, Phenylsulfonyl, das durch ein bis drei Reste aus der Gruppe Halogen, (C₁-C₃)Alkyl und (C₁-C₃)Alkoxy substituiert ist, (C₁-C₃)Alkoxy oder (C₁-C₄)Alkylcarbonyl,
- R⁷: (C₁-C₄)Alkylsulfonyl, Phenylsulfonyl oder Phenylsulfonyl, das durch 1 bis 3 Reste aus der Gruppe Halogen, (C₁-C₃)Alkyl und (C₁-C₃)Alkoxy substituiert ist, oder Di-(C₁-C₄-alkyl)-aminosulfonyl oder
- R⁶ und R⁷: gemeinsam eine Kette der Formel -(CH₂)ₘSO₂-, wobei m 3 oder 4 bedeutet,
bedeuten.

Besonders bevorzugte Verbindungen der Formel (I) oder deren Salze sind solche, worin R²= H, (C₁-C₃)Alkyl, (C₁-C₃)Alkoxy, Halogen oder (C₁-C₃)Alkylthio, R⁴ und R⁵ unabhängig voneinander (C₁-C₃)Alkyl, R⁶ Wasserstoff, ,(C₁-C₄)Alkyl oder (C₁-C₃)Alkylsulfonyl, R⁷ (C₁-C₃)Alkylsulfonyl und A einen Rest der Formel 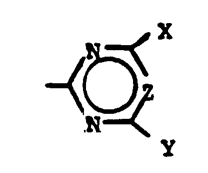 bedeuten,
worin Z CH oder N, X Halogen, (C₁-C₂)Alkyl, (C₁-C₂)Alkoxy, OCF₂H, CF₃ oder OCH₂CF₃ und Y (C₁-C₂)Alkyl, (C₁-C₂)Alkoxy oder OCF₂H sind, und insbesondere die vorstehend definierten Verbindungen, in denen n = Null und W ein Sauerstoffatom bedeuten.

Weiterer Gegenstand der vorliegenden Erfindung sind Verfahren zur Herstellung der Verbindungen der allgemeinen Formel (I) oder deren Salze, dadurch gekennzeichnet, daß man
(a) eine Verbindung der Formel (II) 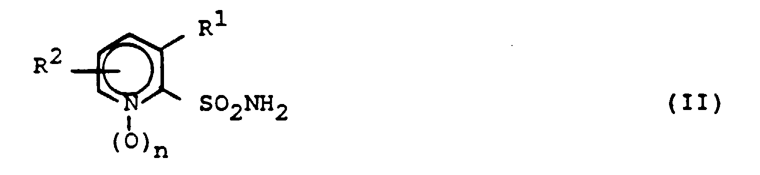 mit einem heterocyclischen Carbamat der Formel (III) 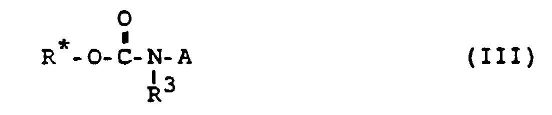 worin R* Phenyl oder (C₁-C₄)Alkyl bedeutet,
   umsetzt oder
(b) ein Pyridylsulfonylcarbamat der Formel (IV) 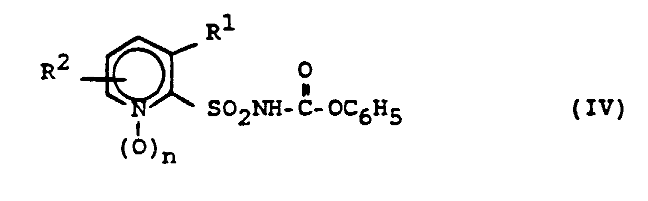 mit einem Aminoheterocyclus der Formel (V) 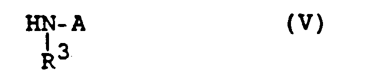 umsetzt oder
(c) ein Sulfonylisocyanat der Formel (VI) 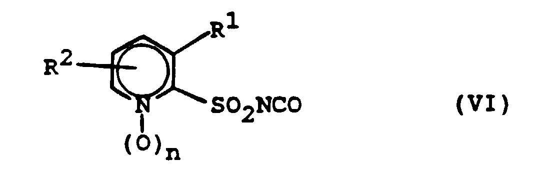 mit einem Aminoheterocyclus der Formel R³-NH-A (V) umsetzt oder
(d) in einer Eintopfreaktion zunächst einen Aminoheterocyclus der Formel R³-NH-A (V) in Gegenwart einer Base, wie z.B. Triethylamin, mit Phosgen umsetzt und das gebildete Intermediat mit einem Pyridinsulfonamid der Formel (II) umsetzt (z.B. analog EP-A-232 067).

Die Umsetzung der Verbindungen der Formeln (II) und (III) erfolgt vorzugsweise basenkatalysiert in einem inerten organischen Lösungsmittel, wie z.B. Dichlormethan, Acetonitril, Dioxan oder THF bei Temperaturen zwischen 0°C und dem Siedepunkt des Lösungsmittels. Als Base wird bevorzugt 1,8-Diazabicyclo[5.4.0]undec-7-en (DBU) oder Trimethylaluminium oder Triethylaluminium verwendet.

Die Sulfonamide (II) sind neue Verbindungen. Sie und ihre Herstellung sind ebenfalls Gegenstand dieser Erfindung. Man erhält sie ausgehend von geeignet substituierten 2-Halogenpyridinen, die man mit S-Nucleophilen wie z.B. Benzylmercaptan oder Thioharnstoff umsetzt. Die so gebildeten Verbindungen überführt man mit Natriumhypochlorit oder Chlor in die Sulfochloride (analog EP-A-272 855), die dann entweder direkt mit Ammoniak oder mit tert.-Butylamin über die tert.-Butylamide mit anschließender Schutzgruppenabspaltung zu den Sulfonamiden der Formel (II) abreagieren.

Die Carbamate der Formel (III) können nach Methoden hergestellt werden, die in den südafrikanischen Patentanmeldungen 82/5671 und 82/5045 bzw. EP-A-70804 (US-A-4 480 101) oder RD 275056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Pyridylsulfonylcarbamate der Formel (IV) erhält man analog EP-A-44 808 oder EP-A-237 292.

Die Pyridylsulfonylisocyanate der Formel (VI) lassen sich analog EP-A-184 385 herstellen und mit den Aminoheterocyclen (V) umsetzen.

Die Salze der Verbindungen der Formel (I) werden vorzugsweise in inerten Lösungsmitteln wie z.B. Wasser, Methanol oder Aceton bei Temperaturen von 0 - 100°C hergestellt. Geeignete Basen zur Herstellung der erfindungsgemäßen Salze sind beispielsweise Alkalicarbonate, wie Kaliumcarbonat, Alkali-und Erdalkalihydroxide, Ammoniak oder Ethanolamin. Als Säuren zur Salzbildung eignen sich besonders HCl, HBr, H₂SO₄ oder HNO₃.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B.. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche apoliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstums stadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; Wade van Valkenburg, "Pesticide Formulations", Marcel Dekker N.Y., 1973 ; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werder beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y.; C. Marsden, "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxyethylierte Alkylphenole, polyoxethylierte Fettalkohole und Fettamine, Fettalkoholpolyglykolethersulfate, Alkansulfonate oder Alkylbenzolsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitanfettsäureester oder Polyoxethylensorbitester. Stäubemittel erhält man durch Vermahlen des Wirkstoffes mit fein verteilten festen Stoffen, z.B. Talkum, natürlichen Tonen wie Kaolin, Bentonit und Pyrophyllit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 85 Gew.-%, meistens 5 bis 80 Gew.-% betragen. Staubförmige Formulierungen enthalten etwa 1 bis 25 Gew.-%, meistens 5 bis 20 Gew.-% an Wirkstoff, versprühbare Lösungen etwa 0,2 bis 20 Gew.-%, meistens 2 bis 20 Gew.-% Wirkstoff. Bei Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate zur Boden- bzw. Streuapplikation sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Auch Mischungen oder Mischformulierungen mit anderen Wirkstoffen, wie z.B. Insektiziden, Akariziden, Herbiziden, Safenern, Düngemitteln, Wachstumsregulatoren oder Fungiziden sind gegebenenfalls möglich.

### A. Chemische Beispiele

### Beispiel 1

### 2-Benzylthio-3-iodpyridin

Eine Lösung von 34,0 g (0,15 mol) 2-Fluor-3-iodpyridin und 18,6 g (0,15 mol) Benzylmercaptan in 250 ml Acetonitril wird mit 22,8 g (0,165 mol) Kaliumcarbonat 8 h unter Rückfluß erhitzt. Man kühlt ab, entfernt das Lösungsmittel am Rotationsverdampfer, nimmt den Rückstand in Dichlormethan auf und wäscht die organische Phase mit Wasser. Nach Trocknen mit Natriumsulfat, Eindampfen und Vakuumdestillation des öligen Rückstands erhält man 37,3 g (76 % d.Th.) 2-Benzylthio-3-iodpyridin vom Siedepunkt 150-153°C bei 0,1 mbar.

### Beispiel 2

### 3-Iod-2-pyridinsulfonamid

Zu einem Gemisch von 25,0 g (76,5 mmol) 2-Benzylthio-3-iodpyridin, 125 ml Dichlormethan, 60 ml Wasser und 38 ml konzentrierter Salzsäure tropft man bei 0°C 510 ml (0,34 mol) einer 5 %igen Natriumhypochloritlösung. Man rührt 30 min bei 0°C nach, extrahiert 3x mit je 100 ml Dichlormethan und trocknet die organische Phase mit Natriumsulfat. Die so erhaltene Lösung wird auf -20°C abgekühlt. Man leitet bei dieser Temperatur 6,8 g (0,4 mol) Ammoniak innerhalb 20 min ein, rührt 2 h bei -20°C nach und läßt auf Raumtemperatur kommen. Das Reaktionsgemisch wird mit Wasser gewaschen, die organische Phase getrocknet und eingedampft. Verreiben des Rückstands mit Diisopropylether ergibt 15,5 g (71 % d.Th.) 3-Iod-2-pyridinsulfonamid vom Schmp. 247-250°C (Zers.)

### Beispiel 3

### 3-(4,6-Dimethoxypyrimidin-2-yl)-1-(3-iod-2-pyridylsulfonyl)-harnstoff

Zu einer Suspension von 2,1 g (7,4 mmol) 3-Iod-2-pyridinsulfonamid und 2,2 g (8,1 mmol) N-(4,6-Dimethoxypyrimidin-2-yl)-phenylcarbamat in 30 ml Acetonitril gibt man 1,2 g (0,081 mol) 1,8-Diazabicyclo[5.4.0]undec-7-en (DBU). Die resultierende Lösung rührt man 45 min bei Raumtemperatur nach und gibt anschließend 20 ml Wasser zu. Man säuert mit Salzsäure auf pH 4 an und saugt das ausgefallene Produkt ab. Man erhält 3,2 g (93 % d.Th.) 3-(4,6-Dimethoxypyrimidin-2-yl)-1-(3-iod-2-pyridylsulfonyl)-harnstoff vom Schmp. 161 - 162°C (Zers.).

### Beispiel 4

### 3-Dimethylsulfamoyloxy-2-pyridinsulfonamid

Zu einem Gemisch von 5,7 g (17,6 mmol) 2-Benzylthio-3-dimethylsulfamoyloxypyridin, 30 ml Dichlormethan, 15 ml Wasser und 8,5 ml konzentrierter Salzsäure tropft man bei 0°C 107 ml (72 mmol) einer 5 %igen Natriumhypochloritlösung. Man rührt 30 min bei 0°C nach, extrahiert 3x mit je 20 ml Dichlormethan und trocknet die organische Phase mit Natriumsulfat. Die so erhaltene Lösung wird auf -70°C abgekühlt. Man leitet bei dieser Temperatur Ammoniak ein bis das Reaktionsgemisch deutlich alkalisch reagiert. Nach 3-stündigem Rühren bei -70°C läßt man auf Raumtemperatur kommen und wäscht mit Wasser. Die organische Phase wird getrocknet und eingedampft. Man erhält 3,0 g (61 % d.Th.) 3-Dimethylsulfamoyloxy-2-pyridinsulfonamid;
- NMR (CDCl₃): δ (ppm) =: 3,06 (s, 6H, N(CH₃)₂), 5,80 (s, 2H, NH₂) 7,48 (dd, 1H), 7,98 (dd, 1H), 8,38 (dd, 1H).

### Beispiel 5

### 3-(4,6-Dimethoxypyrimidin-2-yl)-1-(3-dimethylsulfamoyloxy-2-pyridylsulfonyl)-harnstoff

Zu einer Suspension von 3,09(10,6 mmol) 3-Dimethylsulfamoyloxy-2-pyridinsulfonamid und 3,4 g (12,7 mmol) N-(4,6-Dimethoxypyrimidin-2-yl)-phenylcarbamat in 40 ml Acetonitril gibt man 1,9 g (12,7 mmol)1,8-Diazabicyclo-[5.4.0]undec-7-en (DBU). Die resultierende Lösung rührt man 1 h bei Raumtemperatur nach und gibt anschließend 30 ml Wasser zu. Man säuert mit Salzsäure auf pH 4 an und saugt das ausgefallene Produkt ab. Nach Verreiben mit Diethylether erhält man 2,1 g (42 % d.Th.) 3-(4,6-Dimethoxypyrimidin-2-yl)-1-(3-dimethylsulfamoyloxy-2-pyridylsulfonyl)-harnstoff vom Schmp. 155-157°C.

### Beispiel 6

### 3-(4,6-Dimethoxy-1,3,5-triazin-2-yl)-1-(3-iod-2-pyridylsulfonyl)-harnstoff

Zu 4,3 g (15 mmol) 3-Iod-2-pyridinsulfonamid in 150 ml Dichlormethan tropft man bei Raumtemperatur 9,0 ml (18 mmol) einer 2M Lösung von Trimethylaluminium in Toluol. Nach Beendigung der Gasentwicklung wird 3,85g(18 mmol) Methyl-4,6-dimethoxy-1,3,5-triazin-2-ylcarbamat in 20 ml Dichlormethan zugetropft und die resultierende Lösung 24 Stunden zum Rückfluß erhitzt. Man kühlt ab und gießt in 150 ml eiskalte 1N Salzsäure. Die organische Phase wird abgetrennt und die Wasserphase 2x mit Dichlormethan extrahiert. Man trocknet die organische Phase und dampft ein. Nach Verreiben des Rohproduktes mit Diethylether erhält man 3,1 g (44 % d.Th.) 3-(4,6-Dimethoxy-1,3,5-triazin-2-yl)-1-(3-iod-2-pyridylsulfonyl)-harnstoff vom Schmp. 155°C (Zers.)

Die Verbindungen der nachfolgenden Tabellen 1 bis 4 werden analog zu den Verfahren der Beispiele 1-6 erhalten.

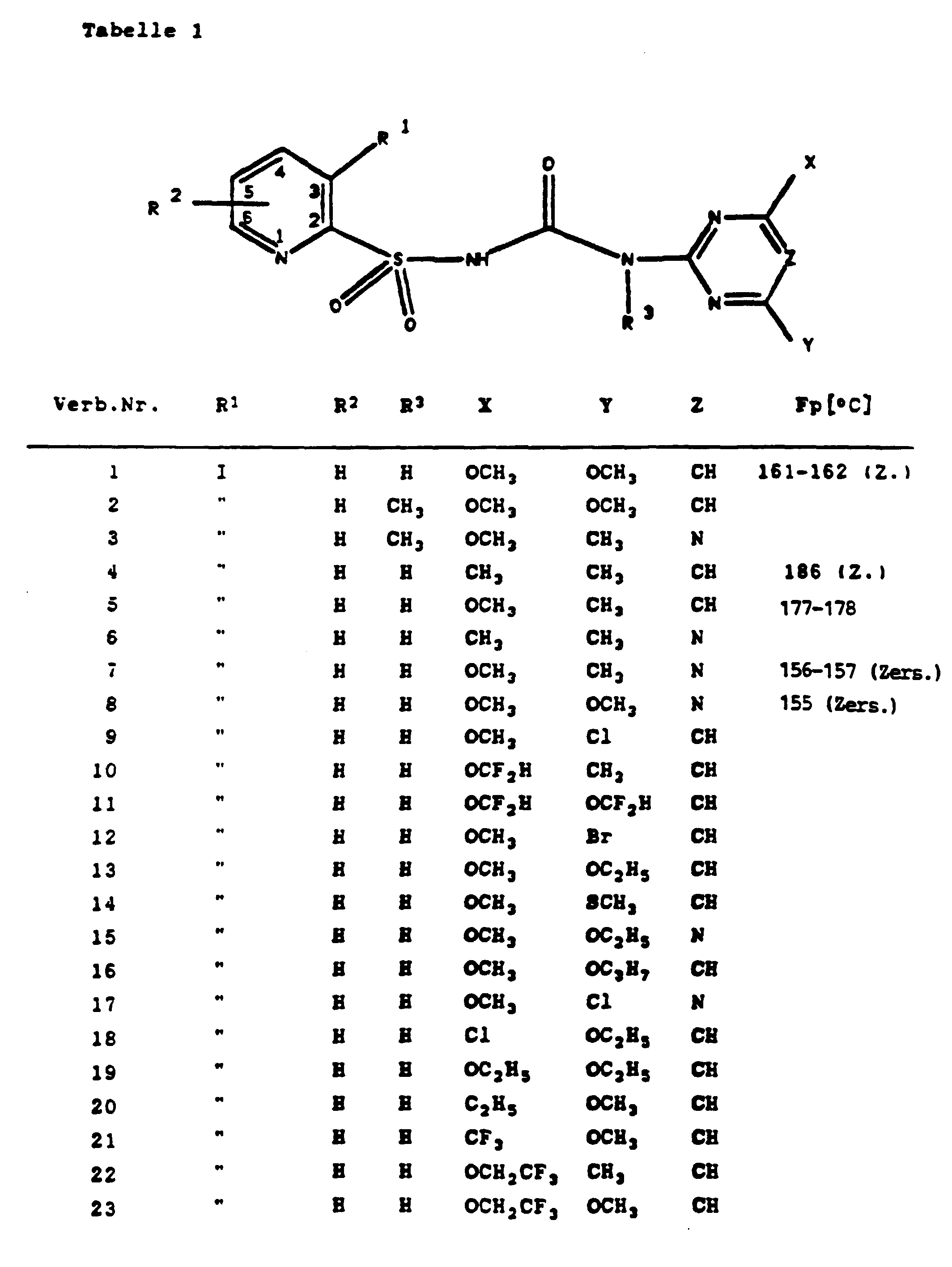

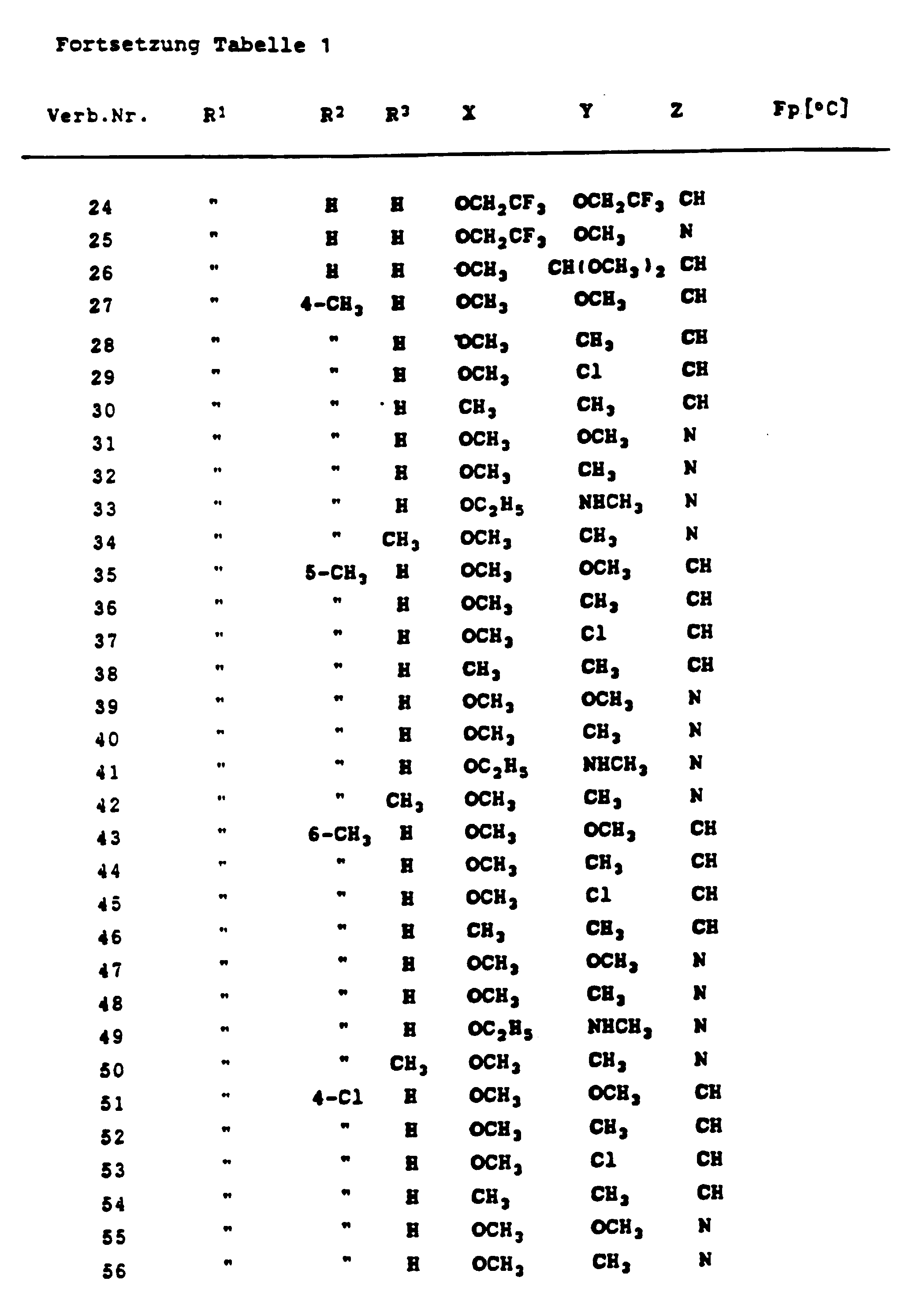

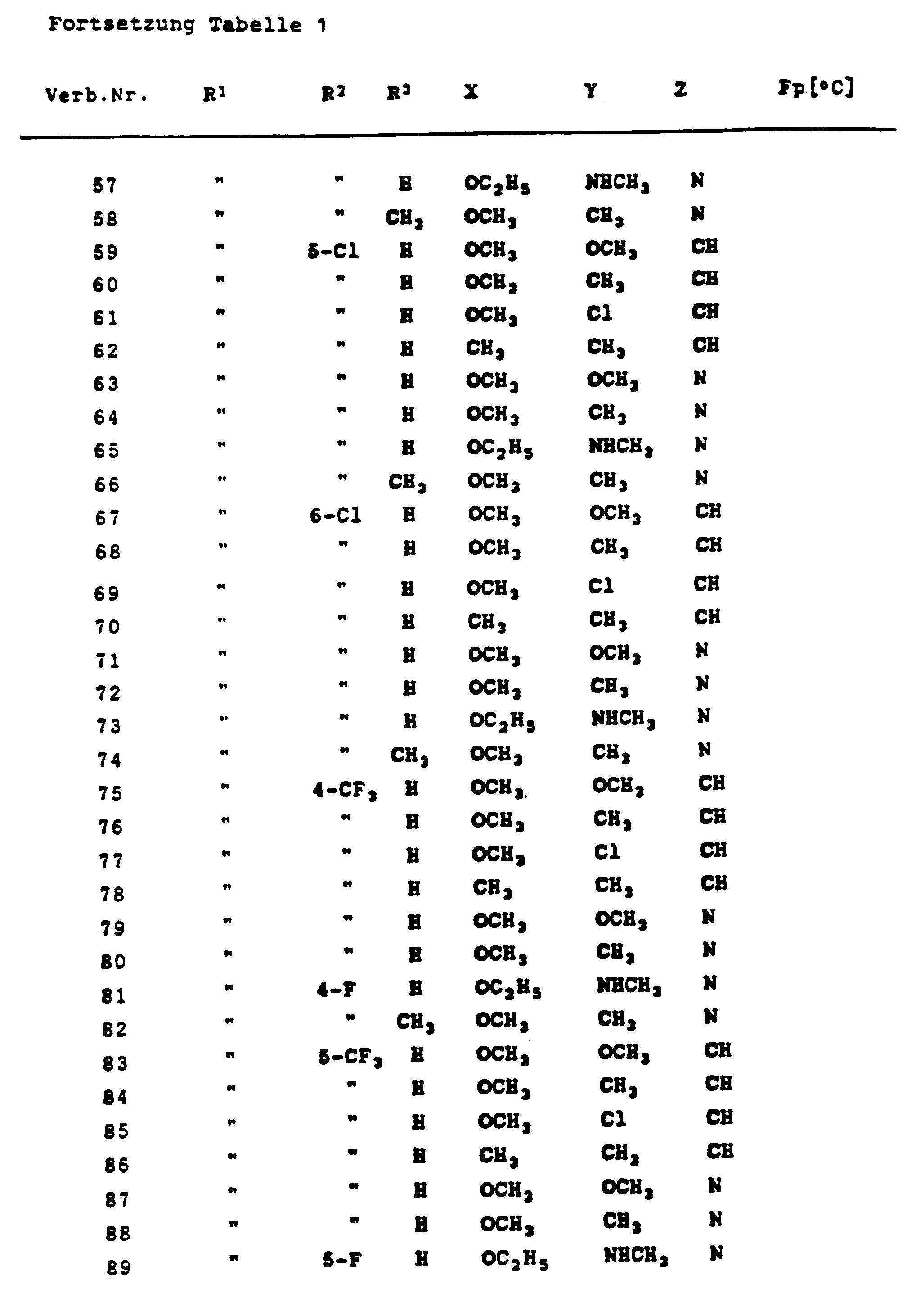

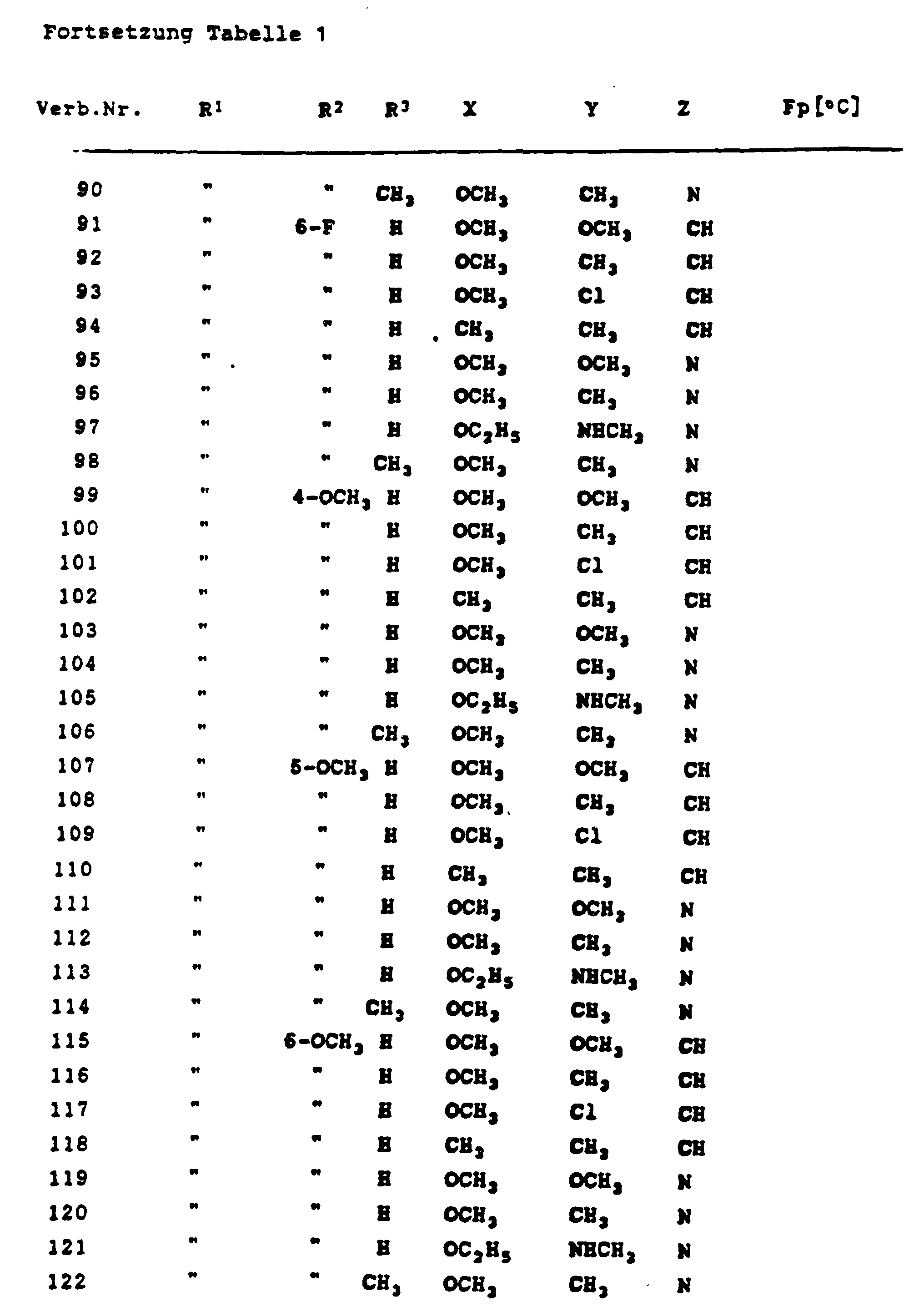

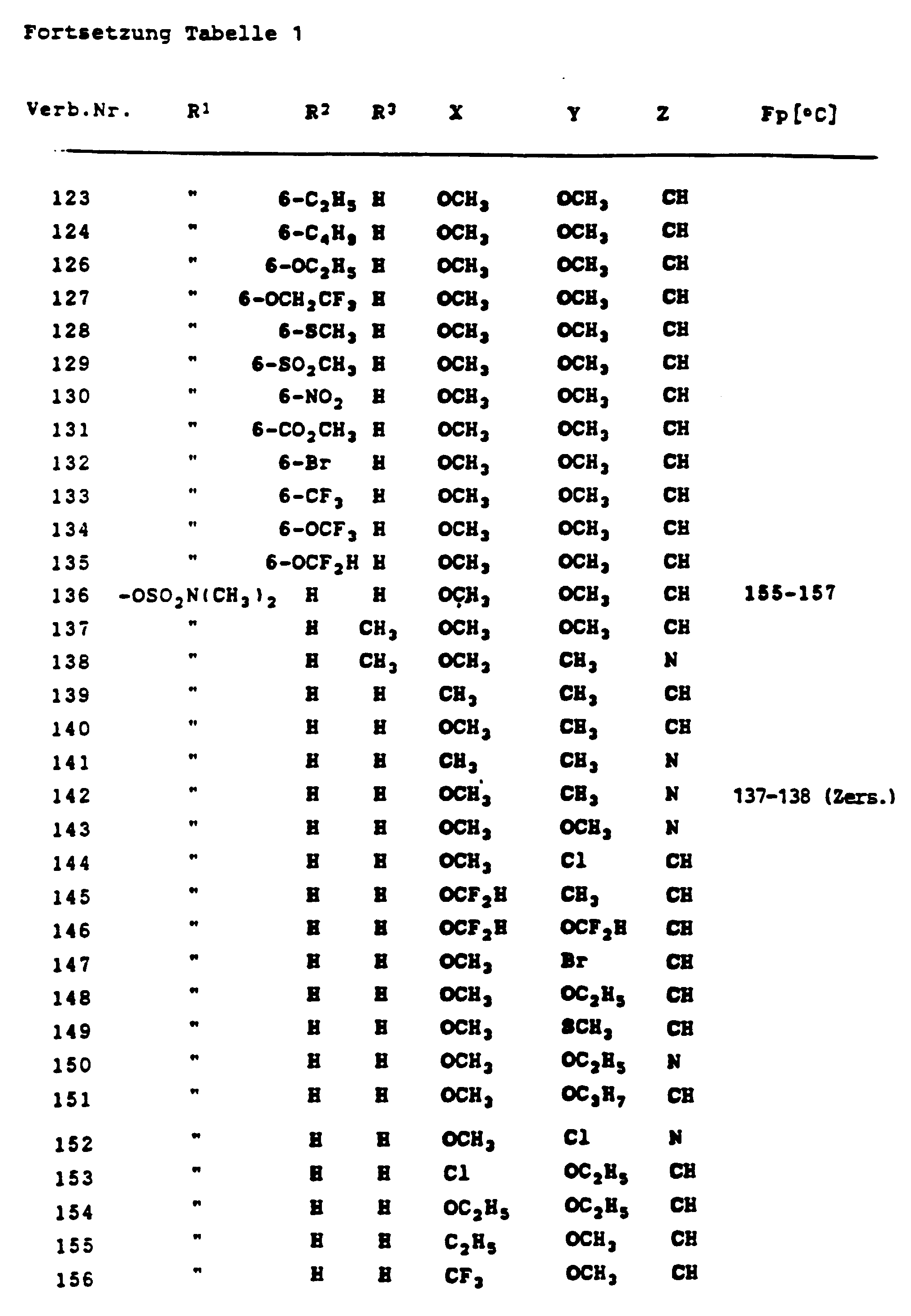

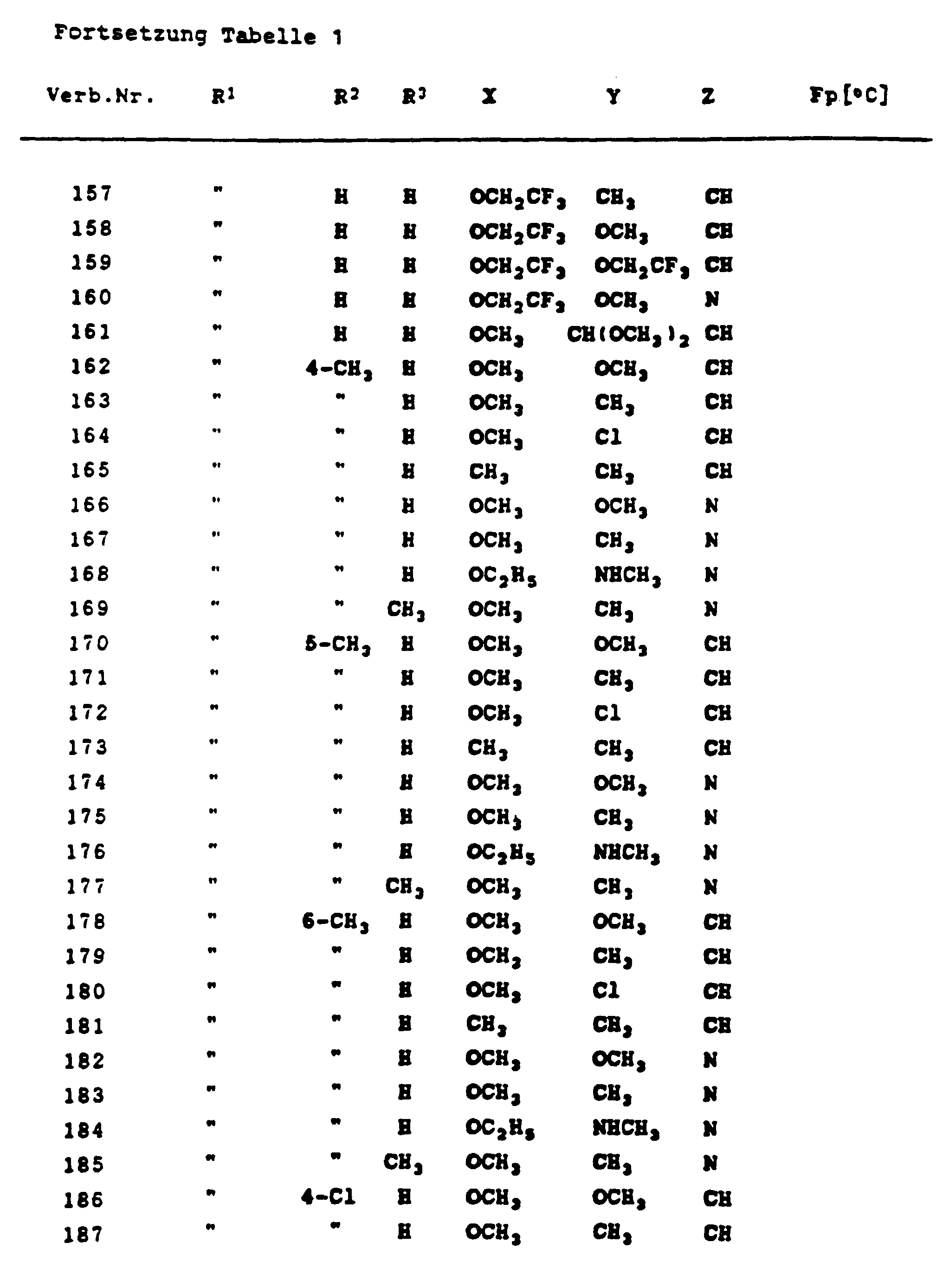

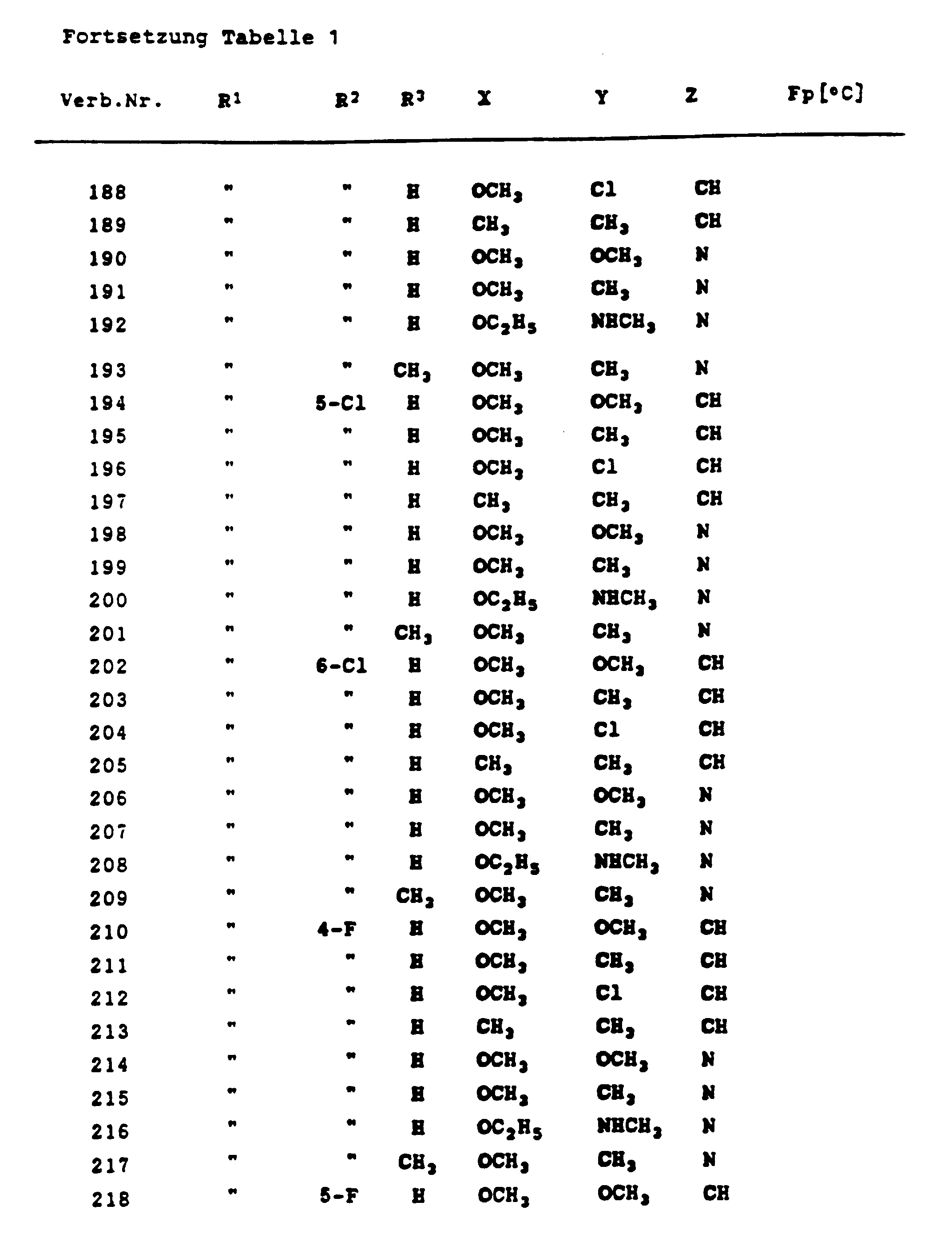

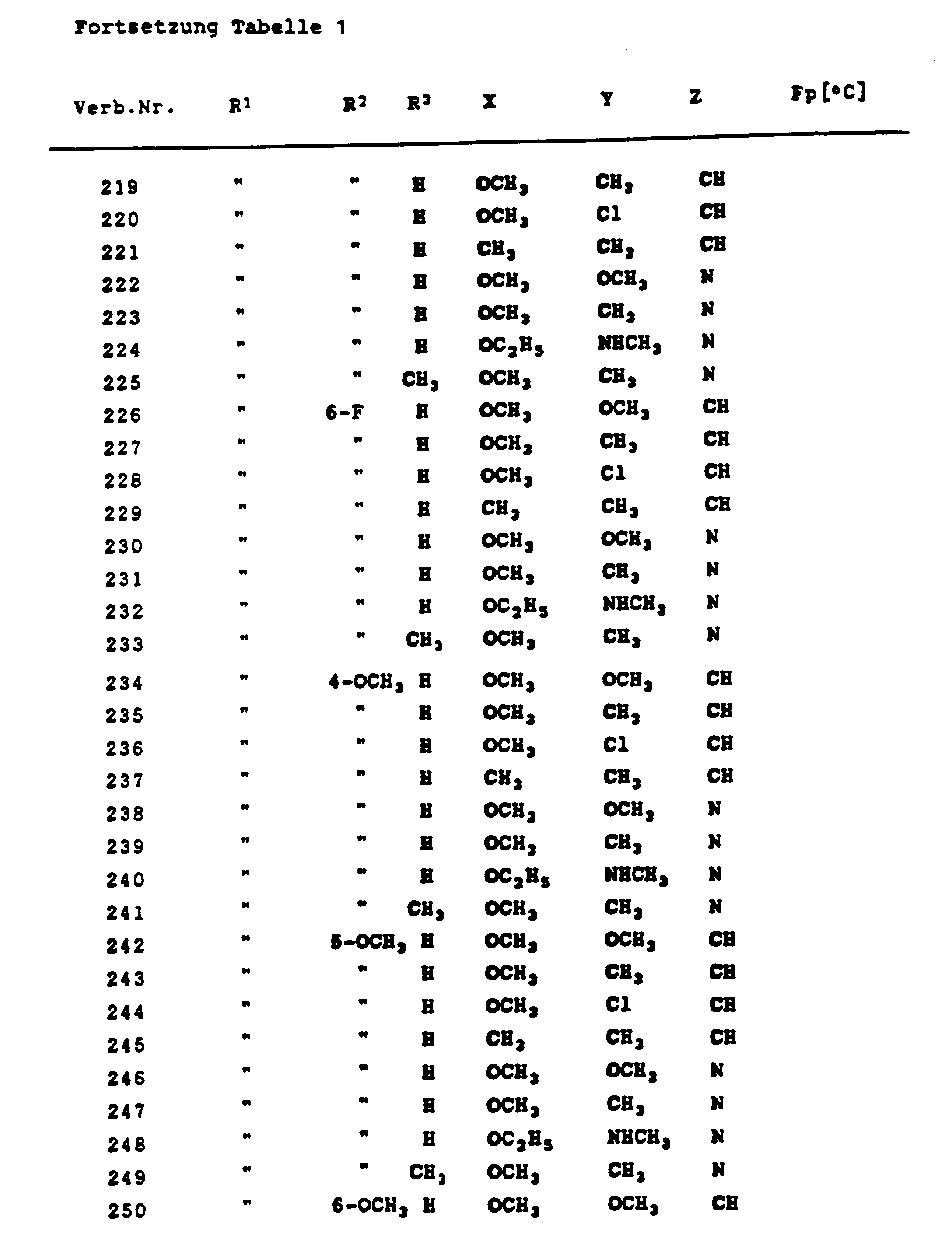

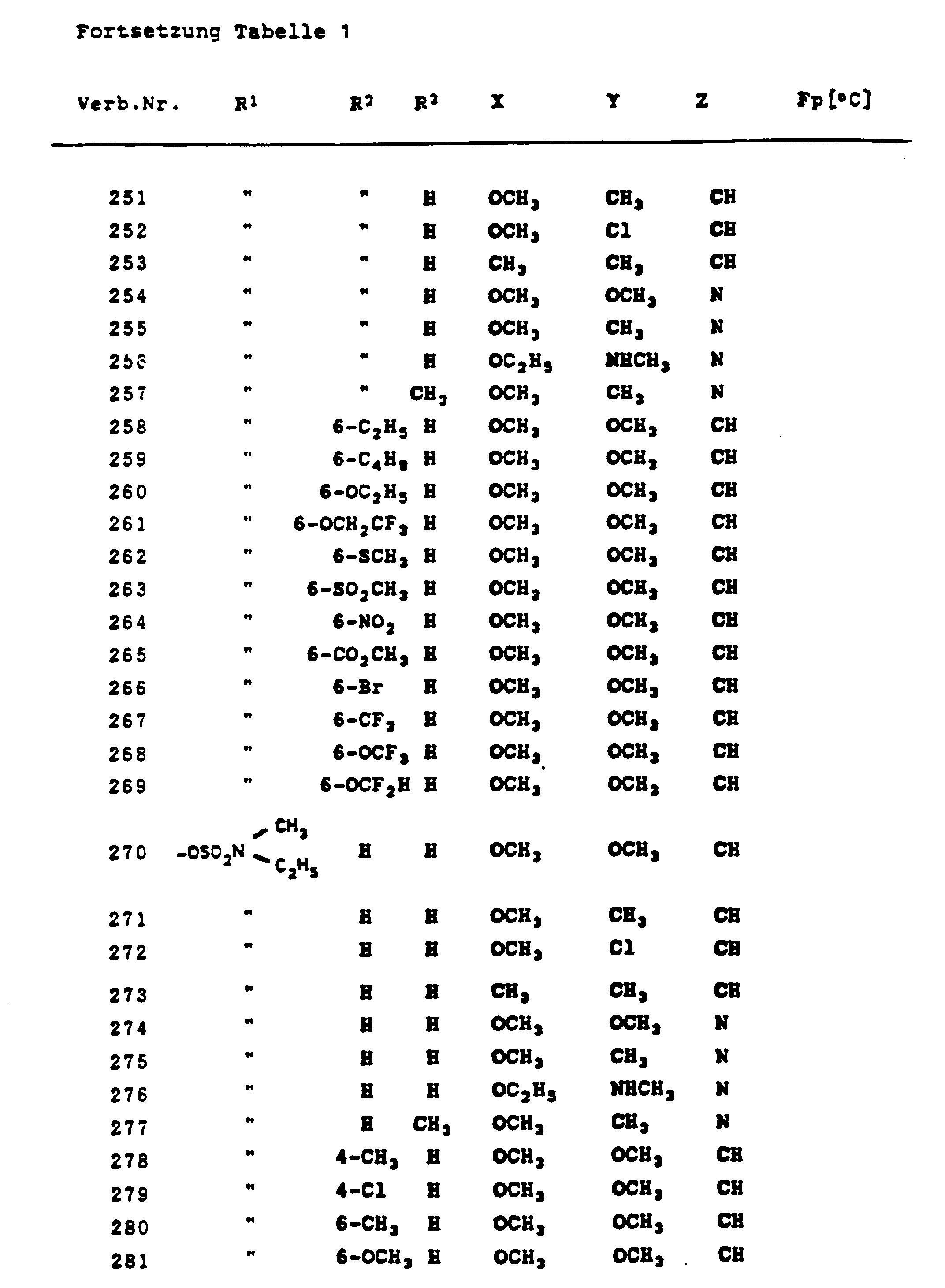

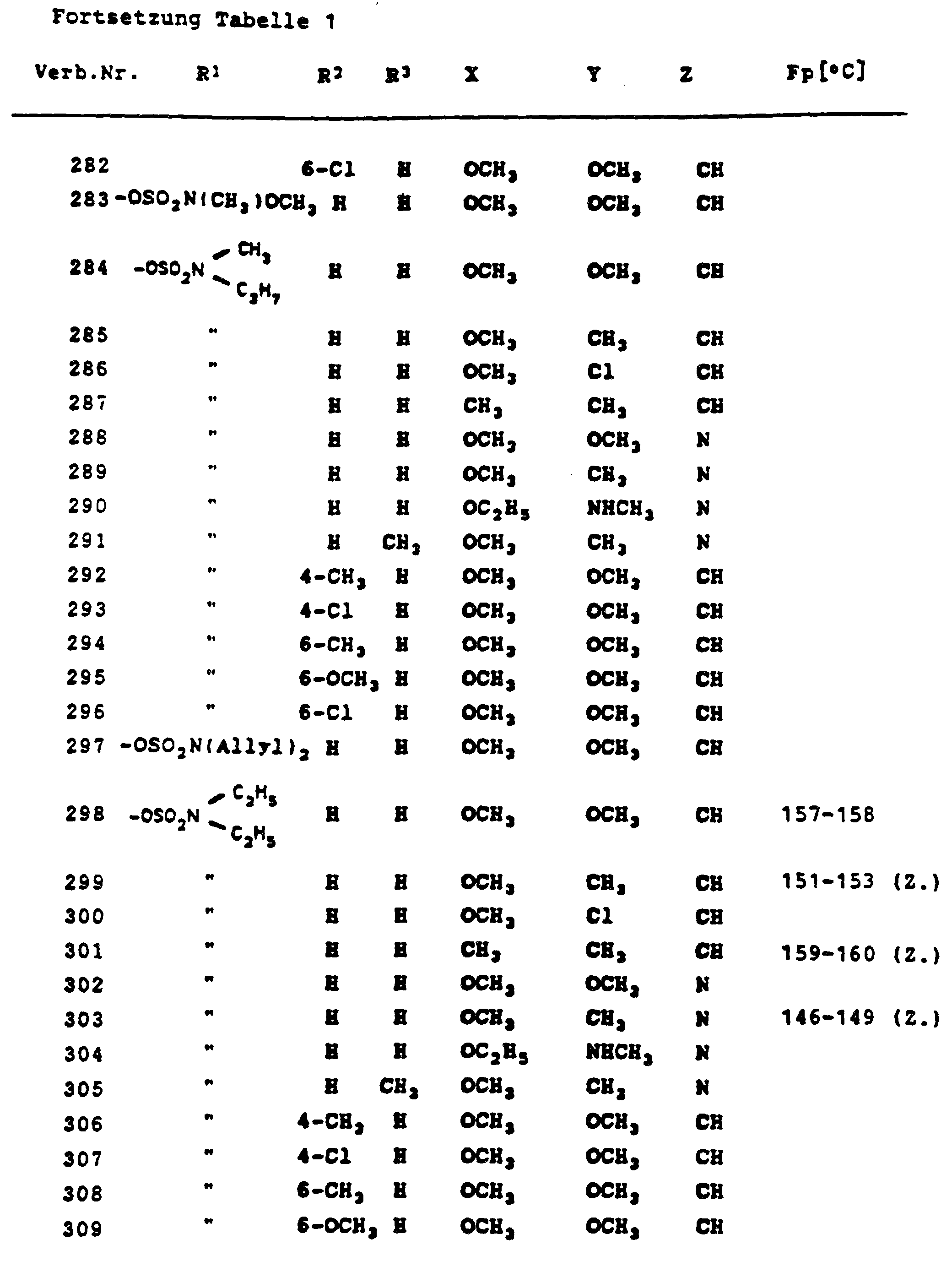

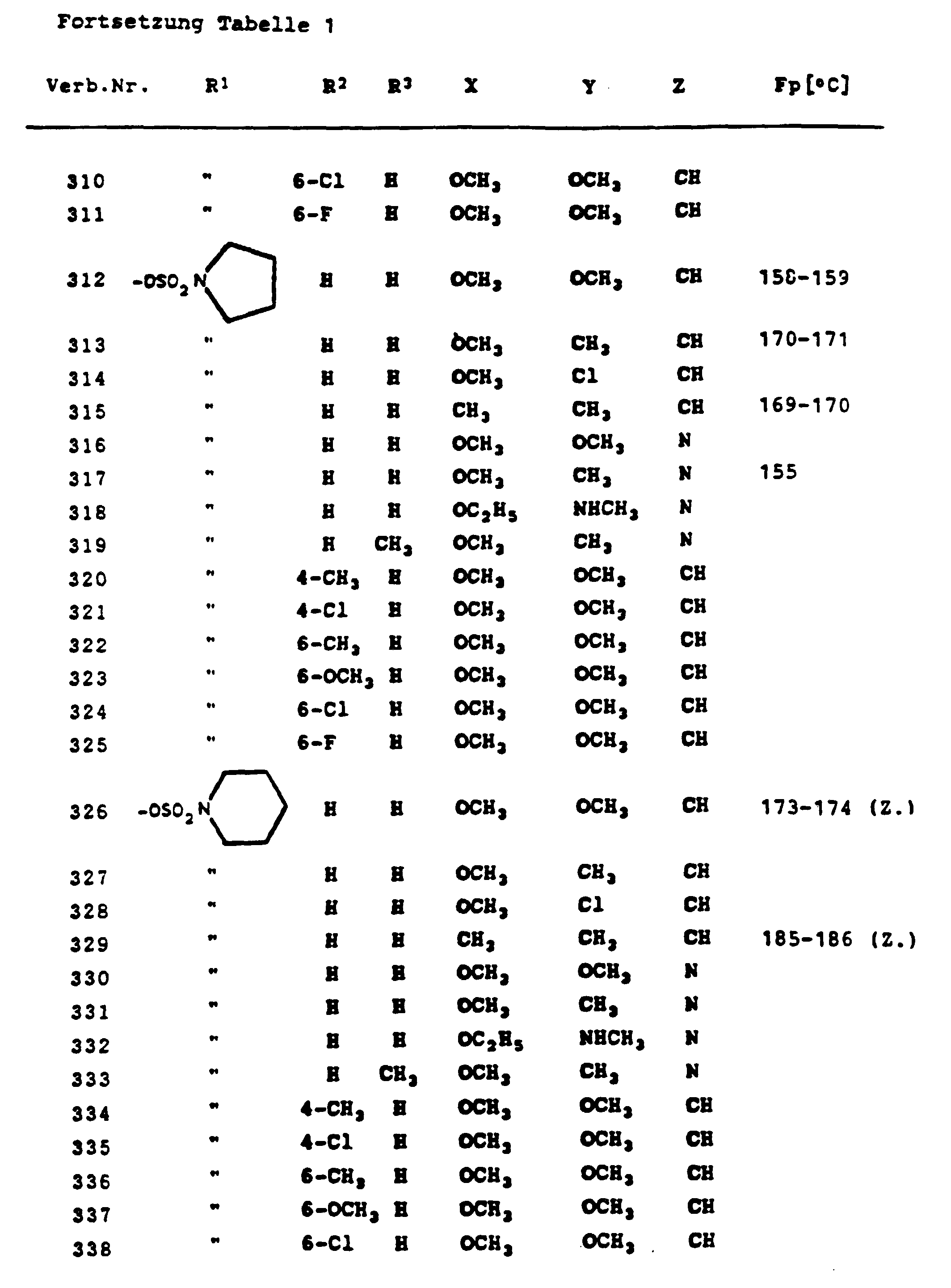

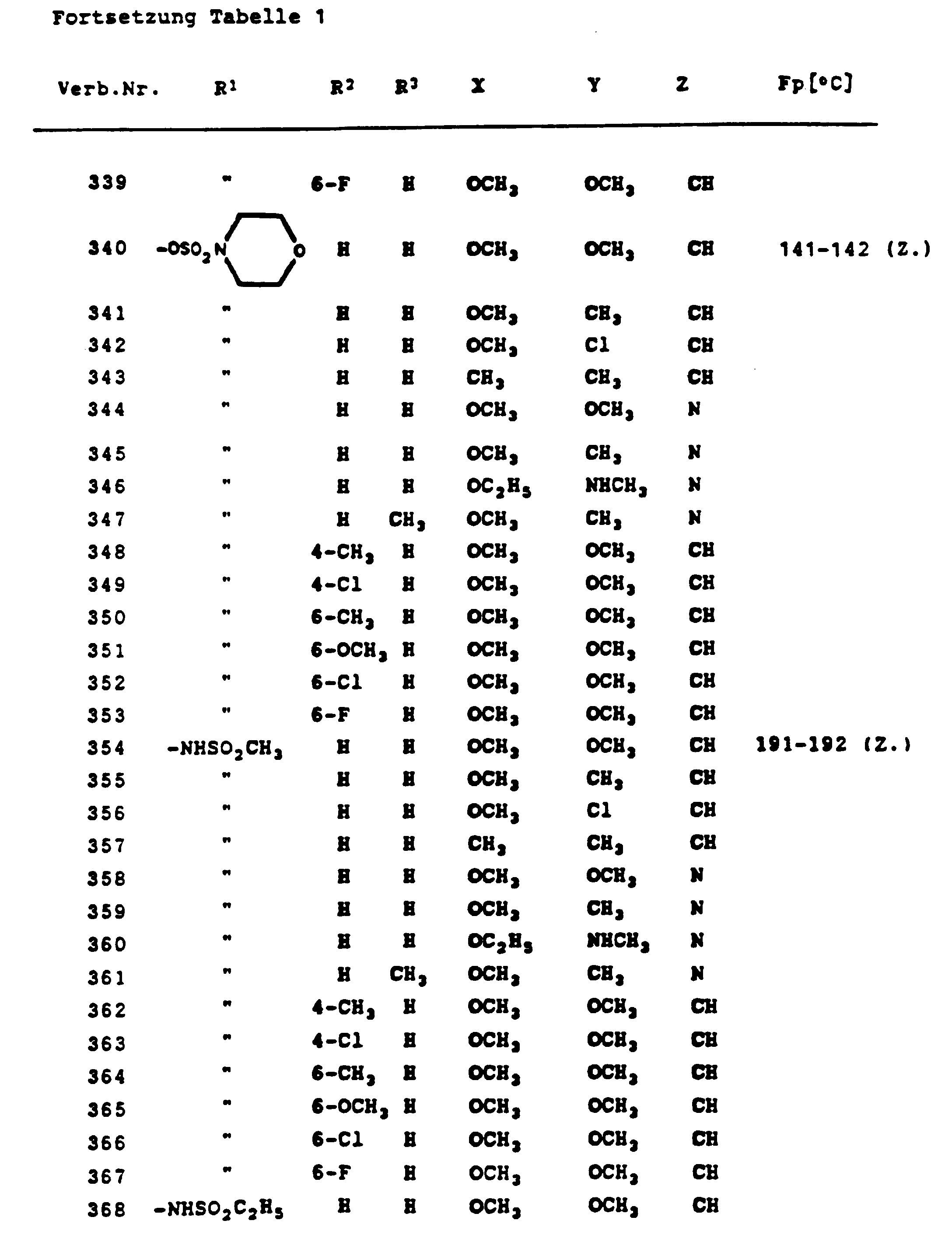

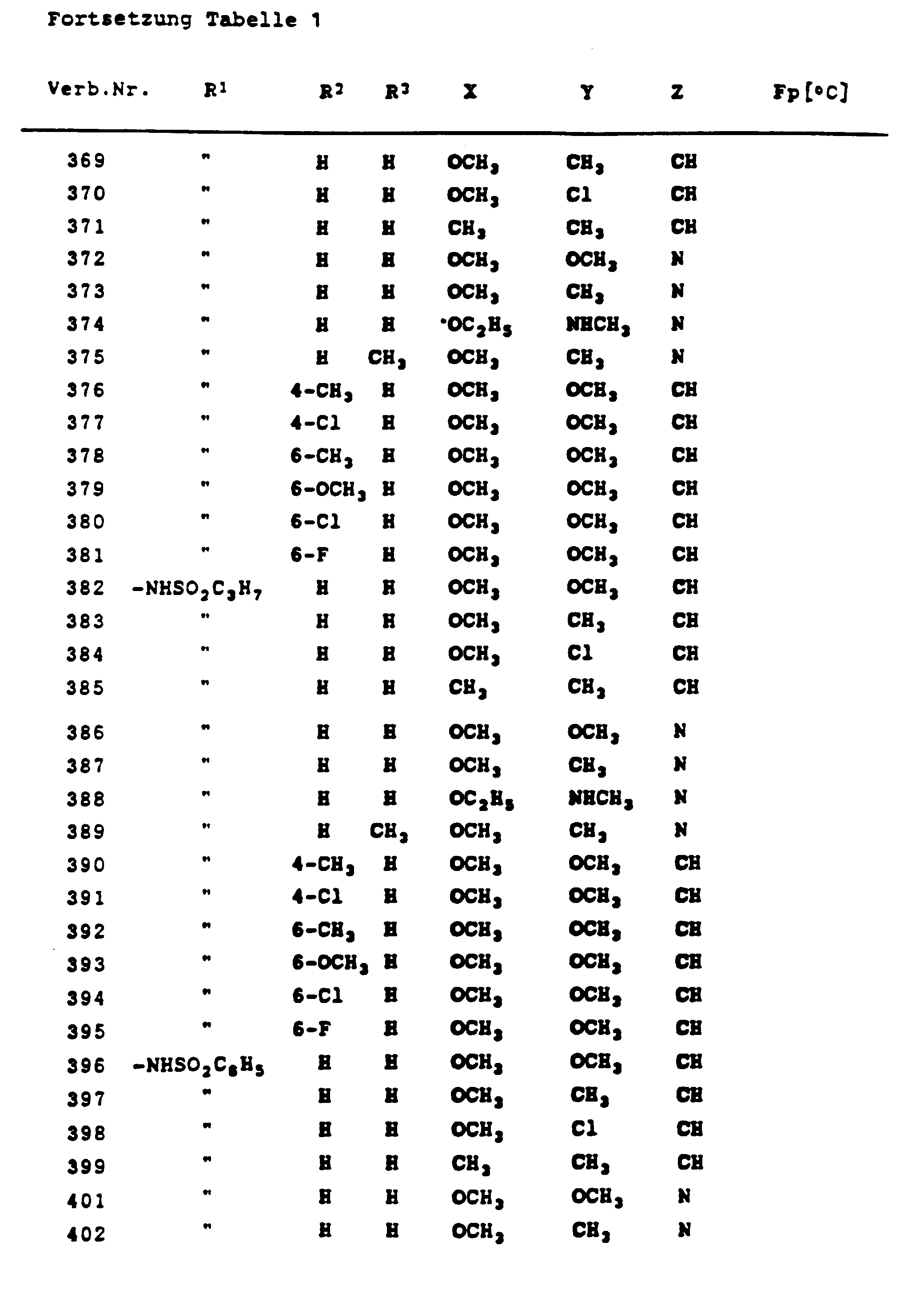

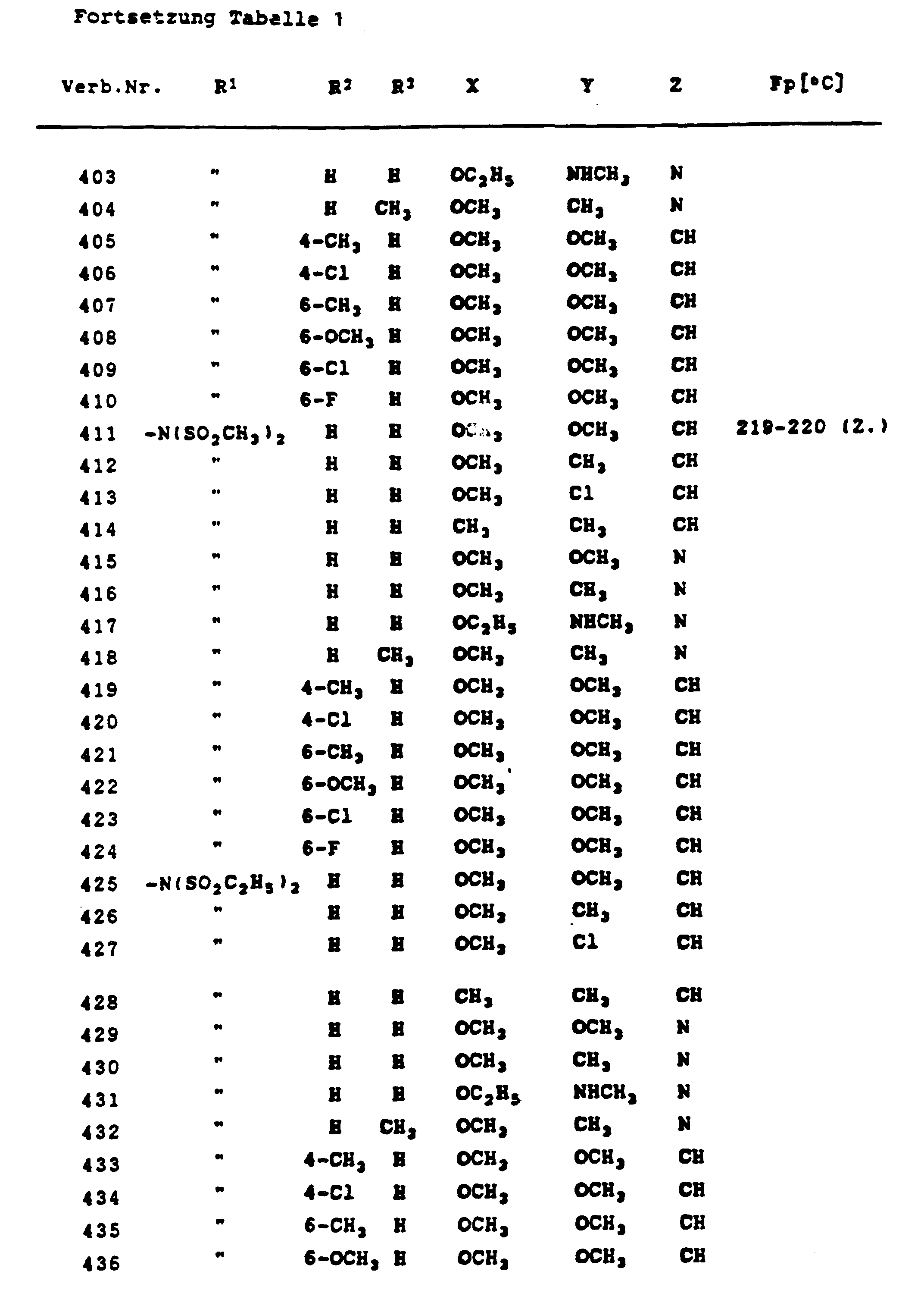

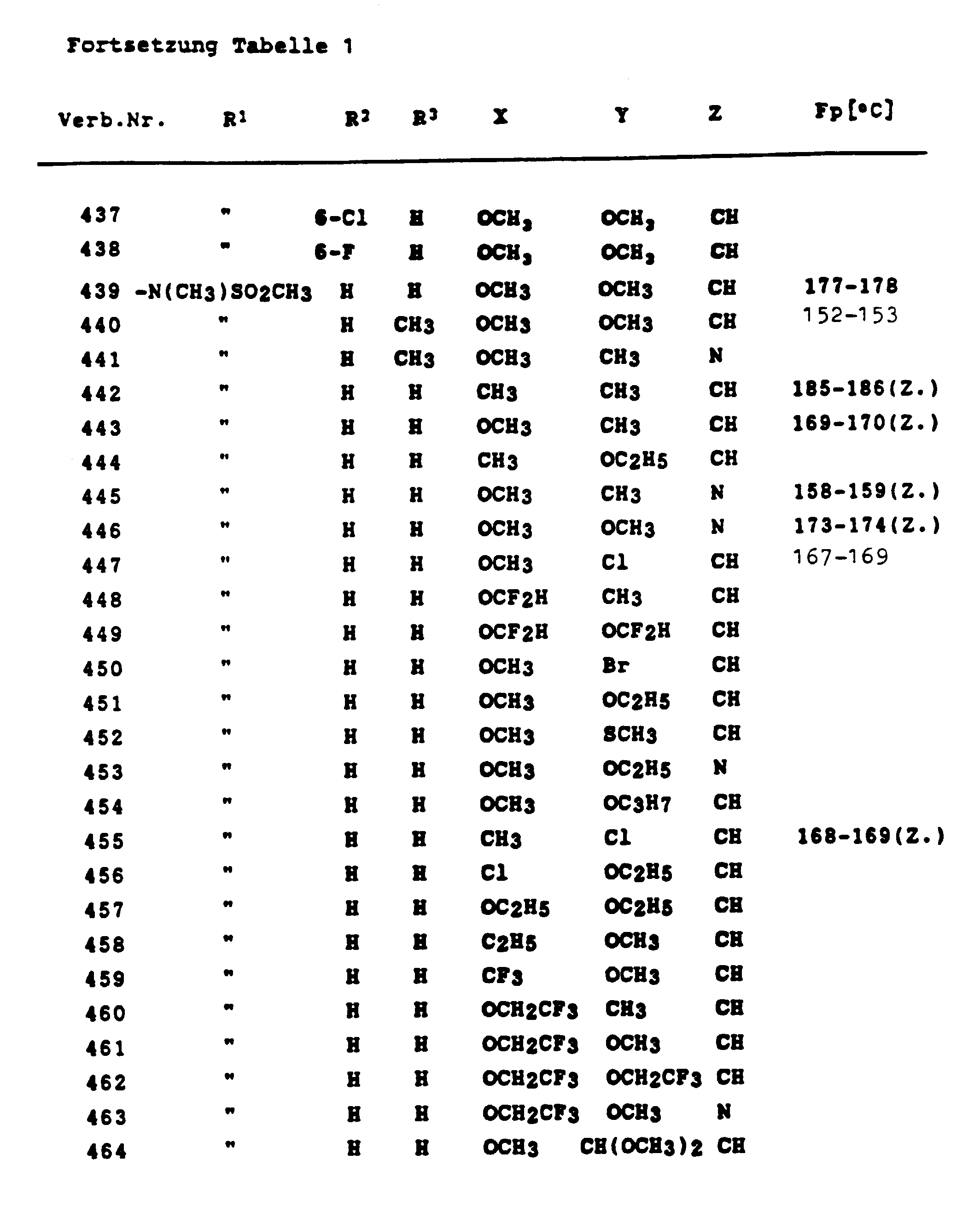

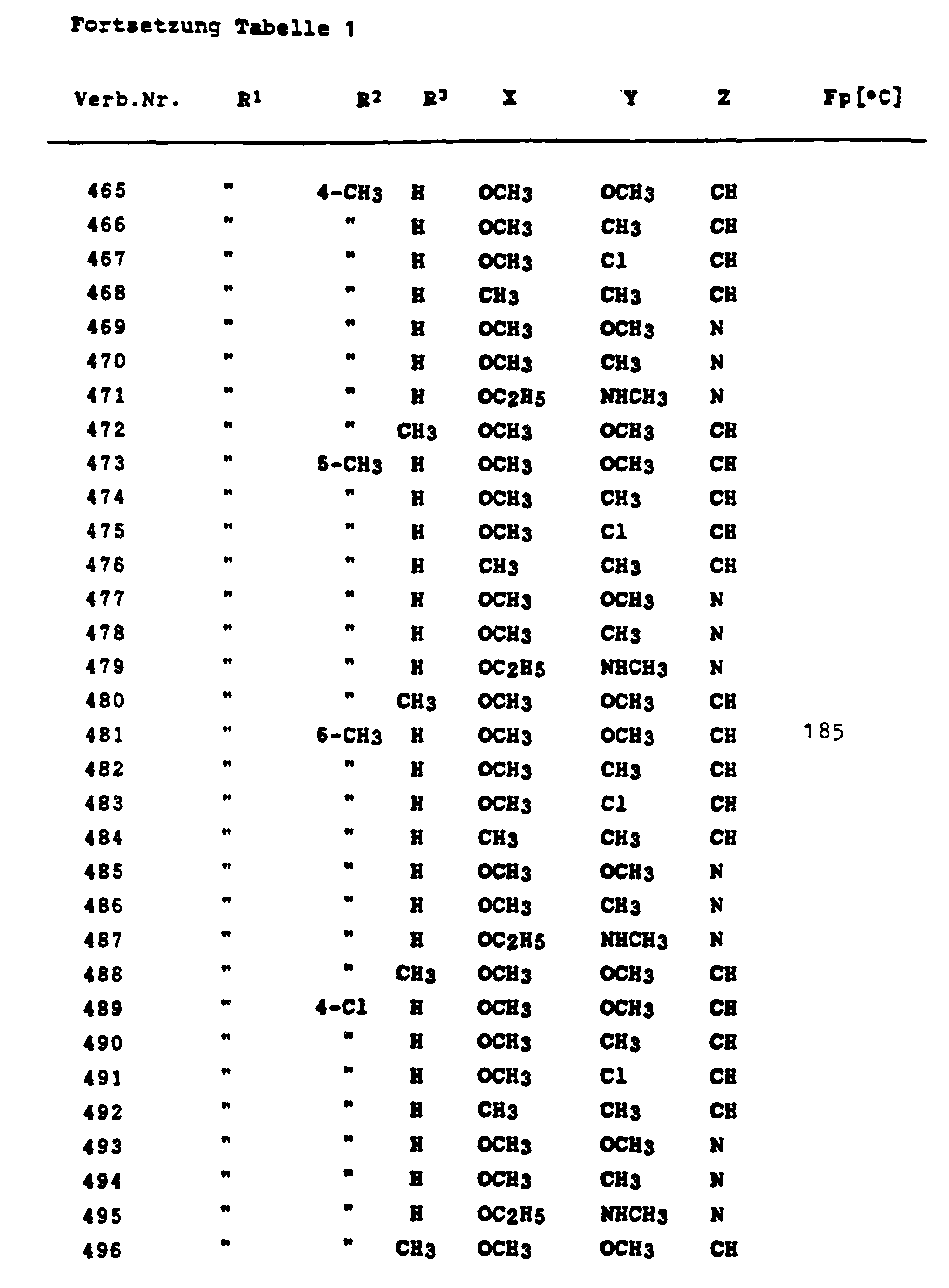

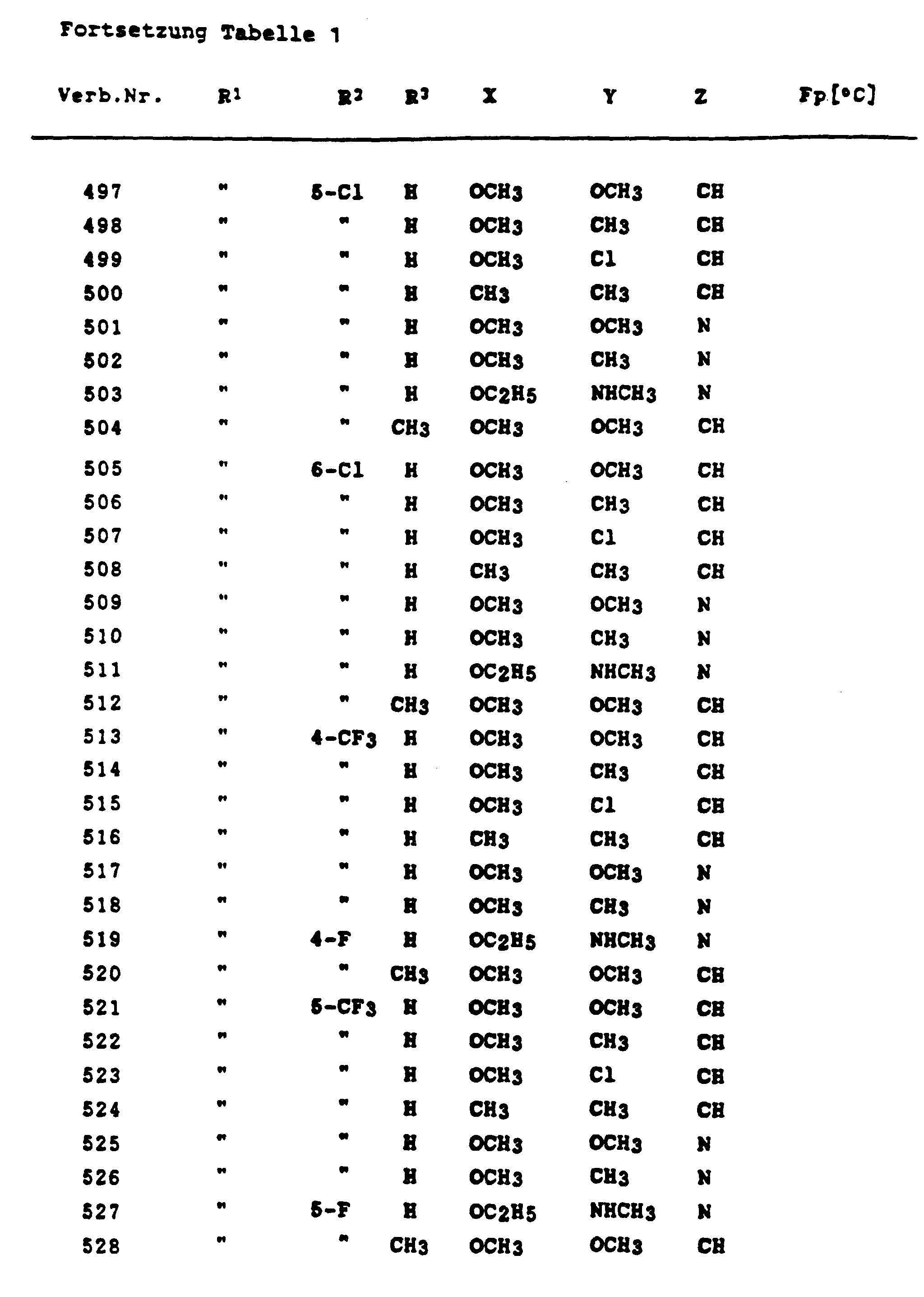

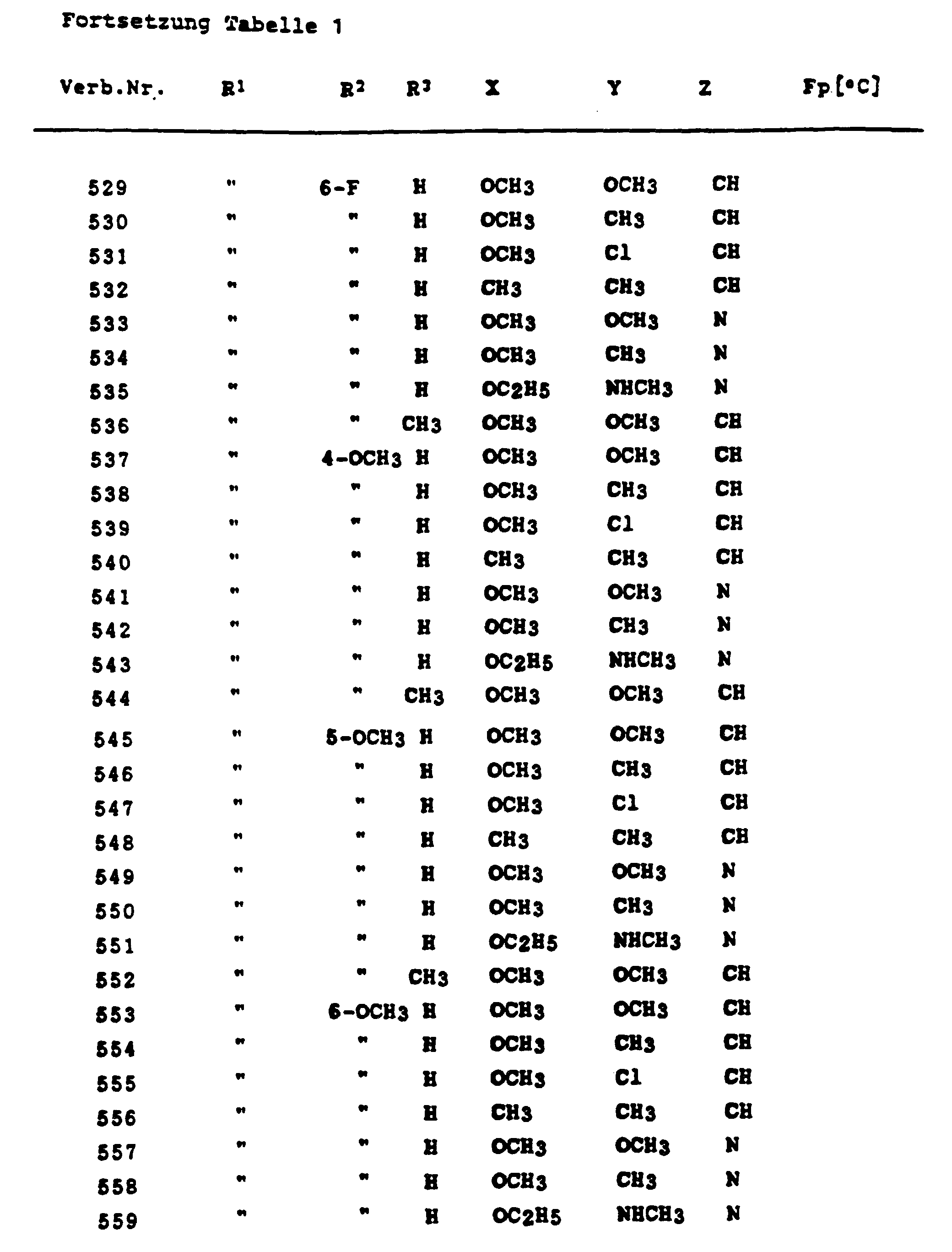

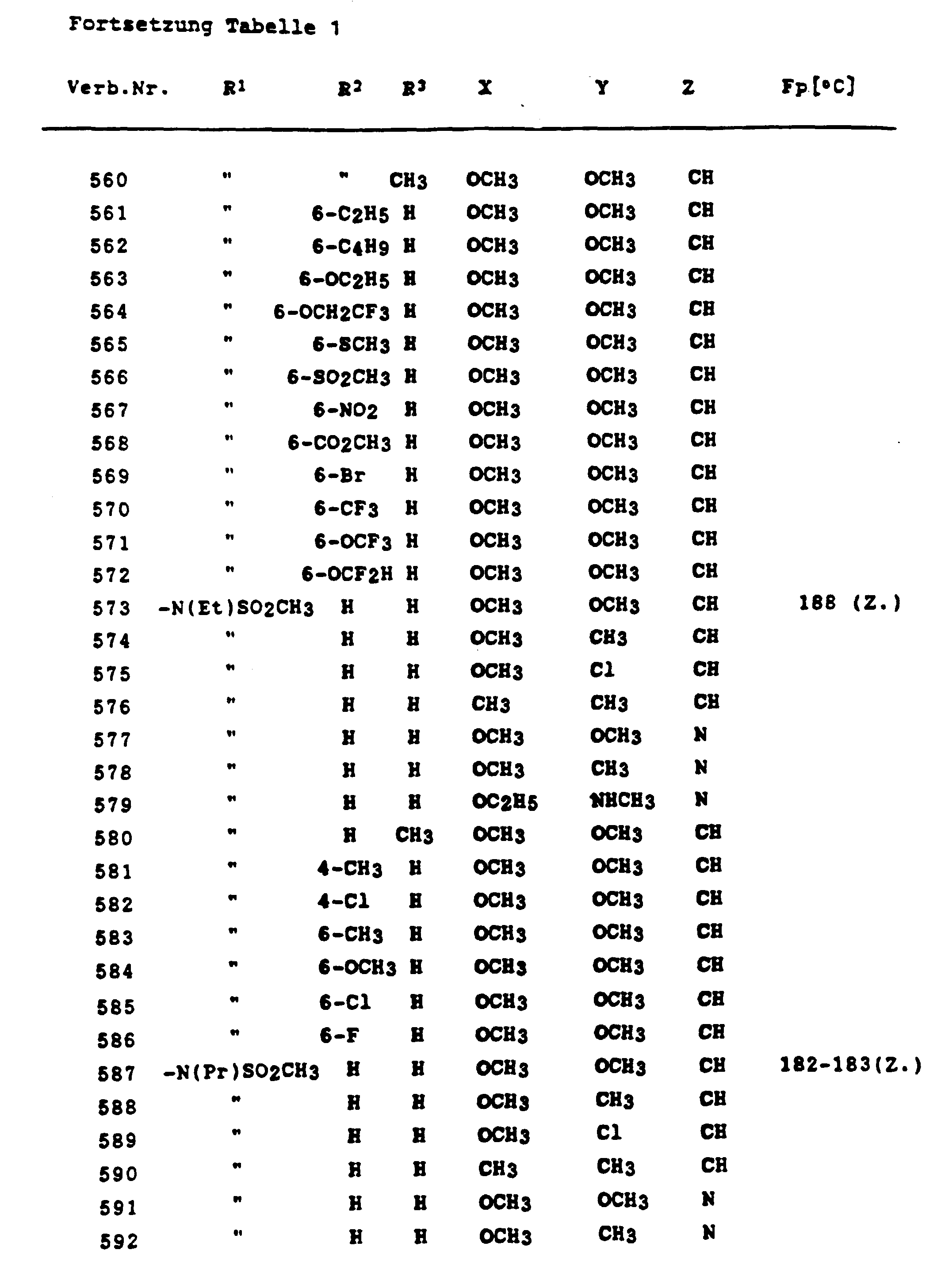

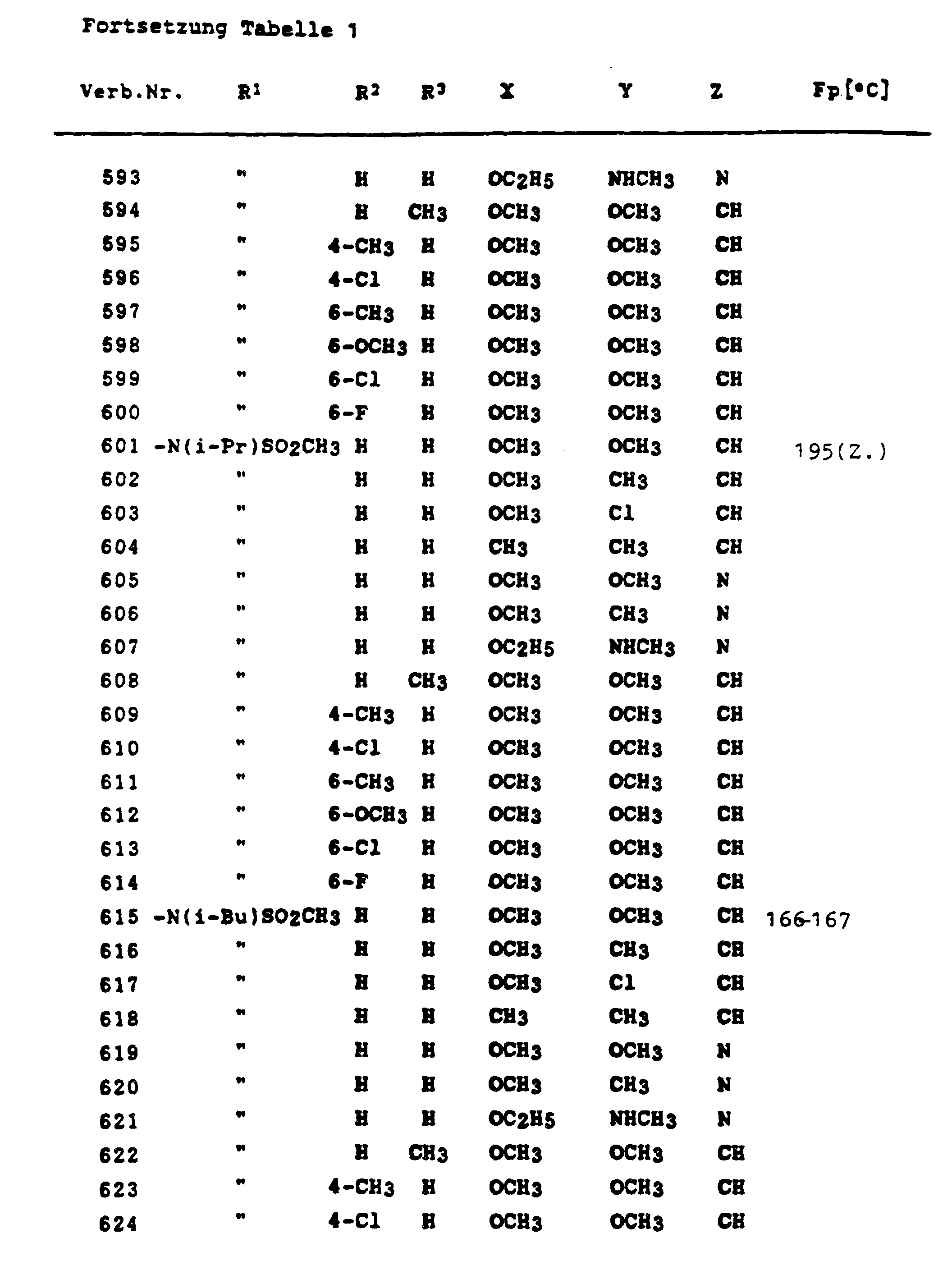

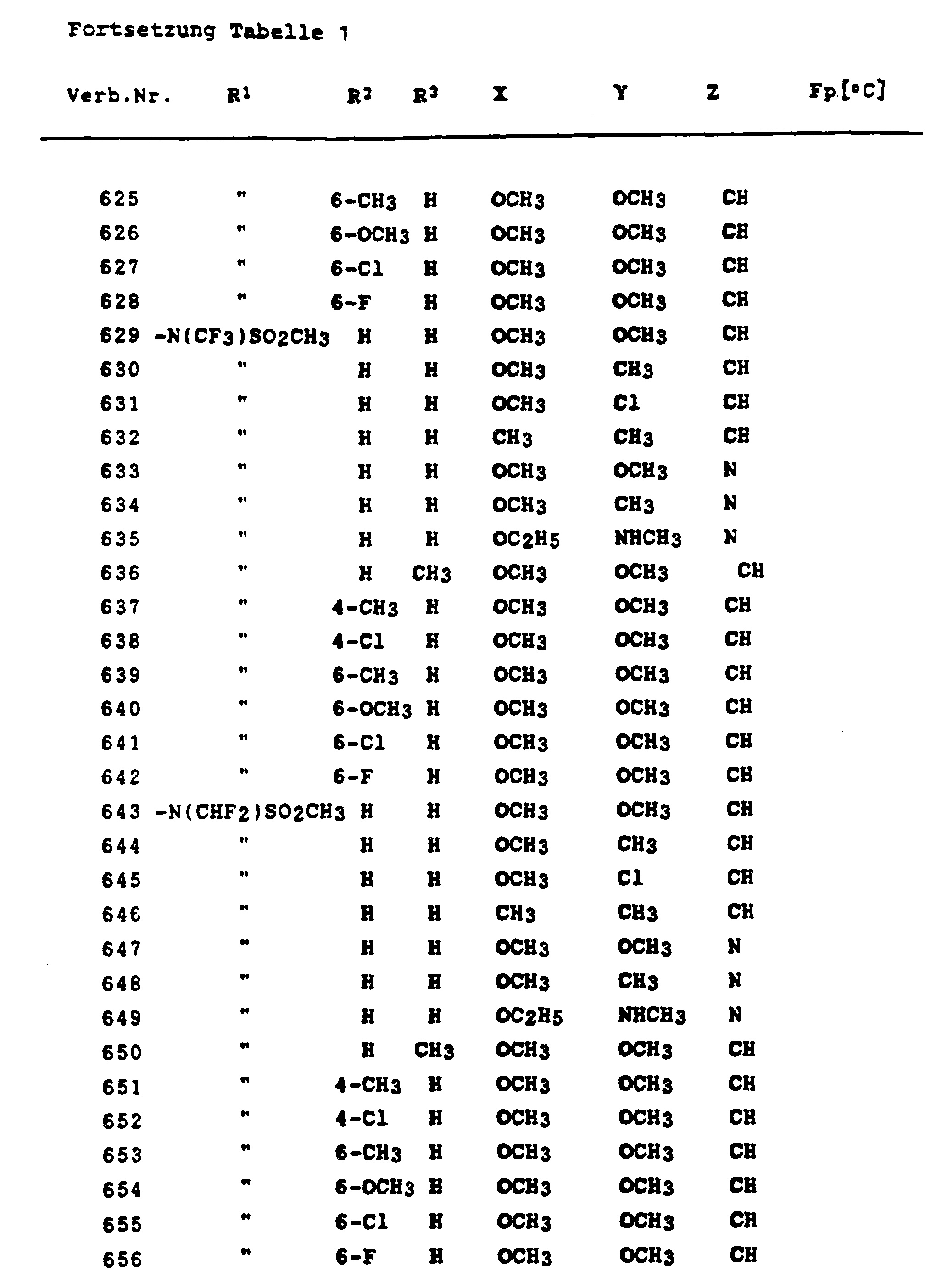

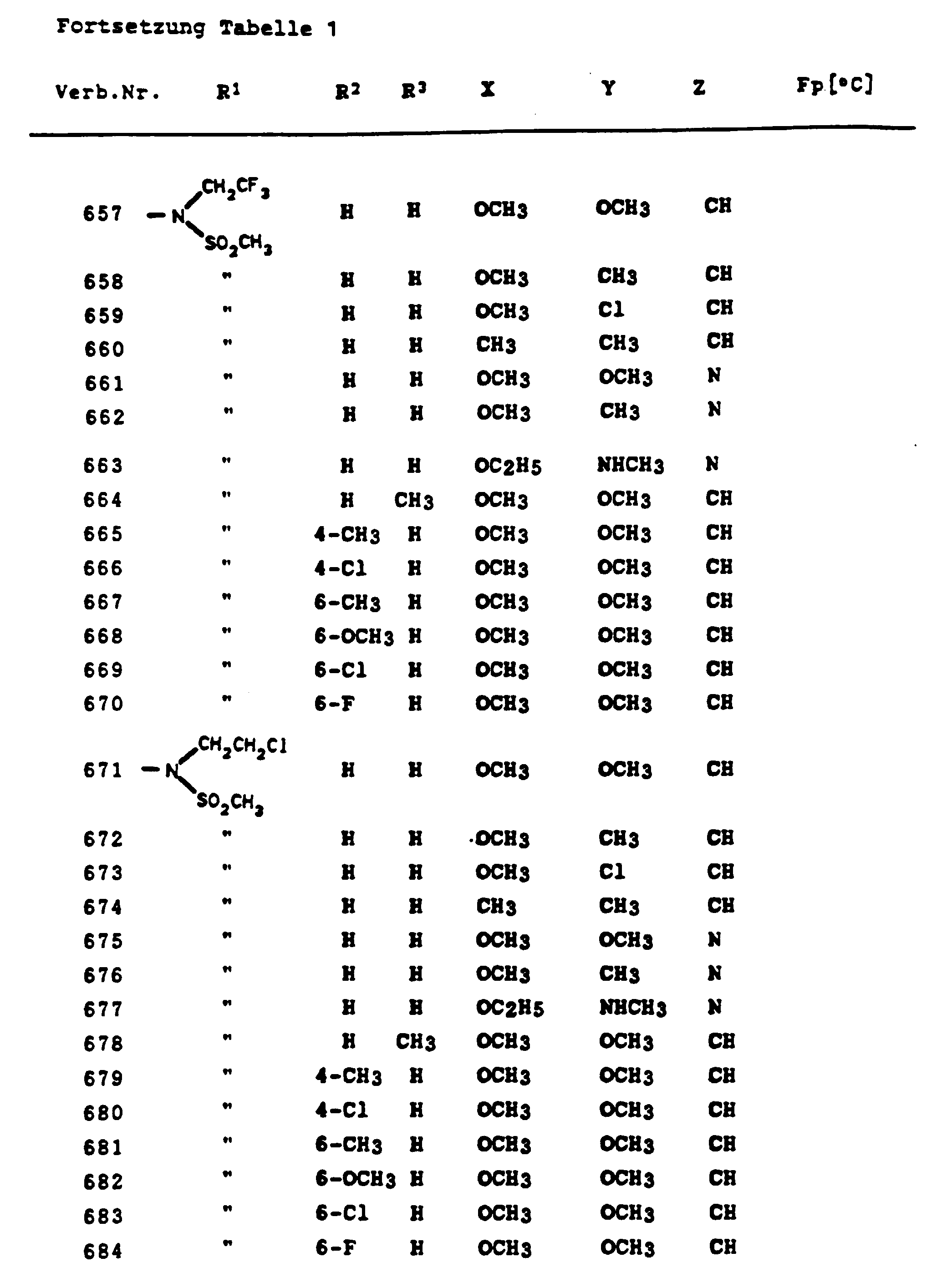

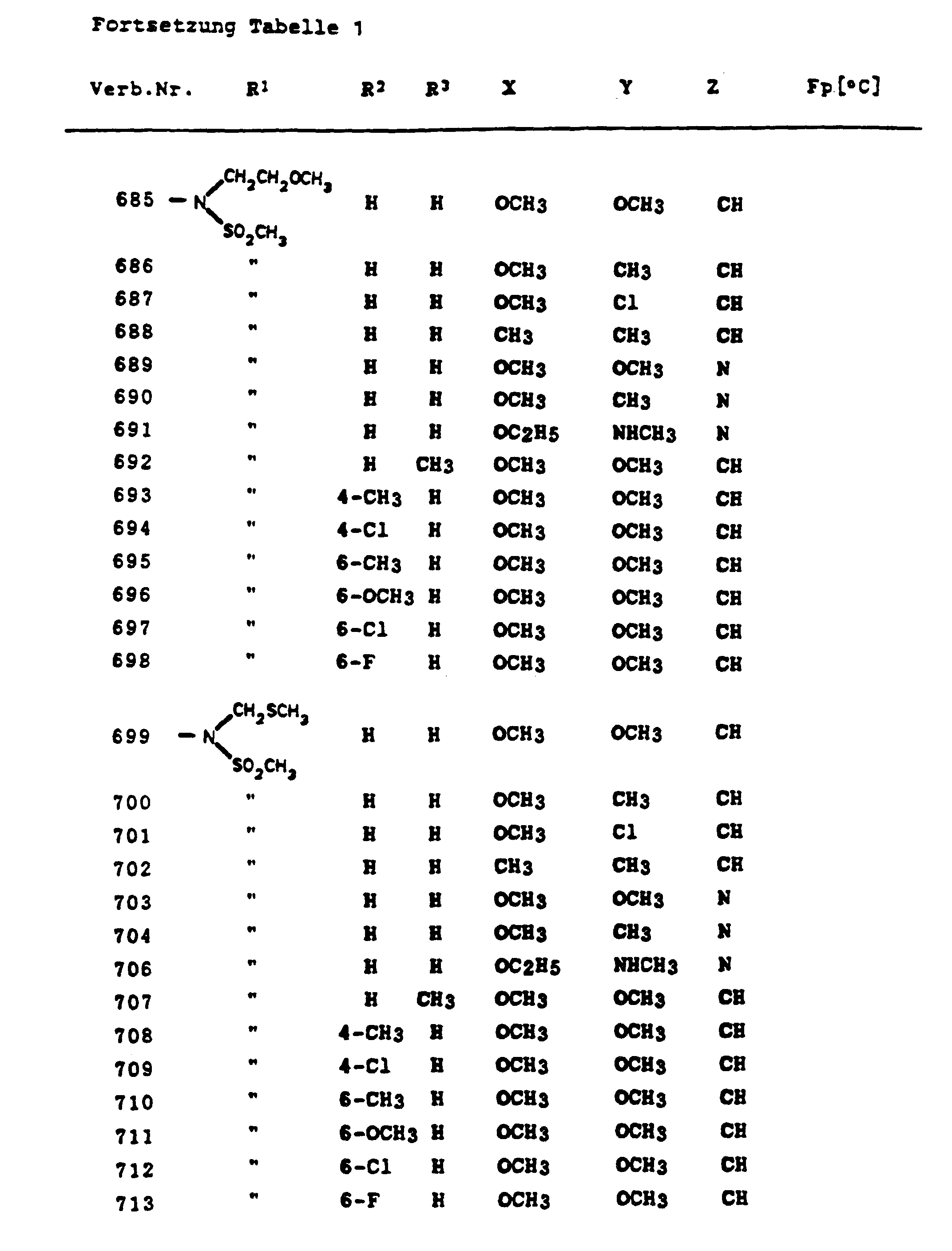

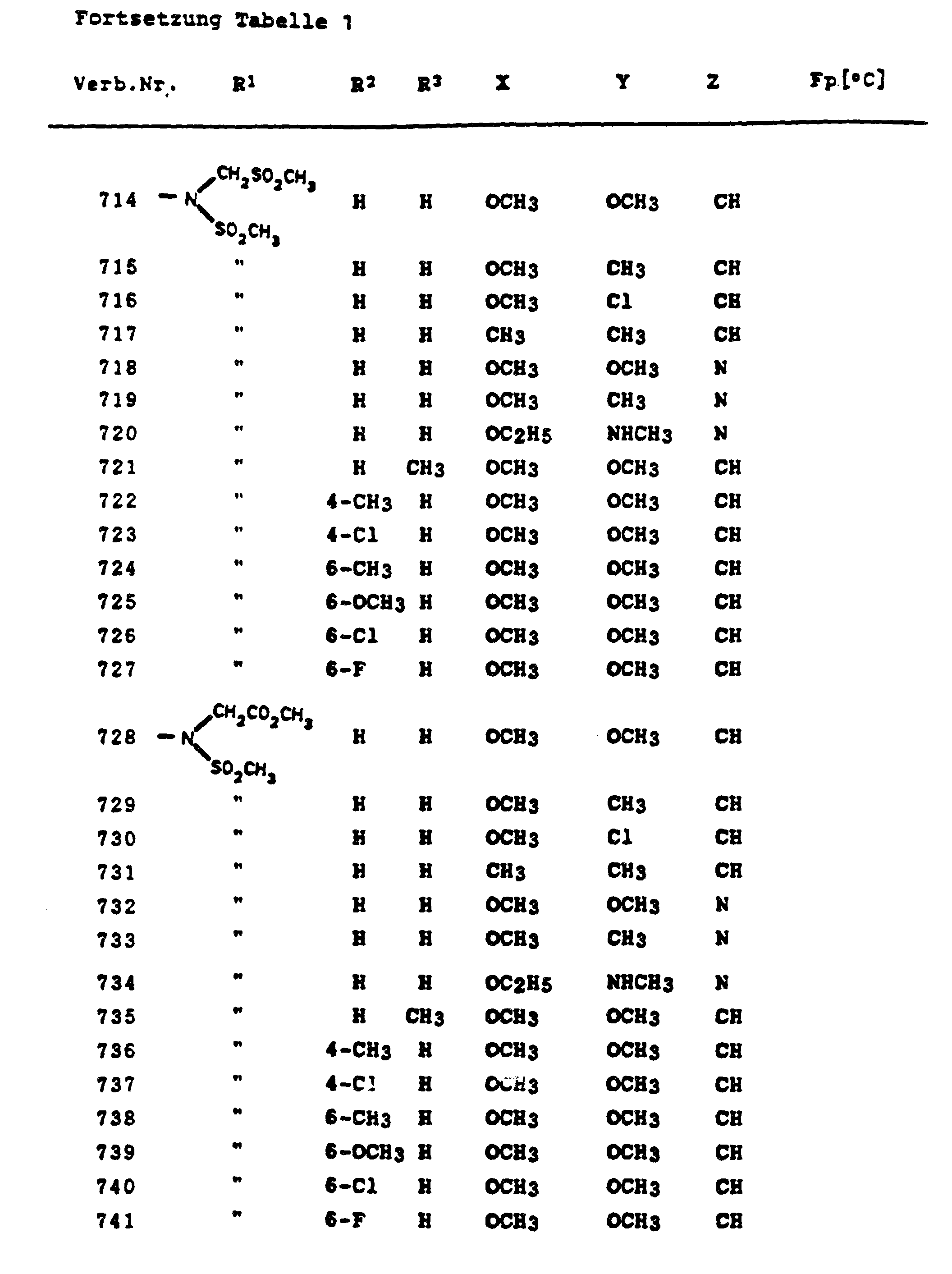

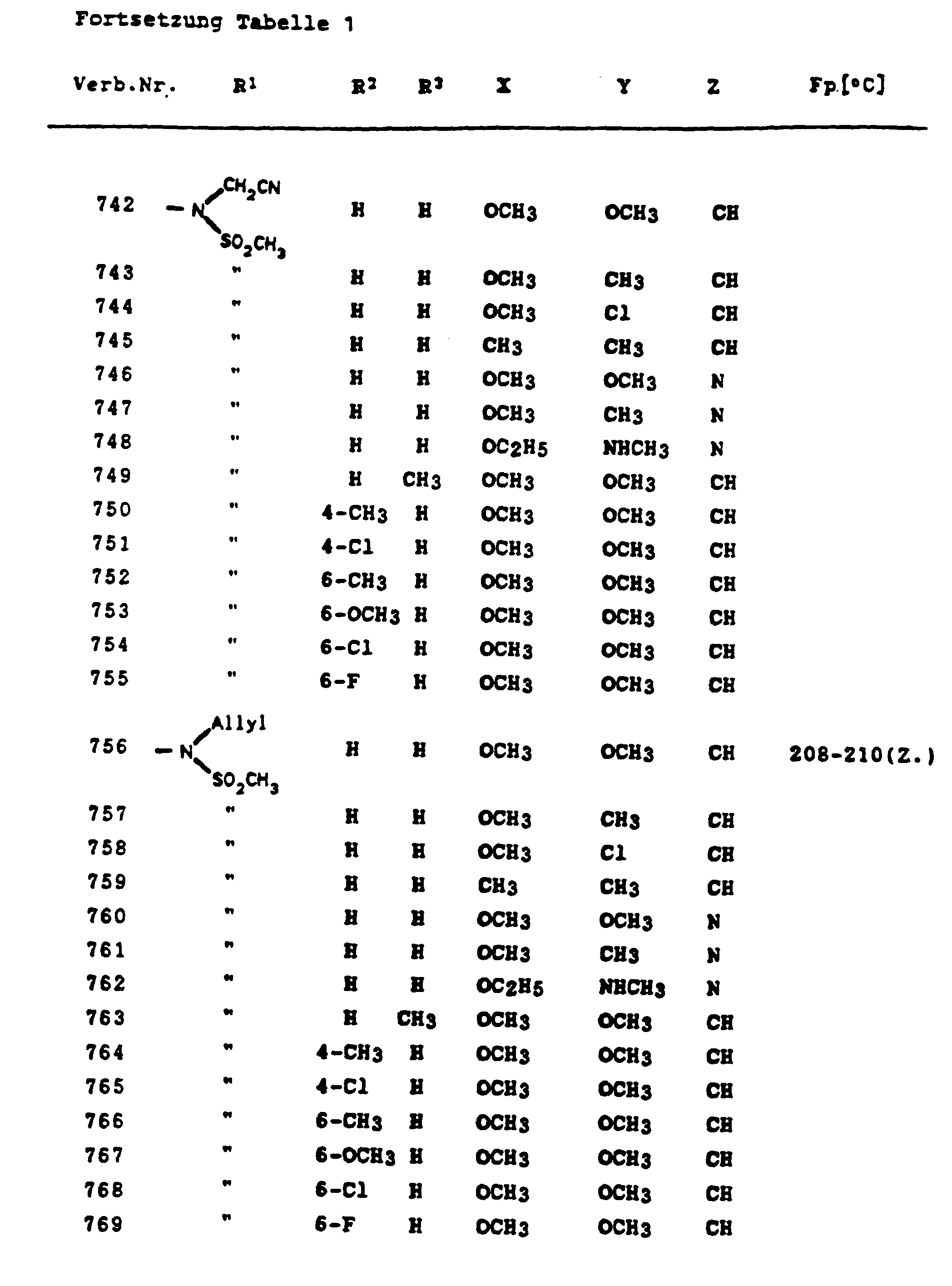

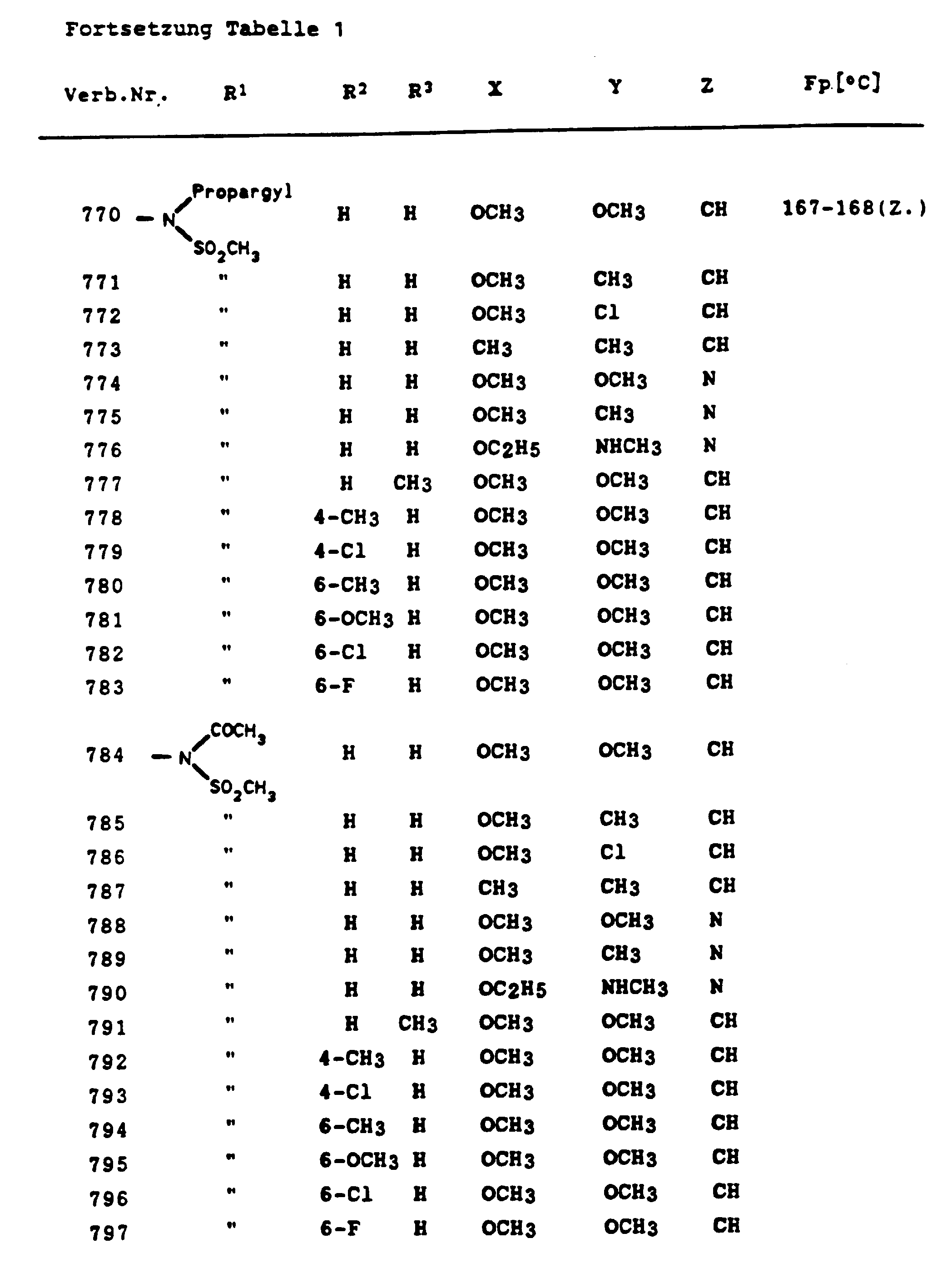

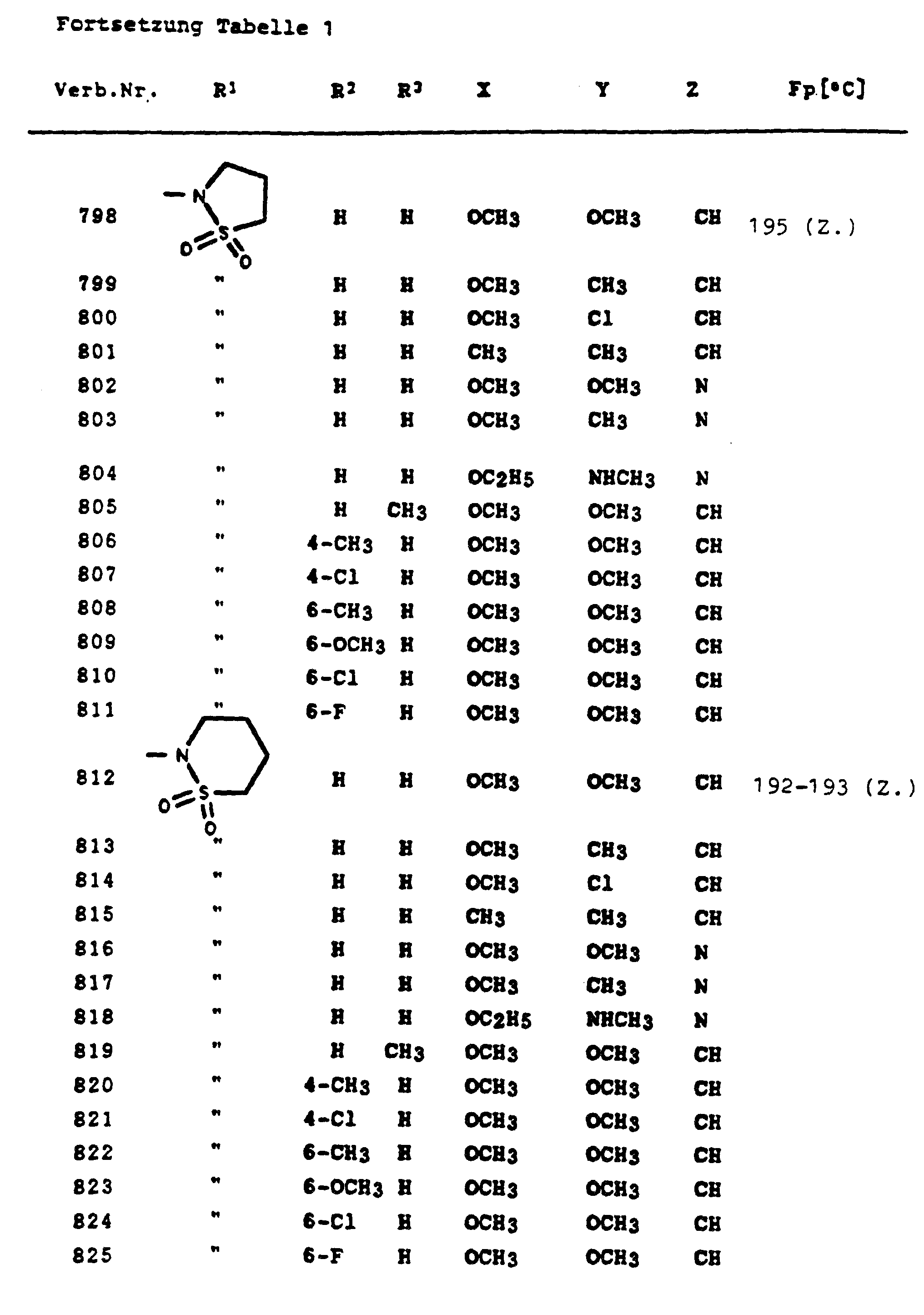

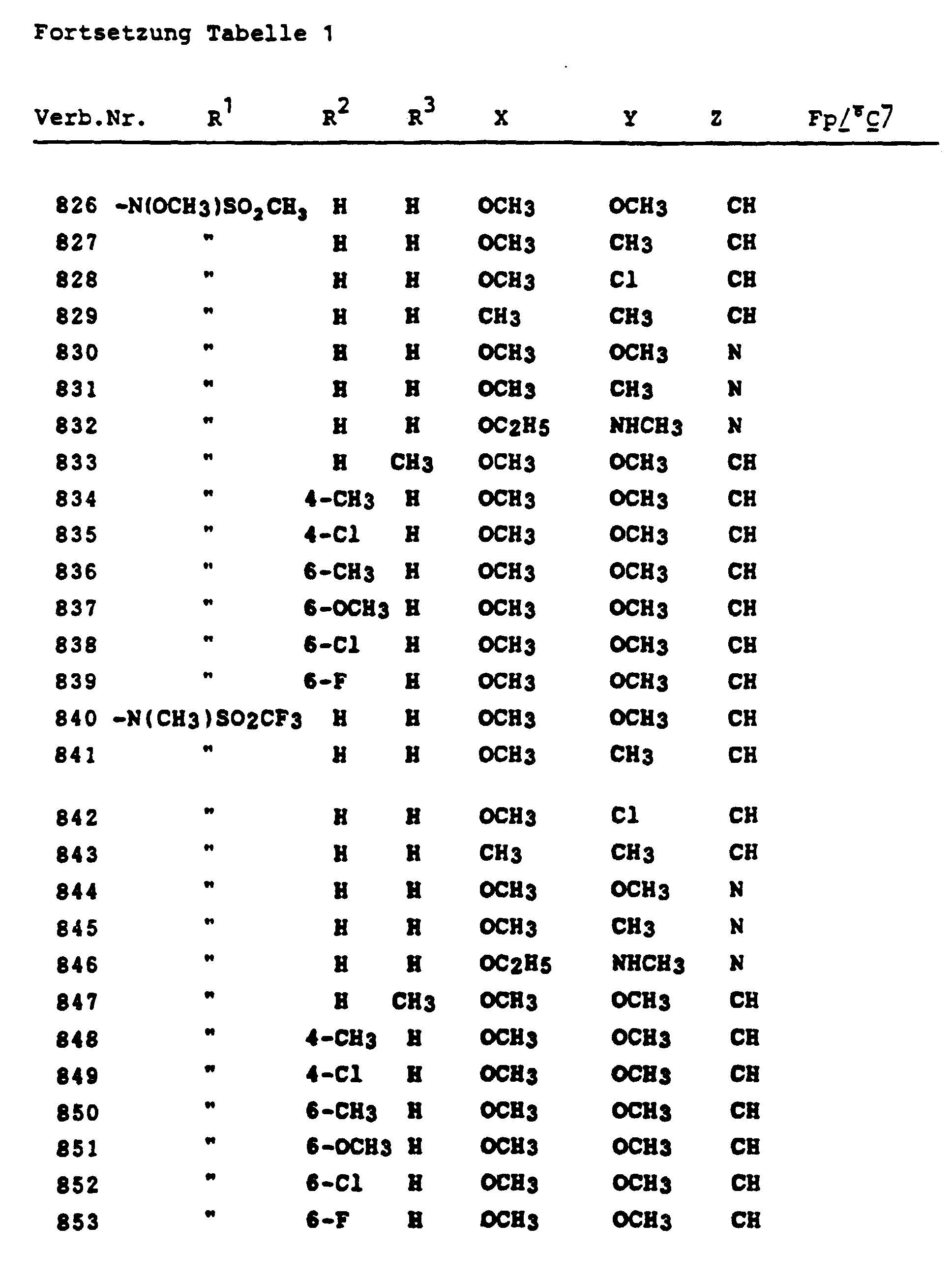

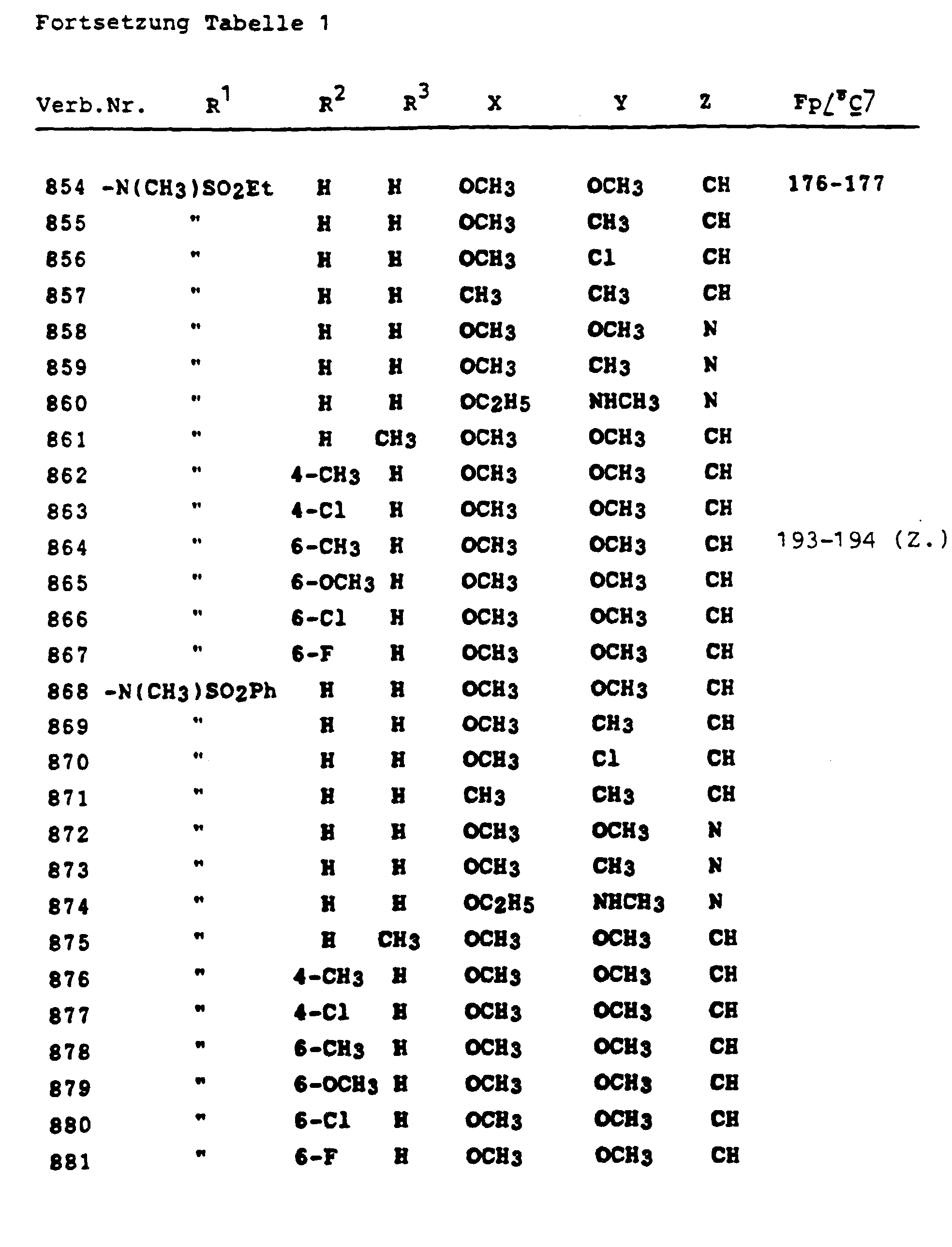

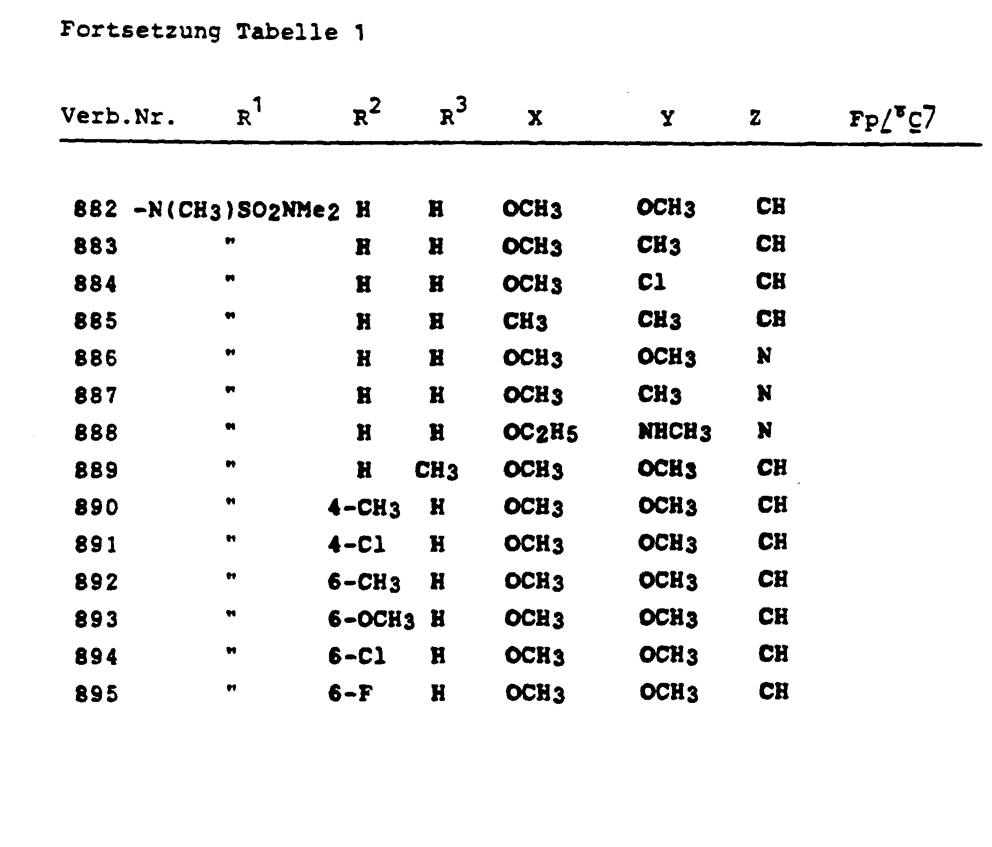

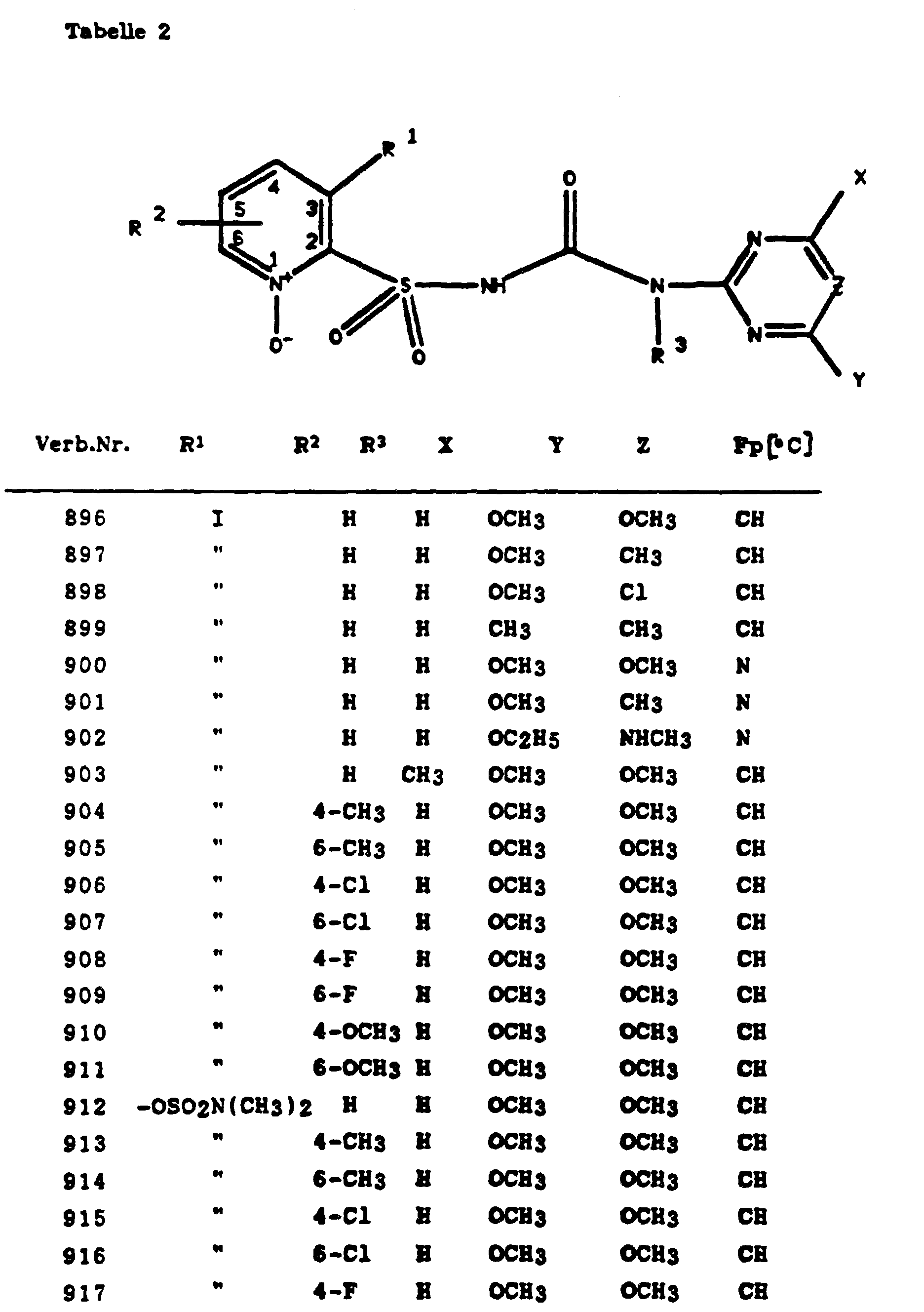

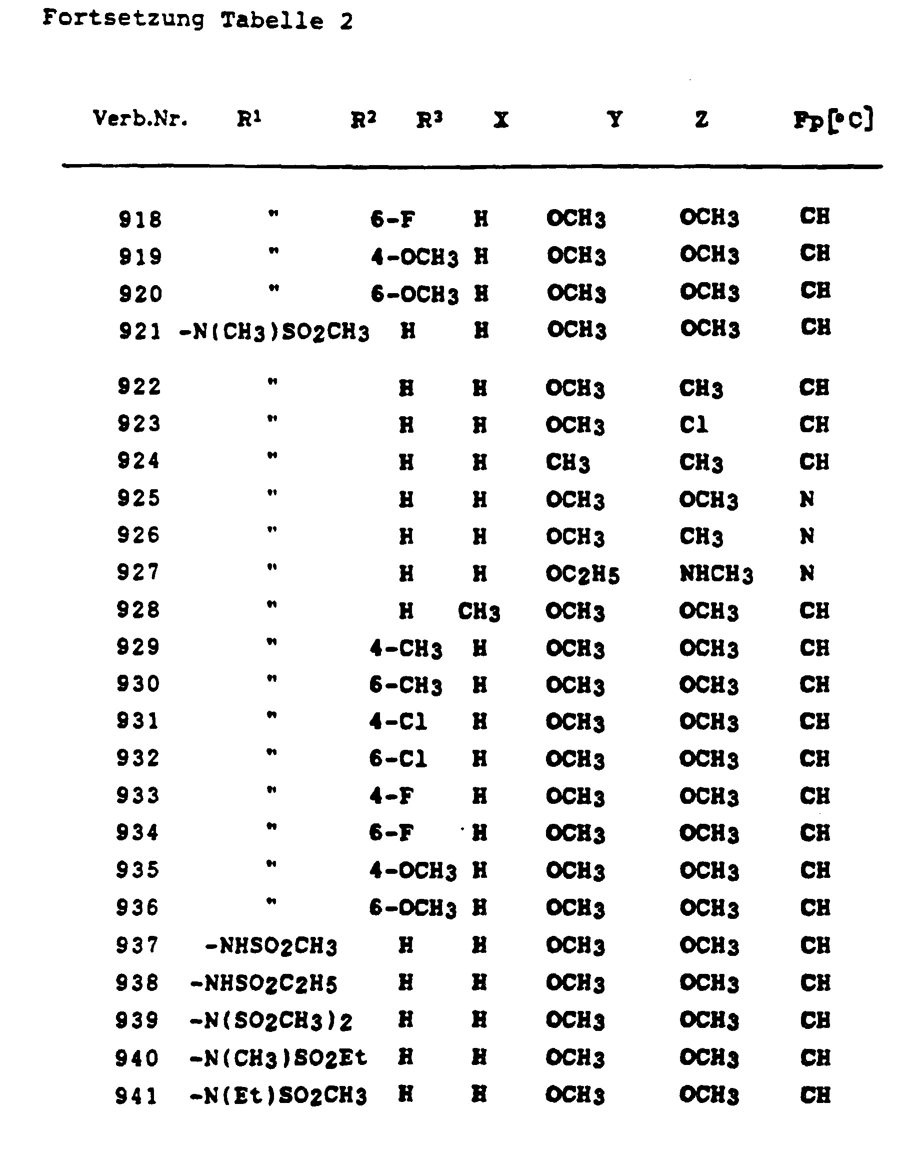

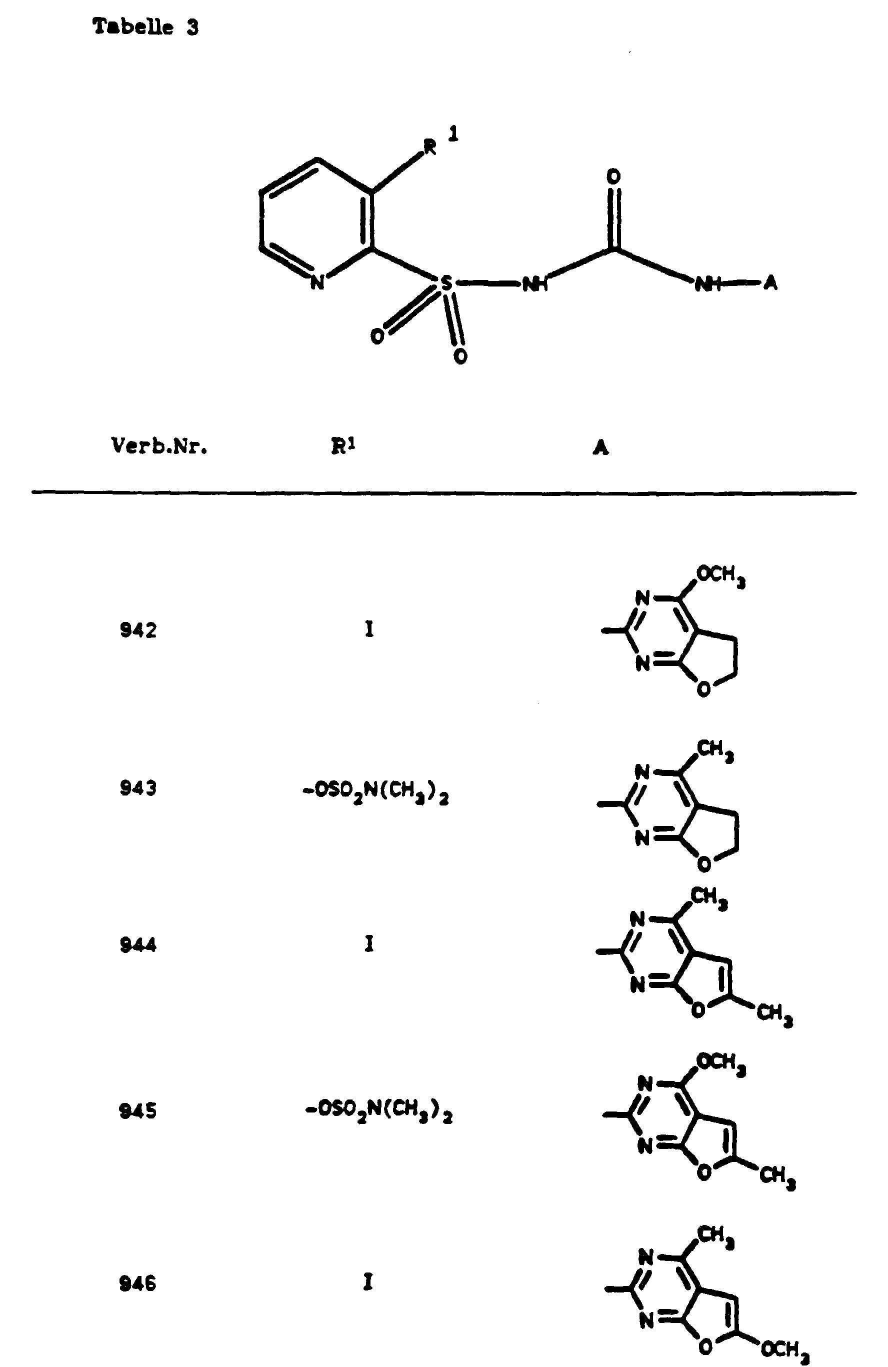

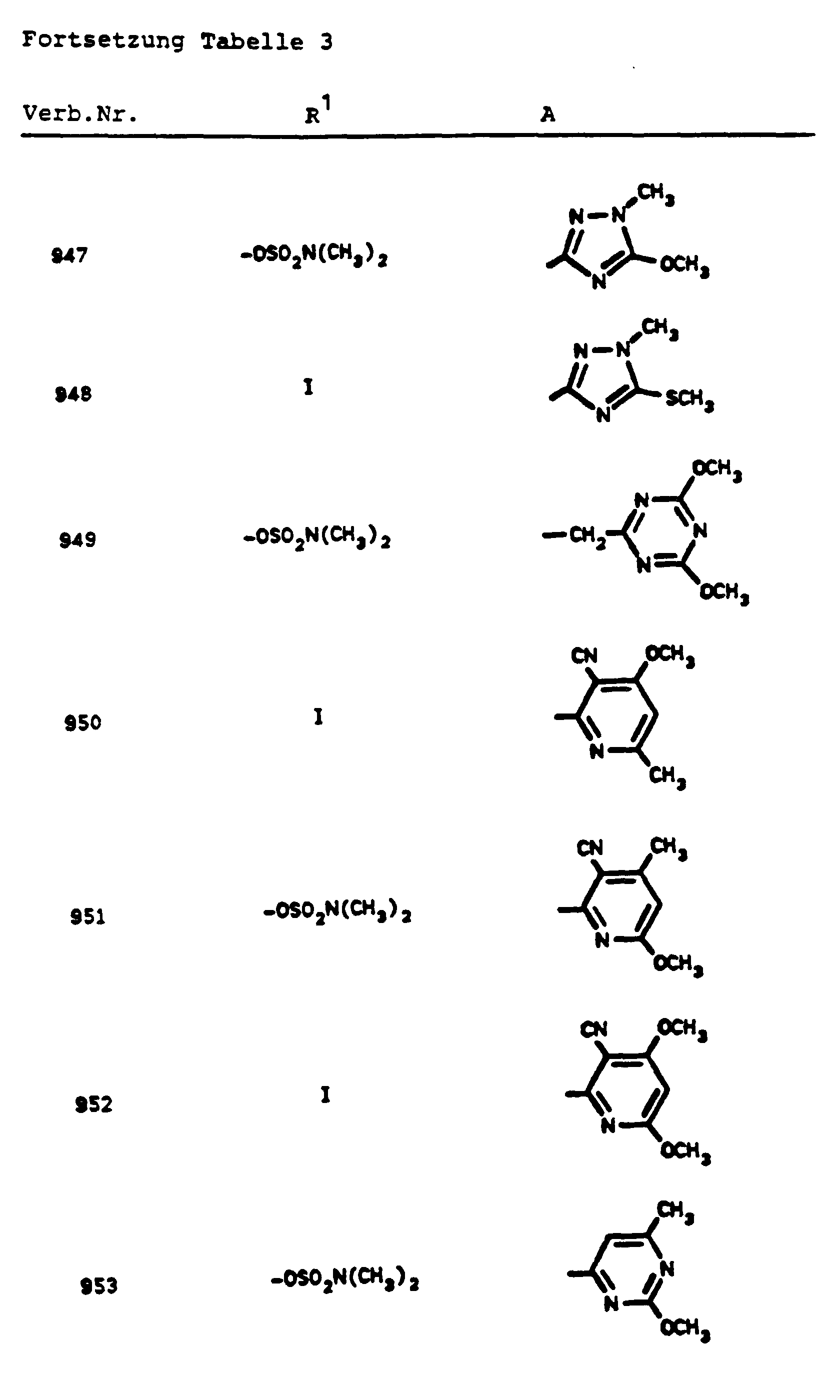

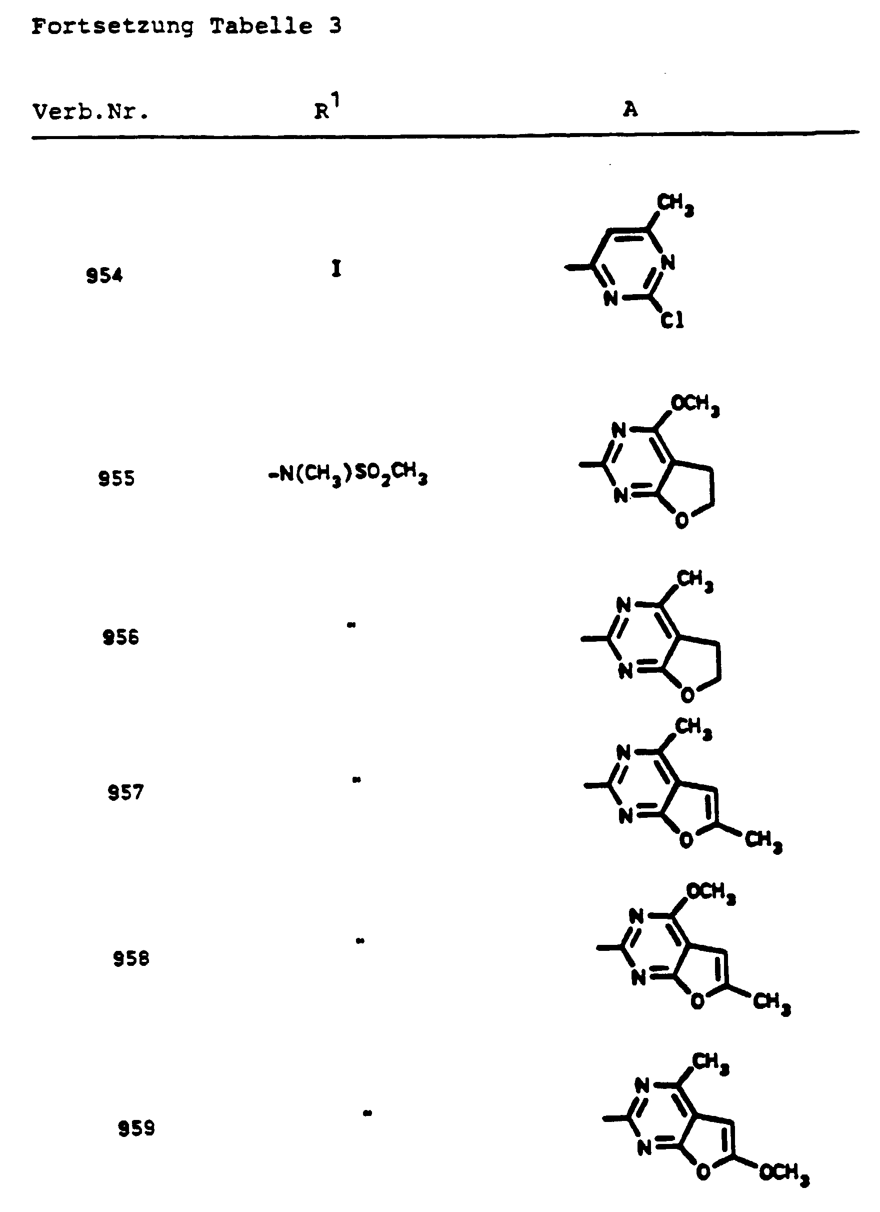

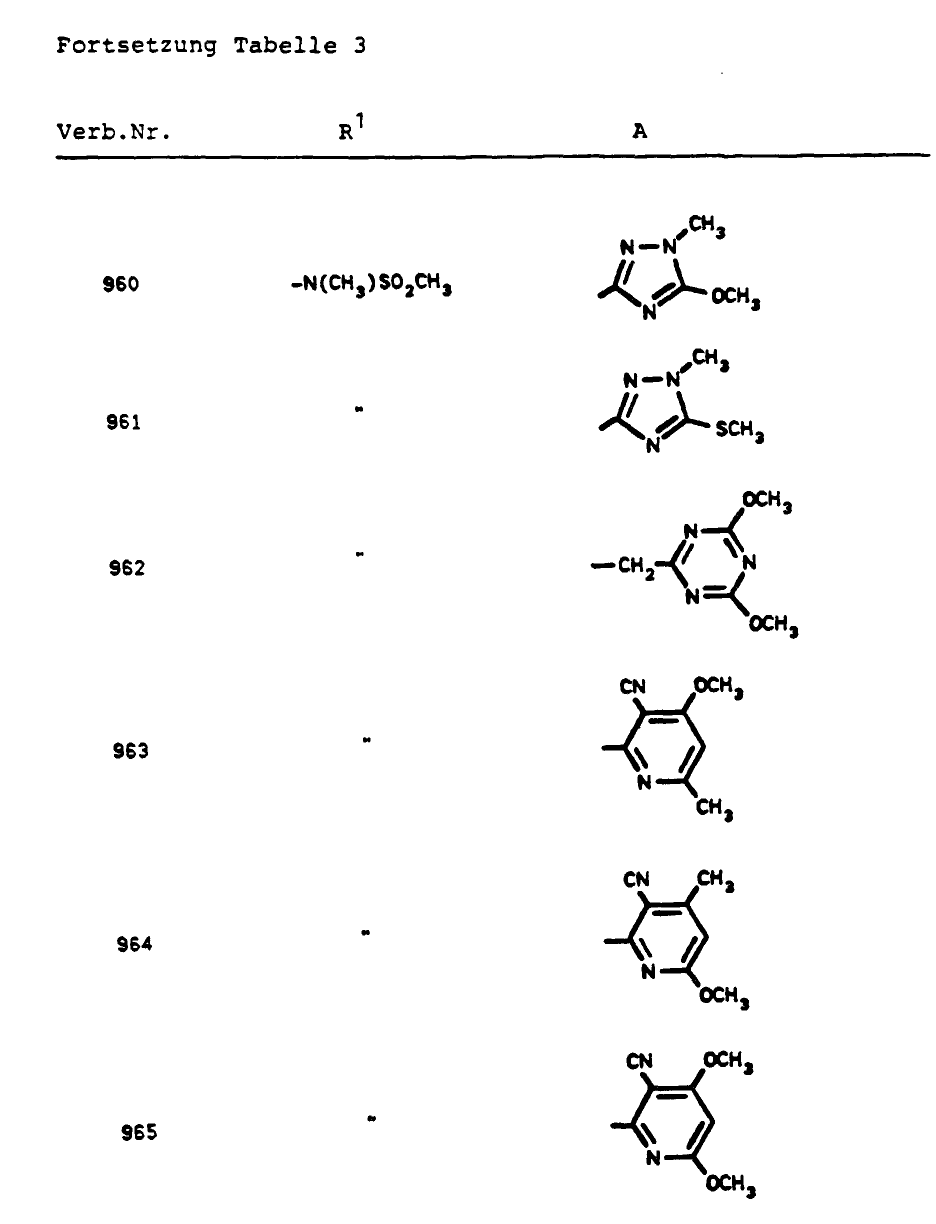

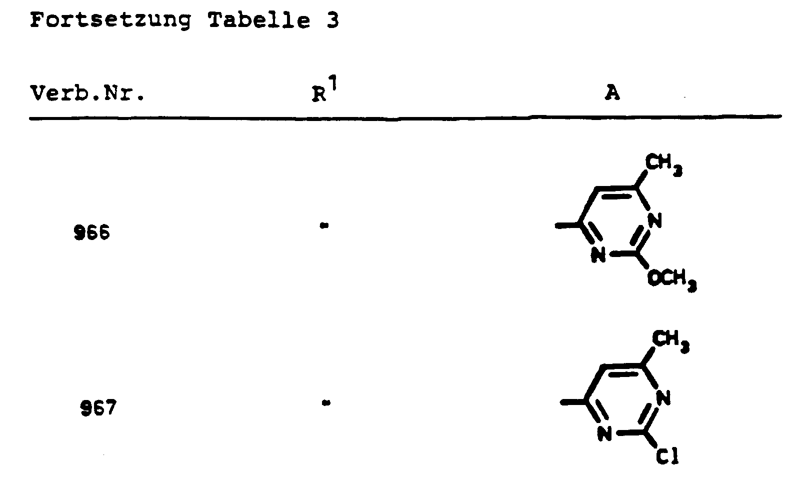

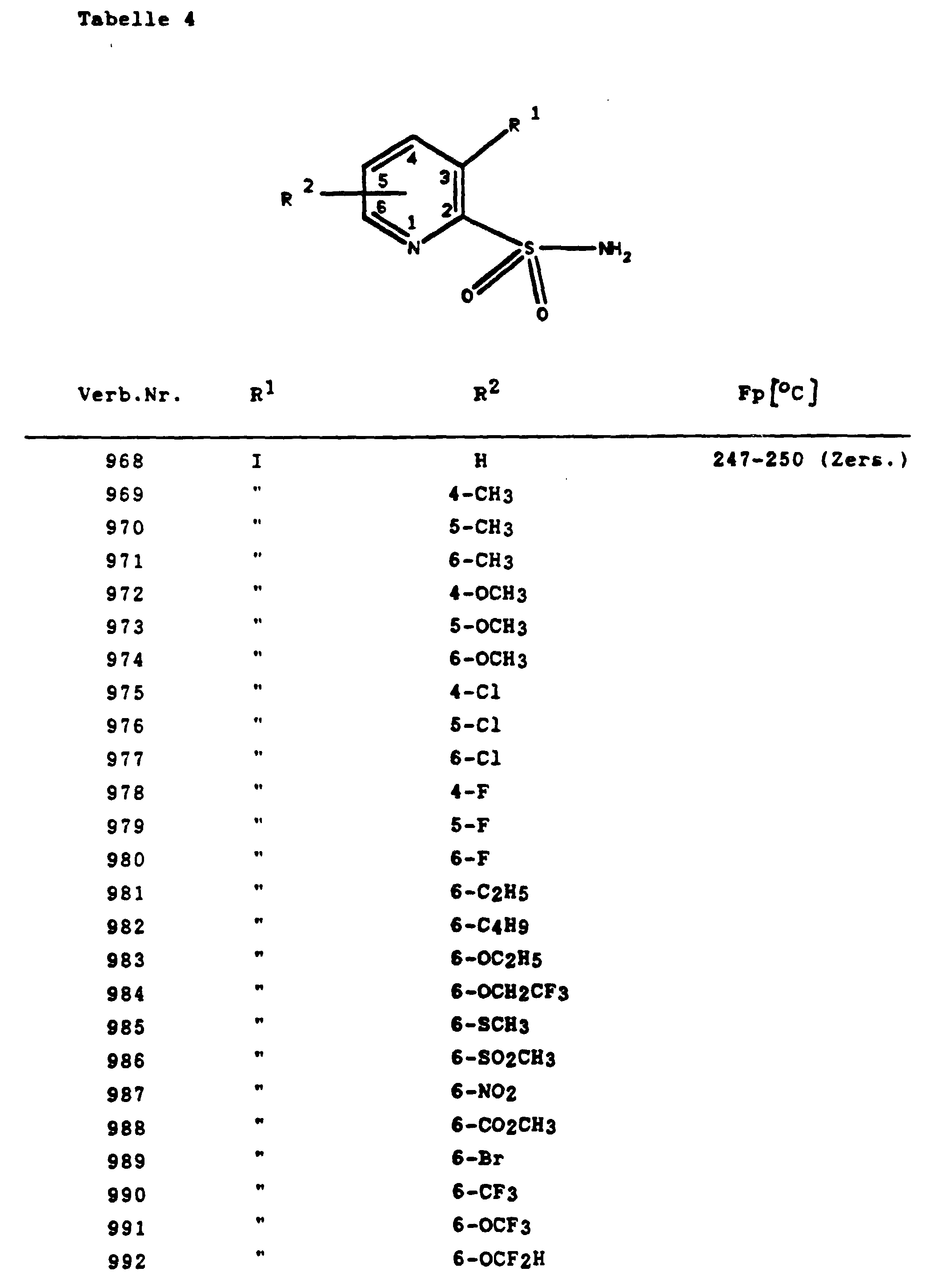

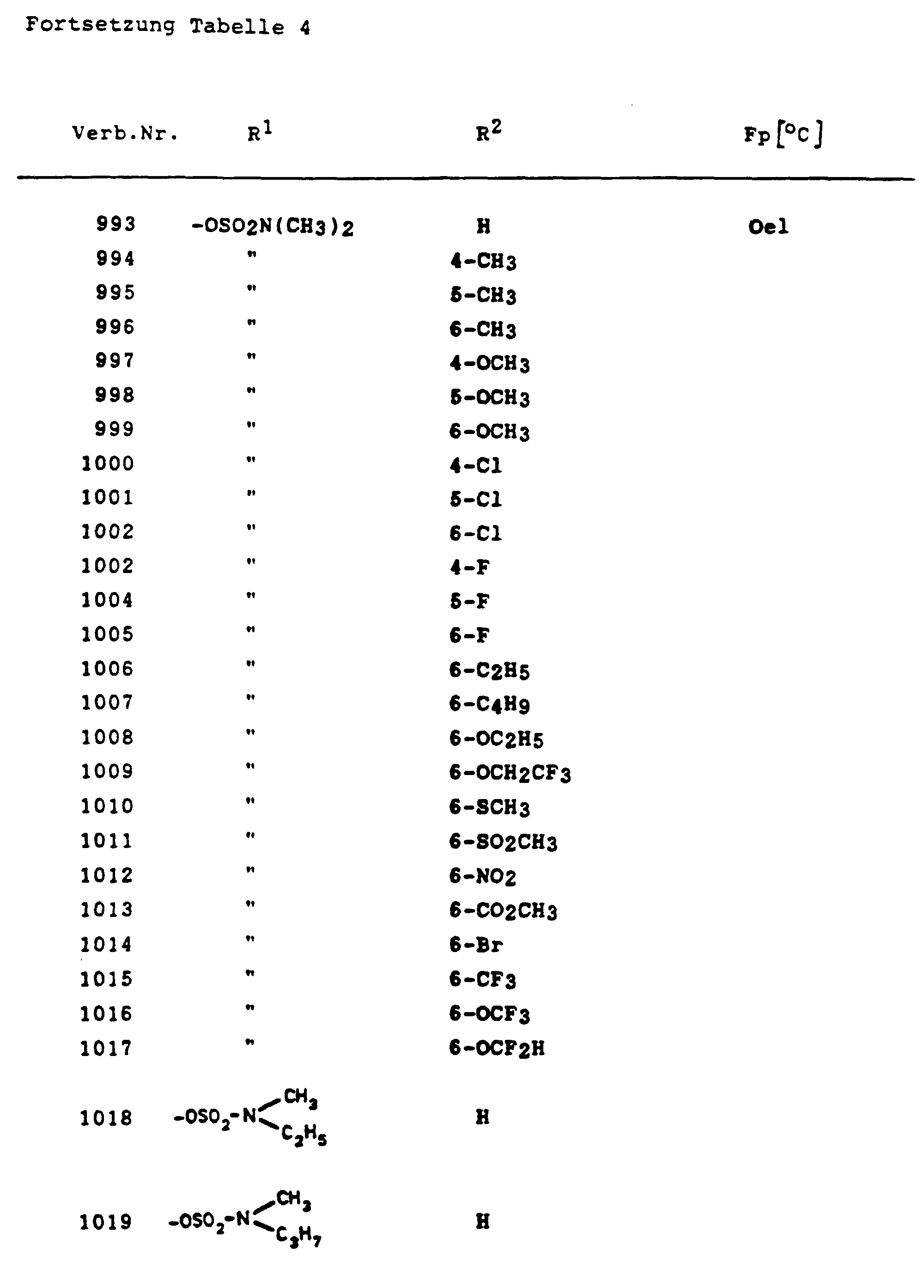

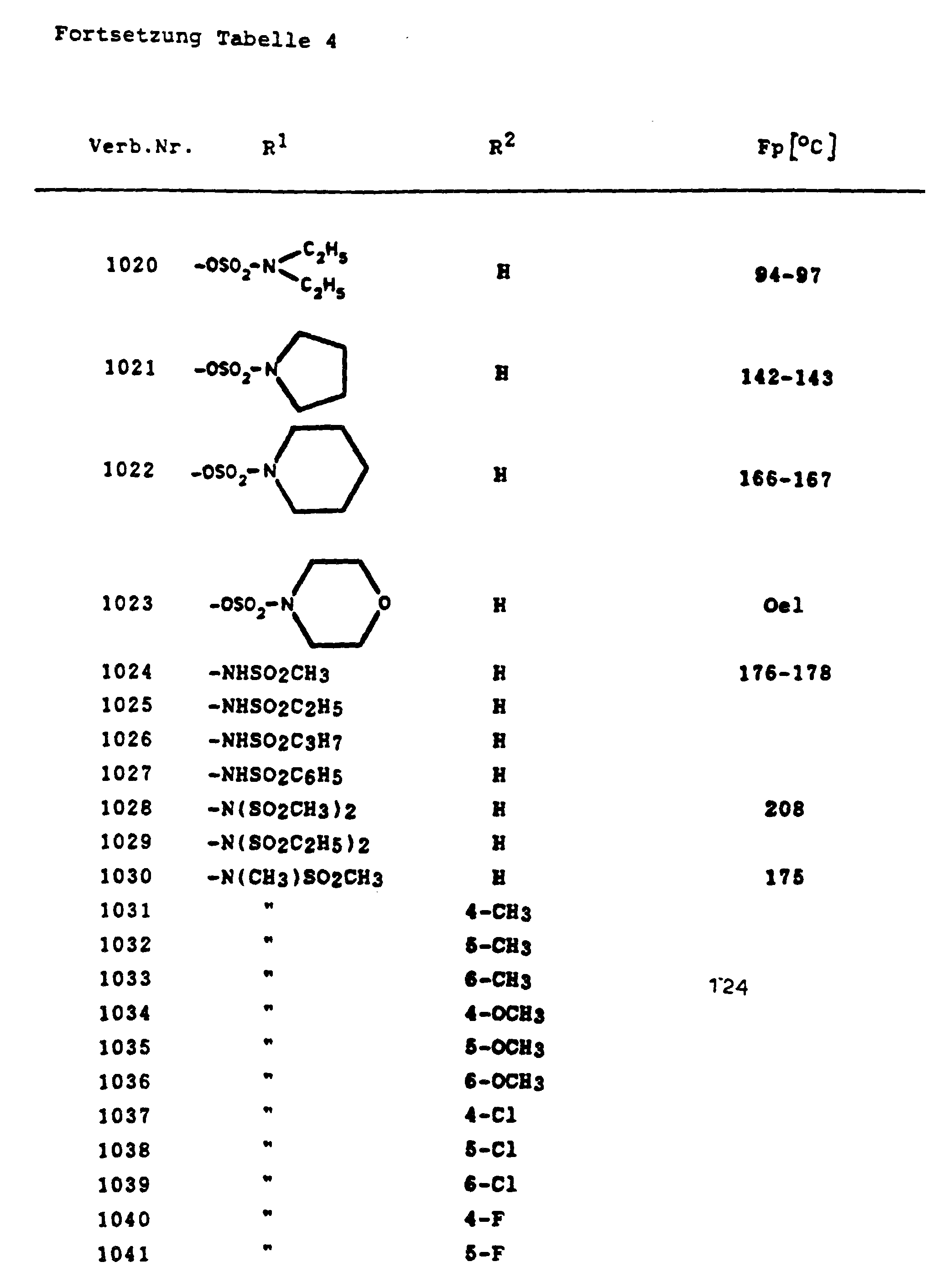

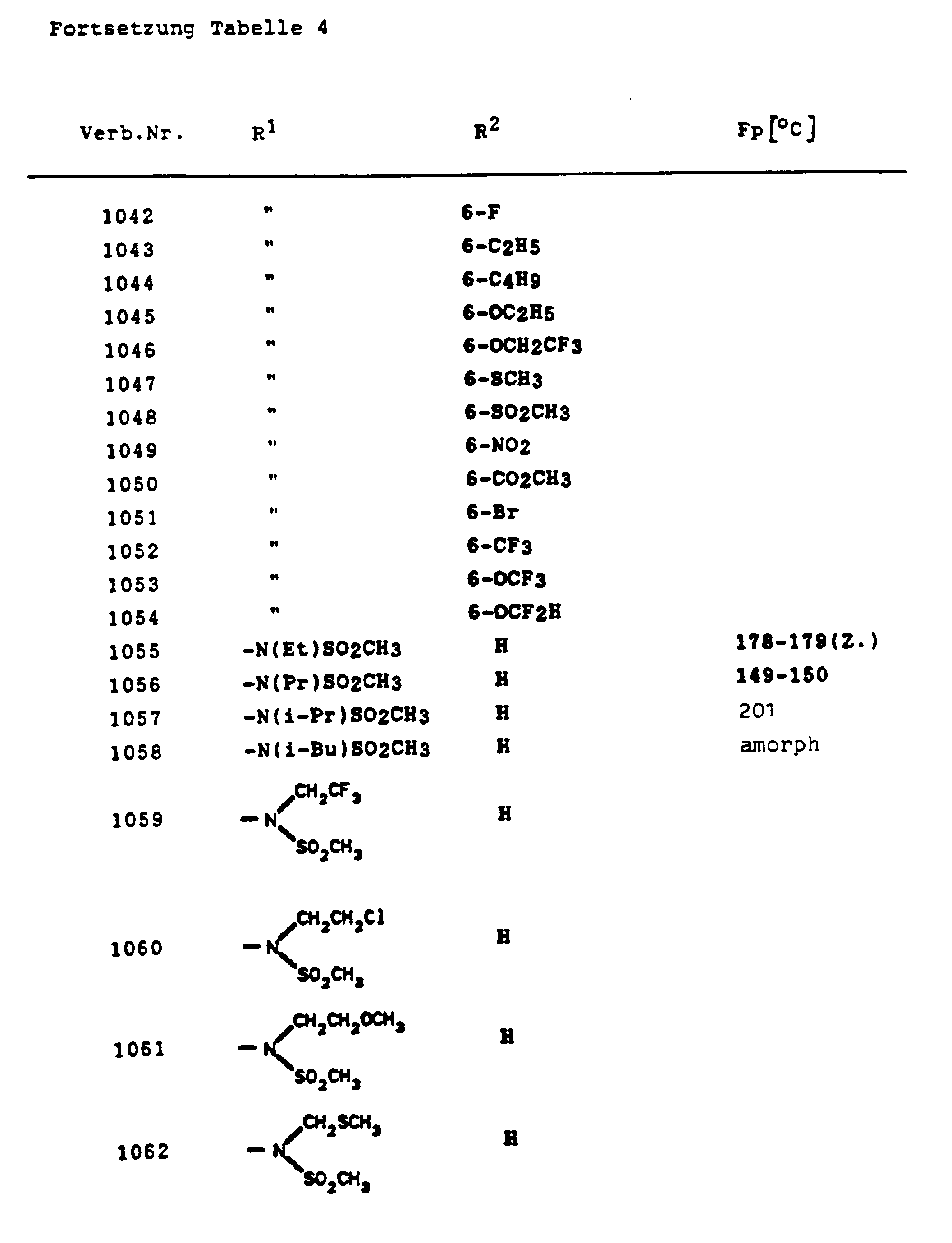

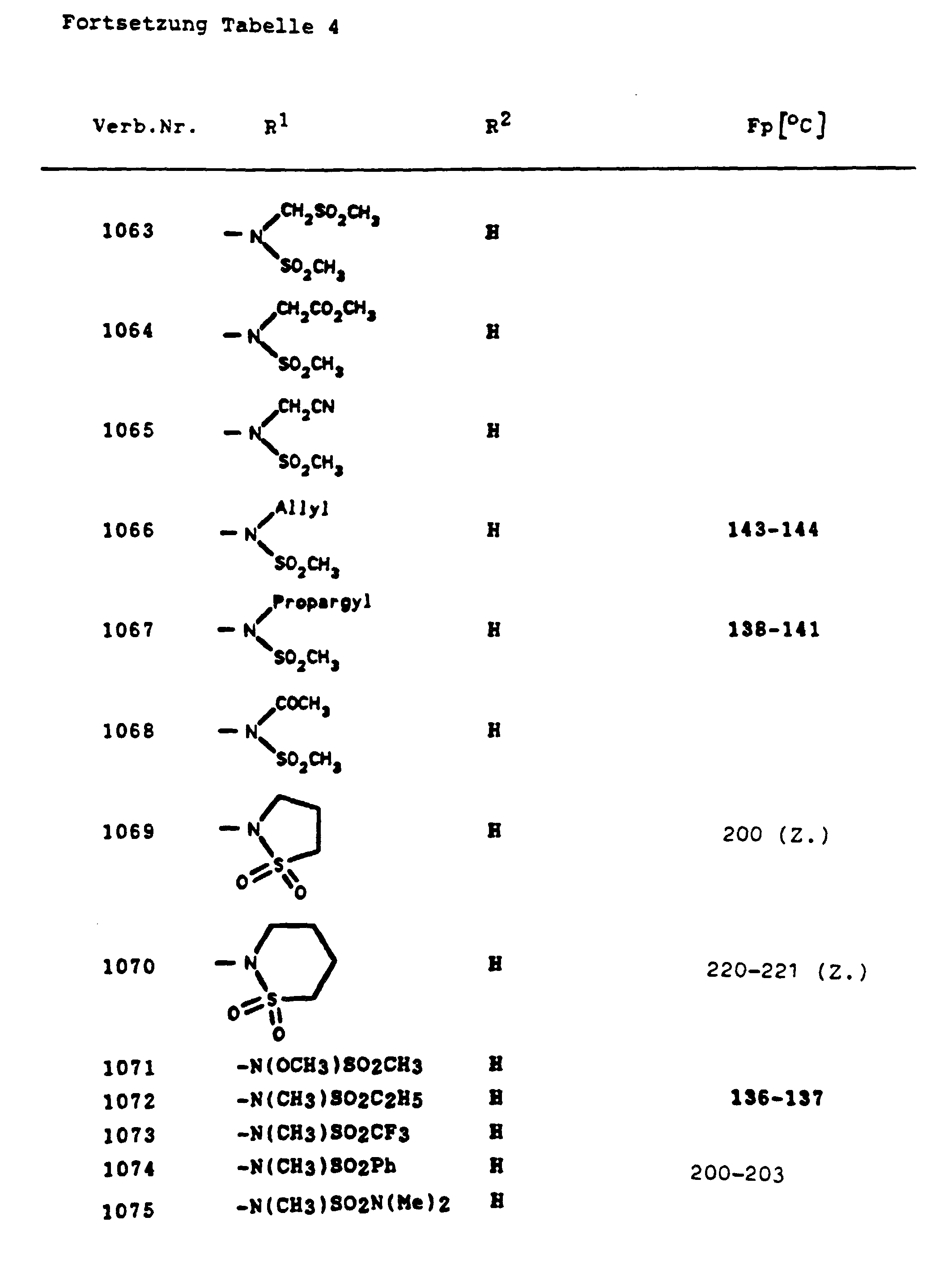

### B. FORMULIERUNGSBEISPIELE

a) Ein Stäubemittel wird erhalten, indem man 10 Gew.-Teile einer Verbindung der Formel (I) und 90 Gew.-Teile Talkum oder Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexan als Lösungsmittel und 10 Gew.-Teilen oxethyliertes Nonylphenol als Emulgator.
e) Ein in Wasser dispergierbares Granulat wird erhalten, indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-dinaphthylmethan-6,6'-disulfonsaures Natrium,
   2 Gewichtsteile oleolymethyltaurinsaures Natrium,
   1 Gewichtsteile Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.
g) Ein Extruder-Granulat erhält man, indem man 20 Gewichtsteile Wirkstoff, 3 Gewichtsteile ligininsulfonsaures Natrium, 1 Gewichtsteil Carboxymethylcellulose und 76 Gewichtsteile Kaolin vermischt, vermahlt und mit Wasser anfeuchtet. Dieses Gemisch wird extrudiert und anschließend in Luftstrom getrocknet.

### C. Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert. Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte zeigen, weisen die erfindungsgemäßen Verbindungen eine gute herbizide Vorauflaufwirksamkeit gegen ein breites Spektrum von Ungräsern und Unkräutern auf (vgl. Tabelle 5).

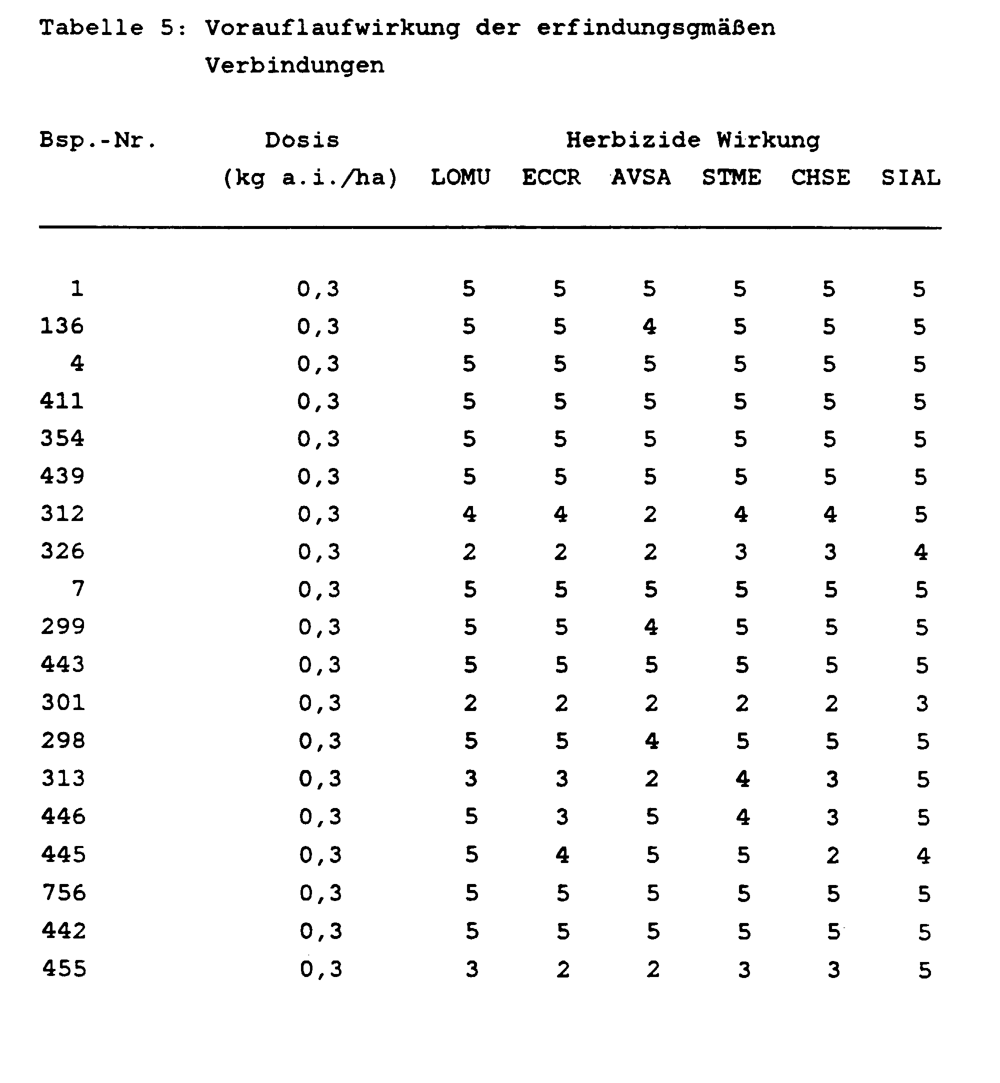

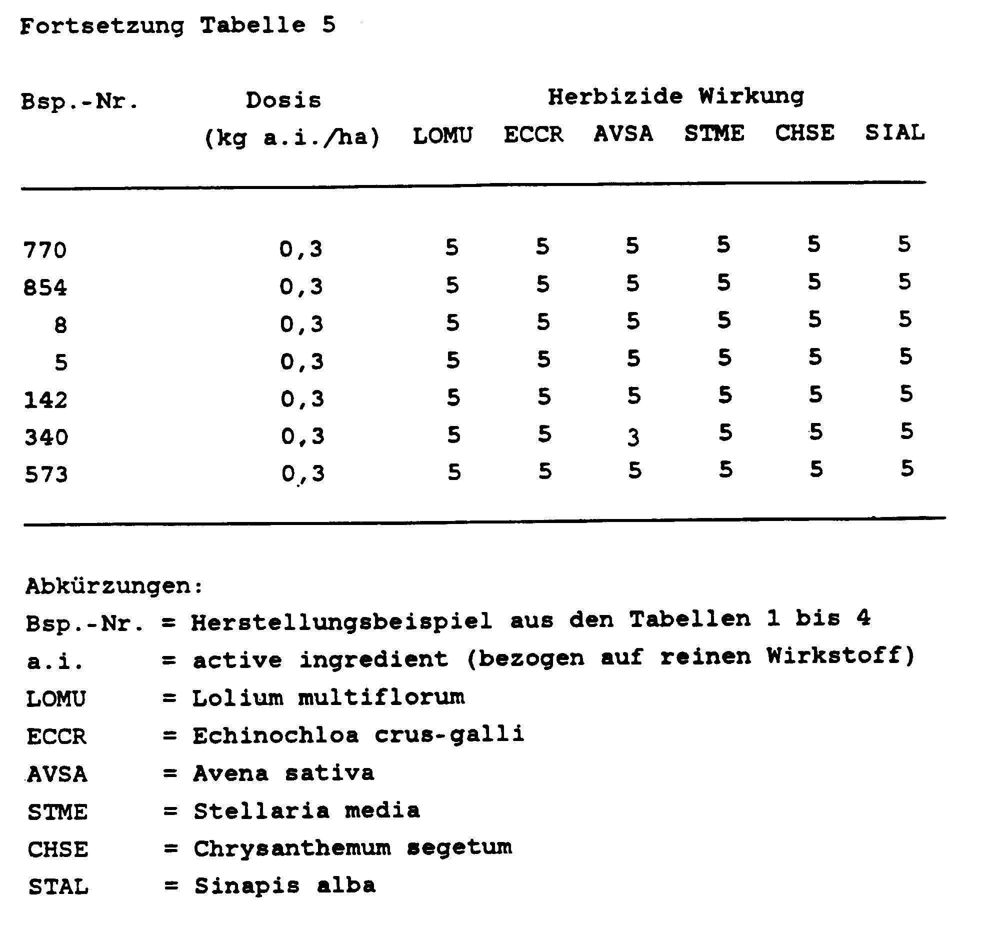

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (vgl. Tabelle 6).

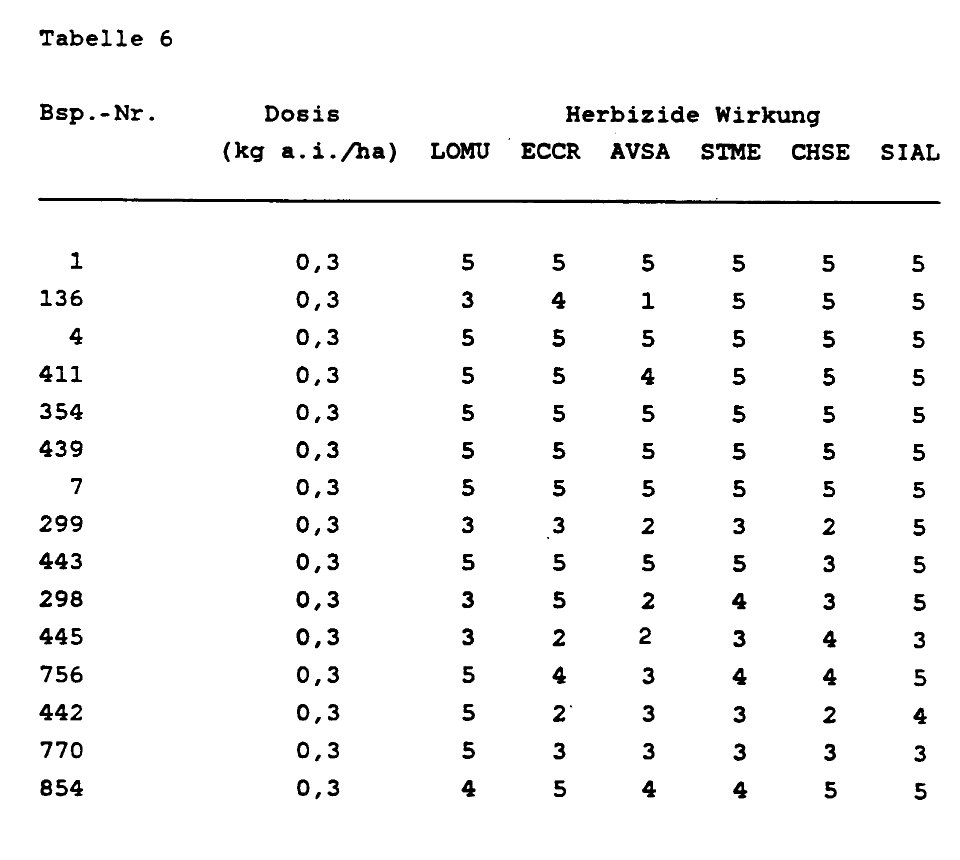

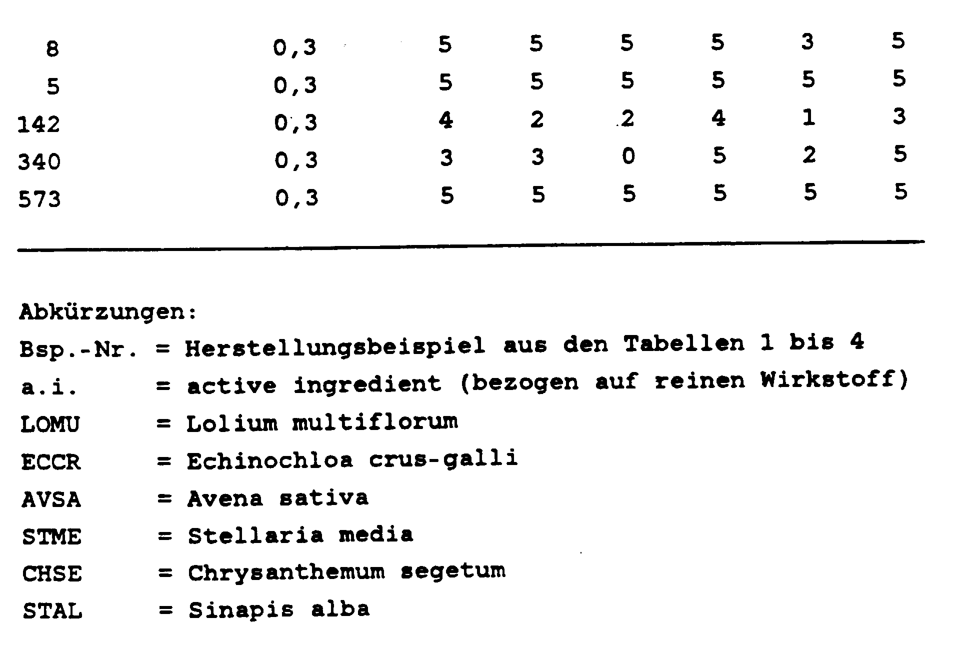

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschtem Pflanzenwuchs in landwirtschaftlichen Kulturen auf.

## Patentansprüche (Patentansprüche für folgende(n) Vertragsstaat(en): CH, DE, FR, GB, IT, LI)

1. Verbindungen der Formel (I) oder deren Salze, 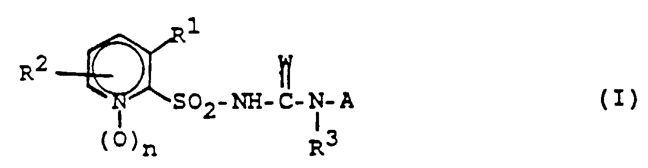 worin
R¹ -OSO₂NR⁴R⁵, -NR⁶R⁷ oder Iod,
R² H, (C₁-C₄)Alkyl, (C₁-C₃)Haloalkyl, Halogen, NO₂, CN, (C₁-C₃)Alkoxy, (C₁-C₃)Haloalkoxy, (C₁-C₃)Alkylthio, (C₁-C₃)Alkoxy-(C₁-C₃)alkyl, (C₁-C₃)Alkoxycarbonyl, (C₁-C₃)-Alkylsulfinyl, (C₁-C₃-Alkylsulfonyl, SO₂NR^{a}R^{b} oder C(O)NR^{a}R^{b},
R^{a}, R^{b} unabhängig voneinander H, (C₁-C₃)Alkyl, (C₃-C₄)-Alkenyl, Propargyl, oder zusammen -(CH₂)₄-, -(CH₂)₅- oder -CH₂CH₂OCH₂CH₂-
R³ H oder CH₃,
R⁴ H, (C₁-C₃)Alkyl, (C₃-C₄)Alkenyl, (C₁-C₃)Alkoxy oder (C₃-C₄)Alkinyl und
R⁵ H, (C₁-C₃)Alkyl, (C₃-C₄)Alkenyl oder (C₃-C₄)Alkinyl oder
R⁴ und R⁵ zusammen -(CH₂)₄-, -(CH₂)₅- oder -CH₂CH₂OCH₂CH₂-,
R⁶ H, (C₁-C₈)Alkyl, das unsubstituiert oder durch ein oder mehrere Reste aus der Gruppe Halogen, (C₁-C₄)Alkoxy, (C₁-C₄)Alkylthio, (C₁-C₄)Alkylsulfinyl, (C₁-C₄)-Alkylsulfonyl, (C₁-C₄-Alkoxy)carbonyl und CN substituiert ist, (C₃-C₆)Alkenyl, das unsubstituiert oder durch ein oder mehrere Halogenatome substituiert ist, (C₃-C₆)-Alkinyl, das unsubstituiert oder durch ein oder mehrere Halogenatome substituiert ist, (C₁-C₄)Alkylsulfonyl, das unsubstituiert oder durch ein oder mehrere Halogenaomte substituiert ist, Phenylsulfonyl, wobei der Phenylrest unsubstituiert oder durch ein oder mehrere Reste aus der Gruppe Halogen, (C₁-C₄)Alkyl und (C₁-C₄)Alkoxy substituiert ist, (C₁-C₄)Alkoxy oder (C₁-C₄-Alkyl)carbonyl, das unsubstituiert oder durch ein oder mehrere Halogenatome substituiert ist,
R⁷ (C₁-C₄)Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, (C₁-C₄)Alkyl und (C₁-C₄)Alkoxy substituiert ist, oder Di-[(C₁-C₄)alkyl]aminosulfonyl, oder
R⁶ und R⁷ bilden gemeinsam eine Kette der Formel -(CH₂)ₘ-SO₂-, wobei die Kette noch durch 1 bis 4 (C₁-C₃)Alkylreste substituiert sein kann und m 3 oder 4 bedeutet,
n Null oder 1,
W O oder S,
A ein Rest der Formel 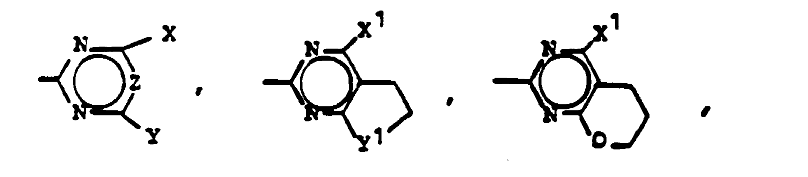 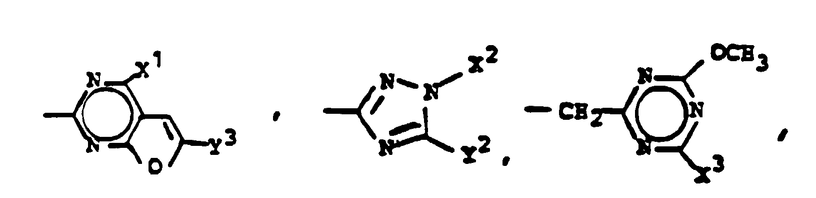 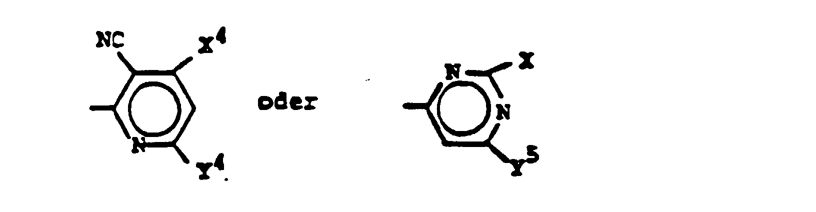
X H, Halogen, (C₁-C₃)Alkyl, (C₁-C₃)Alkoxy, wobei die beiden letztgenannten Reste unsubstituiert oder ein- oder mehrfach durch Halogen oder einfach durch (C₁-C₃)Alkoxy substituiert sind,
Y H, (C₁-C₃)Alkyl, (C₁-C₃)Alkoxy oder (C₁-C₃)Alkylthio, wobei die vorgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, ferner einen Rest der Formel NR⁸R⁹, (C₃-C₆)-Cycloalkyl, (C₂-C₄)Alkenyl, (C₂-C₄)Alkinyl, (C₃-C₄)-Alkenyloxy oder (C₃-C₄)Alkinyloxy,
Z CH oder N,
R⁸ und R⁹ unabhängig voneinander H, (C₁-C₃)Alkyl oder (C₃-C₄)Alkenyl,
X¹ CH₃, OCH₃, OC₂H₅ oder OCF₂H,
Y¹ -O- oder -CH₂-,
X² CH₃, C₂H₅ oder CH₂CF₃,
Y² OCH₃, OC₂H₅, SCH₃, SC₂H₅, CH₃ oder C₂H₅,
X³ CH₃ oder OCH₃,
Y³ H oder CH₃,
X⁴ CH₃, OCH₃, OC₂H₅, CH₂OCH₃ oder Cl,
Y⁴ CH₃, OCH₃, OC₂H₅ oder Cl,
Y⁵ CH₃, C₂H₅, OCH₃ oder Cl
bedeuten.

2. Verbindungen nach Anspruch 1, dadurch gekennzeichnet, daß
R⁴, R⁵ unabhängig voneinander (C₁-C₃)Alkyl, Allyl oder Propargyl oder
R⁴, R⁵ zusammen -(CH₂)₄-, -(CH₂)₅- oder -CH₂CH₂OCH₂CH₂-,
R⁶ H, (C₁-C₄)Alkyl, das unsubstituiert oder durch ein oder mehrere Halogenatome oder einen Rest aus der Gruppe (C₁-C₃)Alkoxy, (C₁-C₃)Alkylthio, (C₁-C₃)-Alkylsulfonyl, (C₁-C₄)Alkoxycarbonyl und CN substituiert ist, (C₃-C₄)Alkenyl, (C₃-C₄)Alkinyl, (C₁-C₄)-Alkylsulfonyl, Phenylsulfonyl, Phenylsulfonyl, das durch ein bis drei Reste aus der Gruppe Halogen, (C₁-C₃)Alkyl und (C₁-C₃)Alkoxy substituiert ist, (C₁-C₃)Alkoxy oder (C₁-C₄)Alkylcarbonyl,
R⁷ (C₁-C₄)Alkylsulfonyl, Phenylsulfonyl oder Phenylsulfonyl, das durch 1 bis 3 Reste aus der Gruppe Halogen, (C₁-C₃)Alkyl und (C₁-C₃)Alkoxy substituiert ist, oder Di- (C₁-C₄-alkyl)-aminosulfonyl oder
R⁶ und R⁷ gemeinsam eine Kette der Formel -(CH₂)ₘSO₂-, wobei m 3 oder 4 bedeutet,
bedeuten.

3. Verbindungen nach Anspruch 1 oder 2, dadurch gekennzeichnet, daß
W ein Sauerstoffatom,
n die Zahl Null und
A einen Rest der Formel 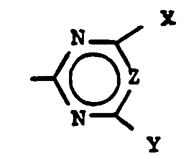
bedeuten.

4. Verbindungen nach einem oder mehreren der Ansprüche 1 bis 3, dadurch gekennzeichnet, daß
R² H, (C₁-C₃)Alkyl, (C₁-C₃)Alkoxy, Halogen oder (C₁-C₃)Alkylthio, R⁴ und R⁵ unabhängig voneinander (C₁-C₃)Alkyl, R⁶ Wasserstoff, (C₁-C₄)Alkyl oder (C₁-C₃)Alkylsulfonyl, R⁷ (C₁-C₃)Alkylsulfonyl und A einen Rest der Formel 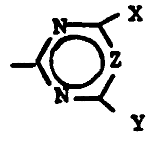 bedeuten,
worin Z CH oder N, X Halogen, (C₁-C₂)Alkyl, (C₁-C₂)Alkoxy, OCF₂H, CF₃ oder OCH₂CF₃ und Y(C₁-C₂)Alkyl, (C₁-C₂)Alkoxy oder OCF₂H sind.

5. Verfahren zur Herstellung von nach Anspruch 1 definierten Verbindungen der Formel (I) oder deren Salzen, dadurch gekennzeichnet, daß man
(a) eine Verbindung der Formel (II) 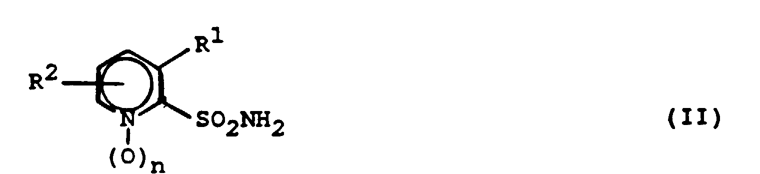 mit einem heterocyclischen Carbamat der Formel (III), 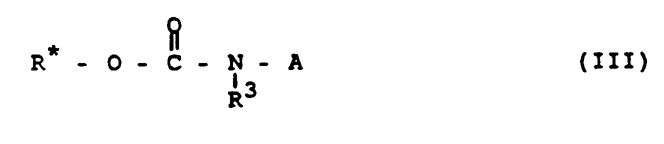 worin R* Phenyl oder (C₁-C₄)Alkyl bedeutet, umsetzt, oder
(b) ein Pyridylsulfonylcarbamat der Formel (IV) 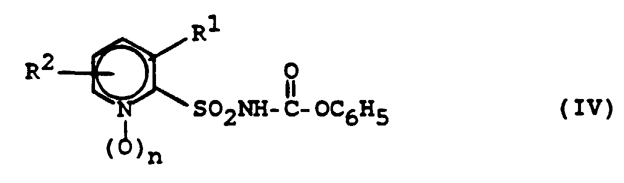 mit einem Aminoheterocyclus der Formel (V) 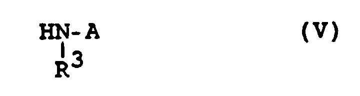 umsetzt oder
(c) ein Sulfonylisocyanat der Formel (VI) 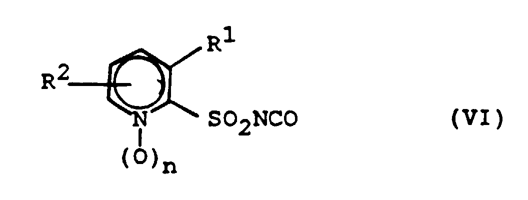 mit einem Aminoheterocyclus der Formel R³-NH-A (V) umsetzt oder
(d) in einer Eintopfreaktion zunächst einen Aminoheterocyclus der Formel R³-NH-A (V) in Gegenwart einer Base mit Phosgen umsetzt und das gebildete Intermediat mit einem Pyridinsulfonamid der Formel (II) umsetzt.

6. Herbizide oder pflanzenwachstumsregulierende Mittel, dadurch gekennzeichnet, daß sie nach einem oder mehreren der Ansprüche 1 bis 4 definierte Verbindungen der Formel (I) oder deren Salze und Formulierungshilfsmittel enthalten.

7. Verfahren zur Bekämpfung von unerwünschten Pflanzen oder zur Wachstumsregulierung von Pflanzen, dadurch gekennzeichnet, daß man eine herbizid wirksame bzw. pflanzenwachstumsregulierend wirksame Menge von nach einem oder mehreren der Ansprüche 1 bis 4 definierten Verbindungen der Formel (I) oder deren Salzen auf die Pflanzen, Pflanzensamen oder die Anbaufläche appliziert.

8. Verbindungen der Formel (II), 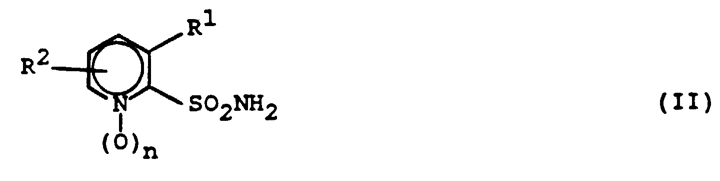 worin R¹, R² und n die nach einem oder mehreren der Ansprüche 1 bis 4 genannten Bedeutung haben.

9. Verfahren zur Herstellung einer Verbindung der Formel (II) nach Anspruch 8, dadurch gekennzeichnet, daß man ein Sulfochlorid der Formel 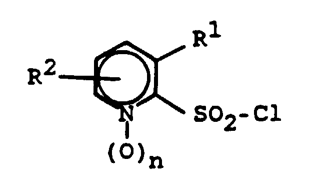 worin
R¹, R² und n die oben definierte Bedeutung haben, durch Umsetzung mit tert.-Butylamin und Abspaltung der tert.-Butylgruppe unter üblichen Bedingungen oder mit Ammoniak in ein Sulfonamid der Formel (II) überführt.

10. Verfahren nach Anspruch 9, dadurch gekennzeichnet, daß man das Sulfochlorid durch Umsetzung einer Verbindung der Formel 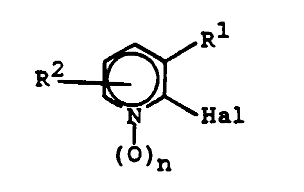 worin
Hal Fluor, Chlor, Brom oder Iod bedeutet sowie R¹, R² und n die oben definierten Bedeutungen haben, mit einem S-Nucleophil und anschließender Umsetzung des erhaltenen Zwischenprodukts mit Natriumhypochlorit oder Chlor herstellt.

## Patentansprüche (Patentansprüche für folgende(n) Vertragsstaat(en): ES)

1. Verwendung von Verbindungen der Formel (I) oder deren Salze 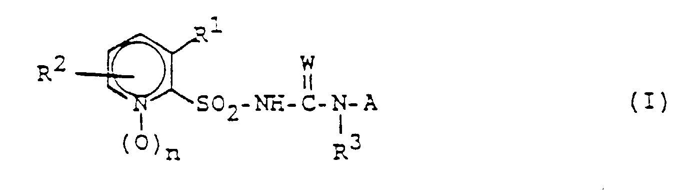 worin
R¹ -OSO₂NR⁴R⁵, -NR⁶R⁷ oder Iod,
R² H, (C₁-C₄)Alkyl, (C₁-C₃)Haloalkyl, Halogen, NO₂, CN, (C₁-C₃)Alkoxy, (C₁-C₃)Haloalkoxy, (C₁-C₃)Alkylthio, (C₁-C₃)Alkoxy-(C₁-C₃)alkyl, (C₁-C₃)Alkoxycarbonyl, (C₁-C₃)-Alkylsulfinyl, (C₁-C₃)Alkylsulfonyl, SO₂NR^{a}R^{b} oder C(O)NR^{a}R^{b}
R^{a}, R^{b} unabhängig voneinander H, (C₁-C₃)Alkyl, (C₃-C₄)-Alkenyl, Propargyl, oder zusammen -(CH₂)₄- , -(CH₂)₅- oder -CH₂CH₂OCH₂CH₂-
R³ H oder CH₃,
R⁴ H, (C₁-C₃)Alkyl, (C₃-C₄)Alkenyl, (C₁-C₃)Alkoxy oder (C₃-C₄)Alkinyl und
R⁵ H, (C₁-C₃)Alkyl, (C₃-C₄)Alkenyl oder (C₃-C₄)Alkinyl oder
R⁴ und R⁵ zusammen -(CH₂)₄-, -(CH₂)₅- oder -CH₂CH₂OCH₂CH₂-,
R⁶ H, (C₁-C₈)Alkyl, das unsubstituiert oder durch ein oder mehrere Reste aus der Gruppe Halogen, (C₁-C₄)Alkoxy, (C₁-C₄)Alkylthio, (C₁-C₄)Alkylsulfinyl, (C₁-C₄)-Alkylsulfonyl, (C₁-C₄-Alkoxy)carbonyl und CN substituiert ist, (C₃-C₆)Alkenyl, das unsubstituiert oder durch ein oder mehrere Halogenatome substituiert ist, (C₃-C₆)-Alkinyl, das unsubstituiert oder durch ein oder mehrere Halogenatome substituiert ist, (C₁-C₄)Alkylsulfonyl, das unsubstituiert oder durch ein oder mehrere Halogenaomte substituiert ist, Phenylsulfonyl, wobei der Phenylrest unsubstituiert oder durch ein oder mehrere Reste aus der Gruppe halogen, (C₁-C₄)Alkyl und (C₁-C₄)Alkoxy substituiert ist, (C₁-C₄)Alkoxy oder (C₁-C₄-Alkyl)carbonyl, das unsubstituiert oder durch ein oder mehrere Halogenatome substituiert ist,
R⁷ (C₁-C₄)Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, (C₁-C₄)Alkyl und (C₁-C₄)Alkoxy substituiert ist, oder Di-[(C₁-C₄)alkyl]aminosulfonyl, oder
R⁶ und R⁷ bilden gemeinsam eine Kette der Formel -(CH₂)ₘ-SO₂-, wobei die Kette noch durch 1 bis 4 (C₁-C₃)Alkylreste substituiert sein kann und m 3 oder 4 bedeutet,
n Null oder 1,
W O oder S,
A ein Rest der Formel 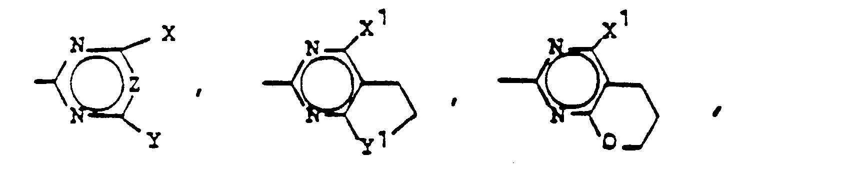 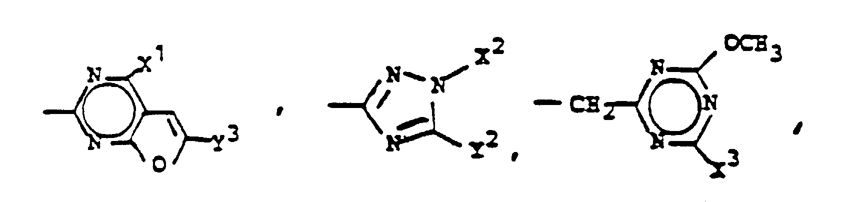 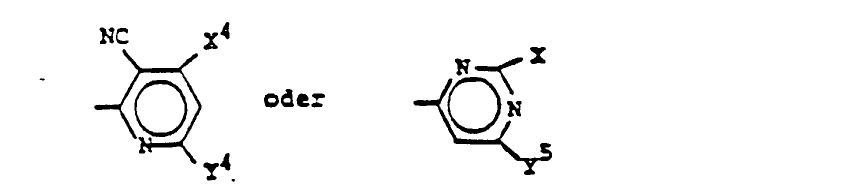
X H, Halogen, (C₁-C₃)Alkyl, (C₁-C₃)Alkoxy, wobei die beiden letztgenannten Reste unsubstituiert oder ein- oder mehrfach durch Halogen oder einfach durch (C₁-C₃)Alkoxy substituiert sind,
Y H, (C₁-C₃)Alkyl, (C₁-C₃)Alkoxy oder (C₁-C₃)Alkylthio, wobei die vorgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, ferner einen Rest der Formel NR⁸R⁹, (C₃-C₆)-Cycloalkyl, (C₂-C₄)Alkenyl, (C₂-C₄)Alkinyl, (C₃-C₄)-Alkenyloxy oder (C₃-C₄)Alkinyloxy,
Z CH oder N,
R⁸ und R⁹ unabhängig voneinander H, (C₁-C₃)Alkyl oder (C₃-C₄)Alkenyl,
X¹ CH₃, OCH₃, OC₂H₅ oder OCF₂H,
Y¹ -O- oder -CH₂-,
X² CH₃, C₂H₅ oder CH₂CF₃,
Y² OCH₃, OC₂H₅, SCH₃, SC₂H₅, CH₃ oder C₂H₅
X³ CH₃ oder OCH₃,
Y³ H oder CH₃,
X⁴ CH₃, OCH₃, OC₂H₅, CH₂OCH₃ oder Cl,
Y⁴ CH₃, OCH₃, OC₂H₅ oder Cl,
Y⁵ CH₃, C₂H₅, OCH₃ oder Cl
bedeuten, als Herbizide oder Pflanzenwachstumsregulatoren.

2. Verwendung gemäß Anspruch 1, dadurch gekennzeichnet, daß
R⁴, R⁵ unabhängig voneinander (C₁-C₃)Alkyl, Allyl oder Propargyl oder
R⁴, R⁵ zusammen - (CH₂)₄-, -(CH₂)₅- oder -CH₂CH₂OCH₂CH₂-,
R⁶ H, (C₁-C₄)Alkyl, das unsubstituiert oder durch ein oder mehrere Halogenatome oder einen Rest aus der Gruppe (C₁-C₃)Alkoxy, (C₁-C₃)Alkylthio, (C₁-C₃)-Alkylsulfonyl, (C₁-C₄)Alkoxycarbonyl und CN substituiert ist, (C₃-C₄)Alkenyl, (C₃-C₄)Alkinyl, (C₁-C₄)-Alkylsulfonyl, Phenylsulfonyl, Phenylsulfonyl, das durch ein bis drei Reste aus der Gruppe Halogen, (C₁-C₃)Alkyl und (C₁-C₃)Alkoxy substituiert ist, (C₁-C₃)Alkoxy oder (C₁-C₄)Alkylcarbonyl, :
R⁷ (C₁-C₄)Alkylsulfonyl, Phenylsulfonyl oder Phenylsulfonyl, das durch 1 bis 3 Reste aus der Gruppe Halogen, (C₁-C₃)Alkyl und (C₁-C₃)Alkoxy substituiert ist, oder Di-(C₁-C₄-alkyl)-aminosulfonyl oder
R⁶ und R⁷ gemeinsam eine Kette der Formel -(CH₂)ₘSO₂-, wobei m 3 oder 4 bedeutet,
bedeuten.

3. Verwendung nach Anspruch 1 oder 2, dadurch gekennzeichnet, daß
W ein Sauerstoffatom,
n die Zahl Null und
A einen Rest der Formel 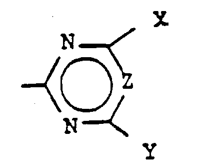
bedeuten.

4. Verwerdung nach einem der mehreren der Ansprüche 1 bis 3, dadurch gekennzeichnet, daß
R² H, (C₁-C₃)Alkyl, (C₁-C₃)Alkoxy, Halogen oder (C₁-C₂)Alkylthio, R⁴ und R⁵ unabhängig voneinander (C₁-C₃)Alkyl, R⁶ Wasserstoff, (C₁-C₄)Alkyl oder (C₁-C₃)Alkylsulfonyl, R⁷ (C₁-C₃)Alkylsulfonyl und A einen Rest der Formel 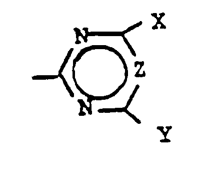 bedeuten,
worin Z CH oder N, X Halogen, (C₁-C₂)Alkyl, (C₁-C₂)Alkoxy; OCF₂H, CF₃ oder OCH₂CF₃ und Y (C₁-C₂)Alkyl, (C₁-C₂)Alkoxy oder OCF₂H sind.

5. Verfahren zur Herstellung von nach Anspruch 1 definierten Verbindungen der Formel (I) oder deren Salzen, dadurch gekennzeichnet, daß man
(a) eine Verbindung der Formel (II) 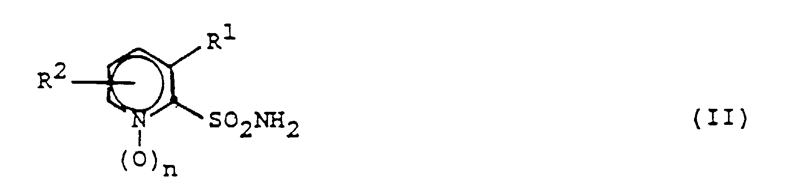 mit einem heterocyclischen Carbamat der Formel (III), 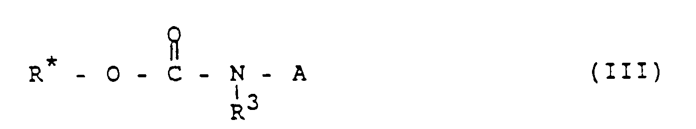 worin R* Phenyl oder (C₁-C₄)Alkyl bedeutet,
umsetzt, oder
(b) ein Pyridylsulfonylcarbamat der Formel (IV) 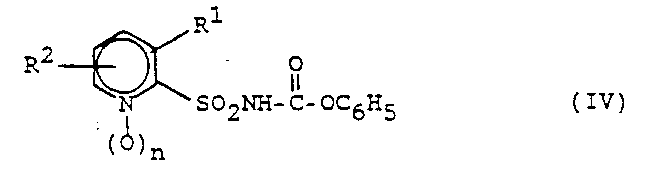 mit einem Aminoheterocyclus der Formel (V) 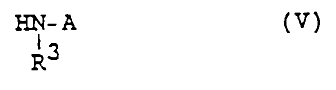 umsetzt oder
(c) ein Sulfonylisocyanat der Formel (VI) 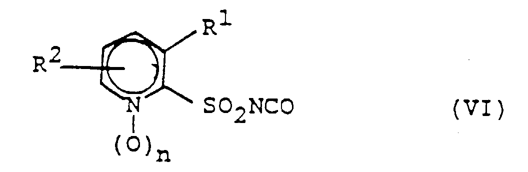 mit einem Aminoheterocyclus der Formel R³-NH-A (V) umsetzt oder
(d) in einer Eintopfreaktion zunächst einen Aminoheterocyclus der Formel R³-NH-A (V) in Gegenwart einer Base mit Phosgen umsetzt und das gebildete Intermediat mit einem Pyridinsulfonamid der Formel (II) umsetzt.

6. Herbizide oder pflanzenwachstumsregulierende Mittel, dadurch gekennzeichnet, daß sie nach einem oder mehreren der Ansprüche 1 bis 4 definierte Verbindungen der Formel (I) oder deren Salze und Formulierungshilfsmittel enthalten.

7. Verfahren zur Bekämpfung von unerwünschten Pflanzen oder zur Wachstumsregulierung von Pflanzen, dadurch gekennzeichnet, daß man eine herbizid wirksame bzw. pflanzenwachstumsregulierend wirksame Menge von nach einem oder mehreren der Ansprüche 1 bis 4 definierten Verbindungen der Formel (I) oder deren Salzen auf die Pflanzen, Pflanzensamen oder die Anbaufläche appliziert.

8. Verfahren zur Herstellung von Verbindungen der Formel (II), 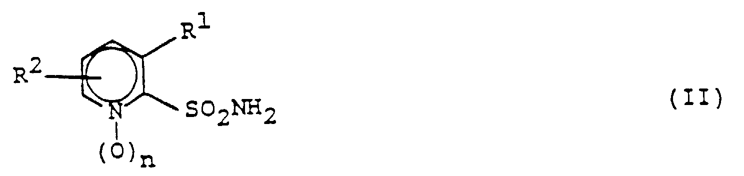 worin R¹, R² und n die in einem oder mehreren der Ansprüche 1 bis 4 definierte Bedeutung haben, dadurch gekennzeichnet, daß man ein Sulfochlorid der Formel 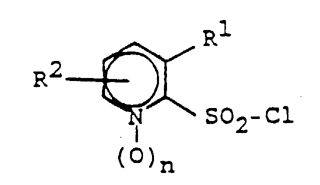 worin
R¹, R² und n die oben definierte Bedeutung haben, durch Umsetzung mit tert.-Butylamin und Abspaltung der tert.-Butylgruppe unter üblichen Bedingungen oder mit Ammoniak in ein Sulfonamid der Formel (II) überführt.

9. Verfahren nach Anspruch 8, dadurch gekennzeichnet, daß man das Sulfochlorid durch Umsetzung einer Verbindung der Formel 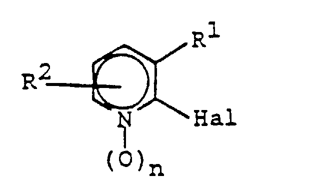 worin
Hal Fluor, Chlor, Brom oder Iod bedeutet sowie R¹, R² und n die oben definierten Bedeutungen haben, mit einem S-Nucleophil und anschließender Umsetzung des erhaltenen Zwischenprodukts mit Natriumhypochlorit oder Chlor herstellt.

## Claims (Claims for the following Contracting State(s): CH, DE, FR, GB, IT, LI)

1. A compound of the formula (I) or its salts 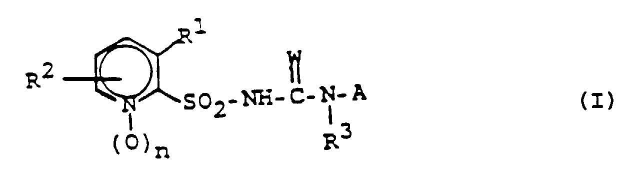 in which
R¹ is -OSO₂NR⁴R⁵, -NR⁶R⁷ or iodine,
R² is H, (C₁-C₄)alkyl, (C₁-C₃)haloalkyl, halogen, NO₂, CN, (C₁-C₃)alkoxy, (C₁-C₃)haloalkoxy, (C₁-C₃)alkylthio, (C₁-C₃)alkoxy-(C₁-C₃)alkyl, (C₁-C₃)alkoxycarbonyl, (C₁-C₃)alkylsulfinyl, (C₁-C₃)alkylsulfonyl, SO₂NR^{a}R^{b} or C(O)NR^{a}R^{b},
R^{a} and R^{b} independently of one another are H, (C₁-C₃)alkyl, (C₃-C₄)alkenyl, propargyl, or together are -(CH₂)₄-, -(CH₂)₅- or -CH₂CH₂OCH₂CH₂-,
R³ is H or CH₃,
R⁴ is H, (C₁-C₃)alkyl, (C₃-C₄)alkenyl, (C₁-C₃)alkoxy or (C₃-C₄)alkynyl, and
R⁵ is H, (C₁-C₃)alkyl, (C₃-C₄)alkenyl or (C₃-C₄)alkynyl, or
R⁴ and R⁵ together are -(CH₂)₄-, -(CH₂)₅- or -CH₂CH₂OCH₂CH₂-,
R⁶ is H, (C₁-C₈)alkyl, which is unsubstituted or substituted by one or more radicals from the group comprising halogen, (C₁-C₄)alkoxy, (C₁-C₄)alkylthio, (C₁-C₄)alkylsulfinyl, (C₁-C₄)alkylsulfonyl, (C₁-C₄)alkoxycarbonyl and CN, (C₃-C₆)alkenyl which is unsubstituted or substituted by one or more halogen atoms, (C₃-C₆)alkynyl which is unsubstituted or substituted by one or more halogen atoms, (C₁-C₄)alkylsulfonyl which is unsubstituted or substituted by one or more halogen atoms, phenylsulfonyl where the phenyl radical is unsubstituted or substituted by one or more radicals from the group comprising halogen, (C₁-C₄)alkyl and (C₁-C₄)alkoxy, (C₁-C₄)alkoxy or (C₁-C₄)alkylcarbonyl which is unsubstituted or substituted by one or more halogen atoms,
R⁷ is (C₁-C₄)alkylsulfonyl which is unsubstituted or substituted by one or more halogen atoms, phenylsulfonyl where the phenyl radical is unsubstituted or substituted by one or more radicals from the group comprising halogen, (C₁-C₄)alkyl and (C₁-C₄)alkoxy, or di-[(C₁-C₄)-alkyl] aminosulfonyl, or
R⁶ and R⁷ together form a chain of the formula -(CH₂)ₘ-SO₂-, where the chain can additionally be substituted by 1 to 4 (C₁-C₃)alkyl radicals and m is 3 or 4,
n is zero or 1,
W is O or S,
A is a radical of the formula 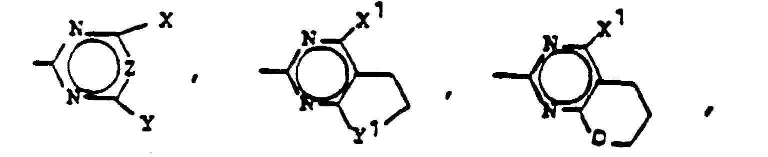 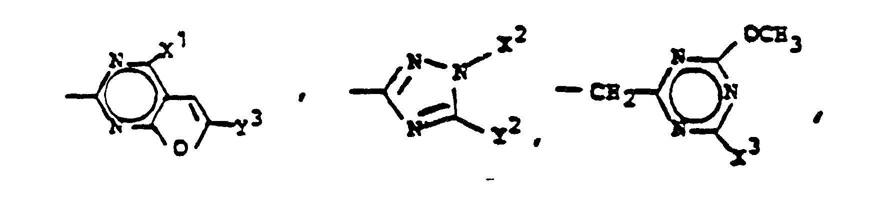 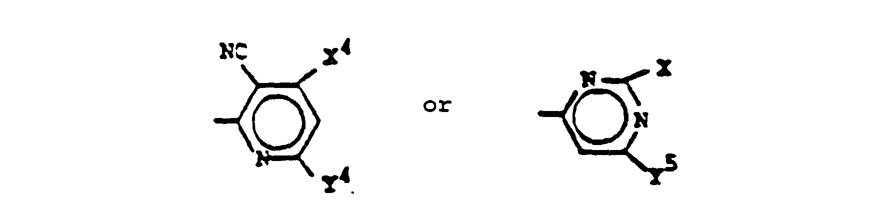
X is H, halogen, (C₁-C₃)alkyl, (C₁-C₃)alkoxy, where the two last-mentioned radicals are unsubstituted or monosubstituted or polysubstituted by halogen or monosubstituted by (C₁-C₃)alkoxy,
Y is H, (C₁-C₃)alkyl, (C₁-C₃)alkoxy or (C₁-C₃)alkylthio, where the abovementioned alkyl-containing radicals are unsubstituted or monosubstituted or polysubstituted by halogen or monosubstituted or disubstituted by (C₁-C₃)alkoxy or (C₁-C₃)alkylthio, or is a radical of the formula NR⁸R⁹, (C₃-C₆)-cycloalkyl, (C₂-C₄)alkenyl, (C₂-C₄)alkynyl, (C₃-C₄)alkenyloxy or (C₃-C₄)alkynyloxy,
Z is CH or N,
R⁸ and R⁹ independently of one another are H, (C₁-C₃)alkyl or (C₃-C₄)alkenyl,
X¹ is CH₃, OCH₃, OC₂H₅ or OCF₂H,
Y¹ is -O- or -OH₂-,
X² is CH₃, C₂H₅ or CH₂CF₃,
Y² is OCH₃, OC₂H₅, SCH₃, SC₂H₅, CH₃ or C₂H₅,
X³ is CH₃ or OCH₃,
Y³ is H or CH₃,
X⁴ is CH₃, OCH₃, OC₂H₅, CH₂OCH₃ or Cl.
Y⁴ is CH₃ , OCH₃ , OC₂H₅ or Cl,
Y⁵ is CH₃, C₂H₅, OCH₃ or Cl.

2. A compound as claimed in claim 1, wherein
R⁴ and R⁵ independently of one another are (C₁-C₃)alkyl, allyl or propargyl or
R⁴ and R⁵ together are -(CH₂)₄-, -(CH₂)₅- or -CH₂CH₂OCH₂CH₂-,
R⁶ is H, (C₁-C₄)alkyl which is unsubstituted or substituted by one or more halogen atoms or by a radical from the group comprising (C₁-C₃)alkoxy, (C₁-C₃)alkylthio, (C₁-C₃)alkylsulfonyl, (C₁-C₄)-alkoxycarbonyl and CN, (C₃-C₄)alkenyl, (C₃-C₄)alkynyl, (C₁-C₄)alkylsulfonyl, phenylsulfonyl, phenylsulfonyl which is substituted by one to three radicals from the group comprising halogen, (C₁-C₃)alkyl and (C₁-C₃)alkoxy, (C₁-C₃)alkoxy or (C₁-C₄)alkylcarbonyl,
R⁷ is (C₁-C₄)alkylsulfonyl, phenylsulfonyl or phenylsulfonyl which is substituted by 1 to 3 radicals from the group comprising halogen, (C₁-C₃)alkyl and (C₁-C₃)alkoxy, or di-(C₁-C₄-alkyl) -aminosulfonyl or
R⁶ and R⁷ together are a chain of the formula -(CH₂)ₘSO₂- where m is 3 or 4.

3. A compound as claimed in claim 1 or 2, wherein
W is an oxygen atom,
n is the number zero and
A is a radical of the formula

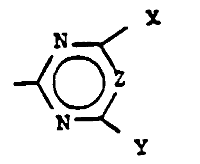

4. A compound as claimed in one or more of claims 1 to 3, wherein
R² is H, (C₁-C₃)alkyl, (C₁-C₃)alkoxy, halogen or (C₁-C₃)alkylthio, R⁴ and R⁵ independently of one another are (C₁-C₃)alkyl, R⁶ is hydrogen, (C₁-C₄)alkyl or (C₁-C₃)alkylsulfonyl, R⁷ is (C₁-C₃)alkylsulfonyl and A is a radical of the formula 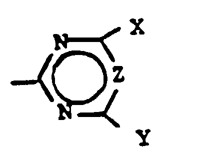 in which Z is CH or N, X is halogen, (C₁-C₂)alkyl, (C₁-C₂)alkoxy, OCF₂H, CF₃ or OCH₂CF₃ and Y is (C₁-C₂)alkyl, (C₁-C₂)alkoxy or OCF₂H.

5. A process for the preparation of a compound of the formula (I) or its salts as defined by claim 1, which comprises
(a) reacting a compound of the formula (II) 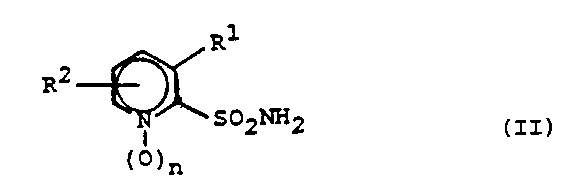 with a heterocyclic carbamate of the formula (III) 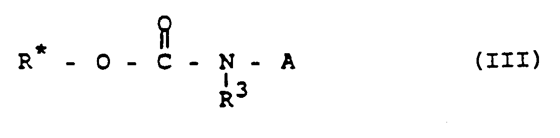 in which R* is phenyl or (C₁-C₄)alkyl, or
(b) reacting a pyridylsulfonylcarbamate of the formula (IV) 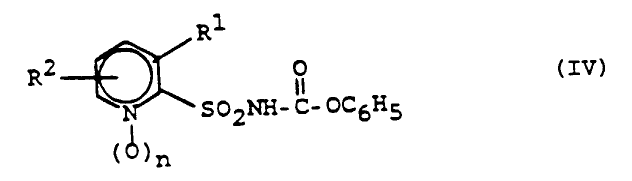 with an aminoheterocycle of the formula (V) 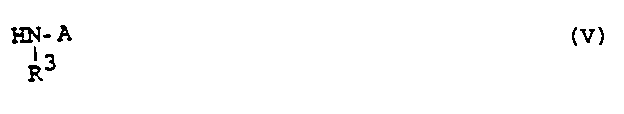 or
(c) reacting a sulfonyl isocyanate of the formula (VI) 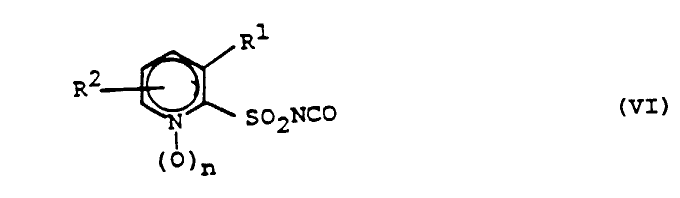 with an aminoheterocycle of the formula R³-NH-A (V) or
(d) first reacting an aminoheterocycle of the formula R³-NH-A (V) in a one-pot reaction with phosgene in the presence of a base and reacting the intermediate formed with a pyridinesulfonamide of the formula (II).

6. A herbicide or plant growth-regulating agent, which contains a compound of the formula (I) or its salts as defined by one or more of claims 1 to 4 and formulation auxiliaries.

7. A method of combating undesired plants or of regulating the growth of plants, wherein an amount of a compound of the formula (I) or its salts as defined by one or more of claims 1 to 4, which is herbicidally effective or effective for regulating the growth of plants, is applied to the plants, plant seed or the cultivated area.

8. A compound of the formula (II) 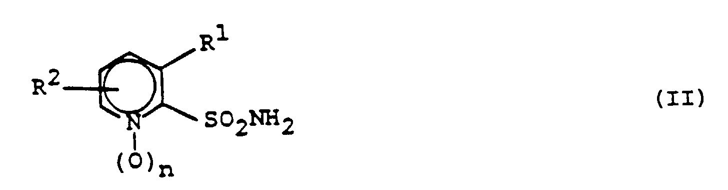 in which R¹, R² and n have the meaning mentioned in one or more of claims 1 to 4.

9. A process for the preparation of a compound of the formula (II) as claimed in claim 8, which comprises converting a sulfochloride of the formula 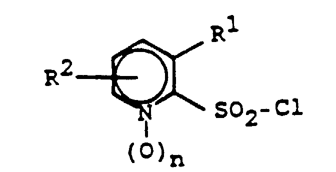 in which
R¹, R² and n have the meaning defined above, into a sulfonamide of the formula (II) by reaction with tert.-butylamine and removal of the tert.-butyl group under customary conditions, or by reaction with ammonia.

10. The process as claimed in claim 9, wherein the sulfochloride is prepared by reaction of a compound of the formula 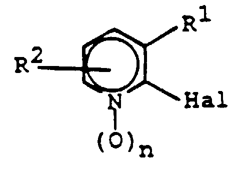 in which
Hal is fluorine, chlorine, bromine or iodine and R¹, R² and n have the meanings defined above, with an S-nucleophile and subsequent reaction of the resulting intermediate with sodium hypochlorite or chlorine.

## Claims (Claims for the following Contracting State(s): ES)

1. The use of a compound of the formula (I) or its salts 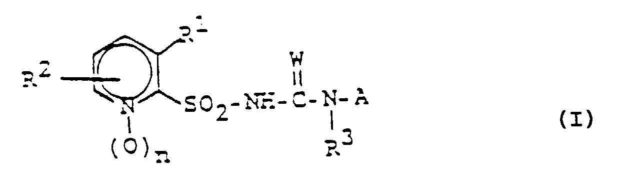 in which
R¹ is -OSO₂NR⁴R⁵, -NR⁶R⁷ or iodine,
R² is H, (C₁-C₄)alkyl, (C₁-C₃)haloalkyl, halogen, NO₂, CN, (C₁-C₃)alkoxy, (C₁-C₃)haloalkoxy, (C₁-C₃)alkylthio, (C₁-C₃)alkoxy-(C₁-C₃)alkyl, (C₁-C₃)alkoxycarbonyl, (C₁-C₃)alkylsulfinyl, (C₁-C₃)alkylsulfonyl, SO₂NR^{a}R^{b} or C(O)NR^{a}R^{b},
R^{a} and R^{b} independently of one another are H, (C₁-C₃)alkyl, (C₃-C₄)alkenyl, propargyl, or together are -(CH₂)₄-, -(CH₂)₅- or -CH₂CH₂OCH₂CH₂-,
R³ is H or CH₃,
R⁴ is H, (C₁-C₃)alkyl, (C₃-C₄)alkenyl, (C₁-C₃)alkoxy or (C₃-C₄)alkynyl, and
R⁵ is H, (C₁-C₃)alkyl, (C₃-C₄)alkenyl or (C₃-C₄)alkynyl, or
R⁴ and R⁵ together are -(CH₂)₄-, -(CH₂)₅- or -CH₂CH₂OCH₂CH₂-,
R⁶ is H, (C₁-C₈)alkyl, which is unsubstituted or substituted by one or more radicals from the group comprising halogen, (C₁-C₄)alkoxy, (C₁-C₄)alkylthio, (C₁-C₄)alkylsulfinyl, (C₁-C₄)alkylsulfonyl, (C₁-C₄)alkoxycarbonyl and CN, (C₃-C₆)alkenyl which is unsubstituted or substituted by one or more halogen atoms, (C₃-C₆)alkynyl which is unsubstituted or substituted by one or more halogen atoms, (C₁-C₄)alkylsulfonyl which is unsubstituted or substituted by one or more halogen atoms, phenylsulfonyl where the phenyl radical is unsubstituted or substituted by one or more radicals from the group comprising halogen, (C₁-C₄)alkyl and (C₁-C₄)alkoxy, (C₁-C₄)alkoxy or (C₁-C₄)alkylcarbonyl which is unsubstituted or substituted by one or more halogen atoms,
R⁷ is (C₁-C₄)alkylsulfonyl which is unsubstituted or substituted by one or more halogen atoms, phenylsulfonyl where the phenyl radical is unsubstituted or substituted by one or more radicals from the group comprising halogen, (C₁-C₄)alkyl and (C₁-C₄)alkoxy, or di-[(C₁-C₄)-alkyl]aminosulfonyl, or
R⁶ and R⁷ together form a chain of the formula -(CH₂)ₘ-SO₂-, where the chain can additionally be substituted by 1 to 4 (C₁-C₃)alkyl radicals and m is 3 or 4,
n is zero or 1,
W is O or S,
A is a radical of the formula 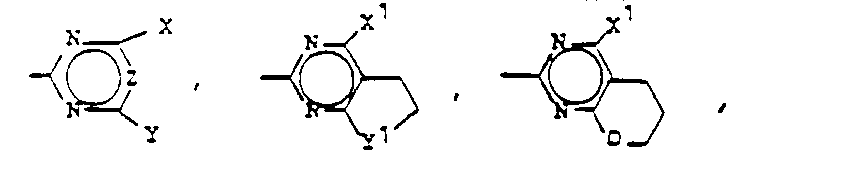 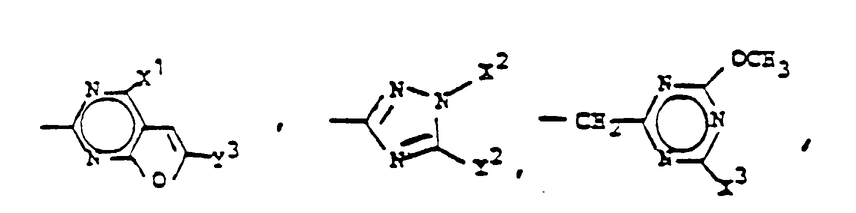 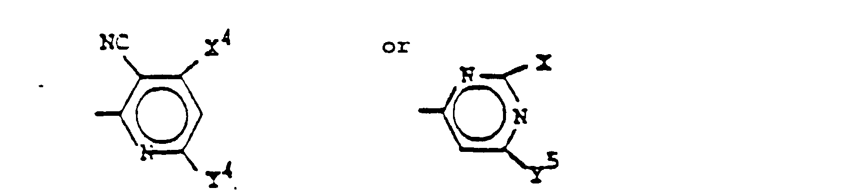
X is H, halogen, (C₁-C₃)alkyl, (C₁-C₃)alkoxy, where the two last-mentioned radicals are unsubstituted or monosubstituted or polysubstituted by halogen or monosubstituted by (C₁-C₃)alkoxy,
Y is H, (C₁-C₃)alkyl, (C₁-C₃)alkoxy or (C₁-C₃)alkylthio, where the abovementioned alkyl-containing radicals are unsubstituted or monosubstituted or polysubstituted by halogen or monosubstituted or disubstituted by (C₁-C₃)alkoxy or (C₁-C₃)alkylthio, or is a radical of the formula NR⁸R⁹, (C₃-C₆)-cycloalkyl, (C₂-C₄)alkenyl, (C₂-C₄)alkynyl, (C₃-C₄)alkenyloxy or (C₃-C₄)alkynyloxy,
Z is CH or N,
R⁸ and R⁹ independently of one another are H, (C₁-C₃)alkyl or (C₃-C₄)alkenyl,
X¹ is CH₃, OCH₃, OC₂H₅ or OCF₂H,
Y¹ is -O- or -CH₂-,
X² is CH₃, C₂H₅ or CH₂CH₃,
Y² is OCH₃, OC₂H₅, SCH₃, SC₂H₅, CH₃ or C₂H₅,
X³ is CH₃ or OCH₃,
Y³ is H or CH₃,
X⁴ is CH₃, OCH₃, OC₂H₅, CH₂OCH₃ or Cl,
Y⁴ is CH₃, OCH₃, OC₂H₅ or Cl,
Y⁵ is CH₃, C₂H₅, OCH₃ or Cl,
as a herbicide or plant growth regulator.

2. The use as claimed in claim 1, wherein
R⁴ and R⁵ independently of one another are (C₁-C₃)alkyl, allyl or propargyl or
R⁴ and R⁵ together are -(CH₂)₄-, -(CH₂)₅- or -CH₂CH₂OCH₂CH₂-,
R⁶ is H, (C₁-C₄)alkyl which is unsubstituted or substituted by one or more halogen atoms or by a radical from the group comprising (C₁-C₃)alkoxy, (C₁-C₃)alkylthio, (C₁-C₃)alkylsulfonyl, (C₁-C₄)-alkoxycarbonyl and CN, (C₃-C₄)alkenyl, (C₃-C₄)alkynyl, (C₁-C₄)alkylsulfonyl, phenylsulfonyl, phenylsulfonyl which is substituted by one to three radicals from the group comprising halogen, (C₁-C₃)alkyl and (C₁-C₃)alkoxy, (C₁-C₃)alkoxy or (C₁-C₄)alkylcarbonyl,
R⁷ is (C₁-C₄)alkylsulfonyl, phenylsulfonyl or phenylsulfonyl which is substituted by 1 to 3 radicals from the group comprising halogen, (C₁-C₃)alkyl and (C₁-C₃)alkoxy, or di-(C₁-C₄-alkyl) -aminosulfonyl or
R⁶ and R⁷ together are a chain of the formula -(CH₂)ₘSO₂- where m is 3 or 4.

3. The use as claimed in claim 1 or 2, wherein
W is an oxygen atom,
n is the number zero and
A is a radical of the formula

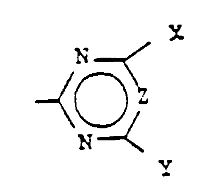

4. The use as claimed in one or more of claims 1 to 3, wherein
R² is H, (C₁-C₃)alkyl, (C₁-C₃)alkoxy, halogen or (C₁-C₃)alkylthio, R⁴ and R⁵ independently of one another are (C₁-C₃)alkyl, R⁶ is hydrogen, (C₁-C₄)alkyl or (C₁-C₃)alkylsulfonyl, R⁷ is (C₁-C₃)alkylsulfonyl and A is a radical of the formula 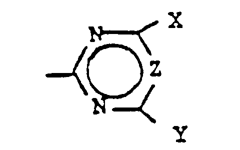 in which Z is CH or N, X is halogen, (C₁-C₂)alkyl, (C₁-C₂)alkoxy, OCF₂H, CF₃ or OCH₂CF₃ and Y is (C₁-C₂)alkyl, (C₁-C₂)alkoxy or OCF₂H.

5. A process for the preparation of a compound of the formula (I) or its salts as defined by claim 1, which comprises
(a) reacting a compound of the formula (II) 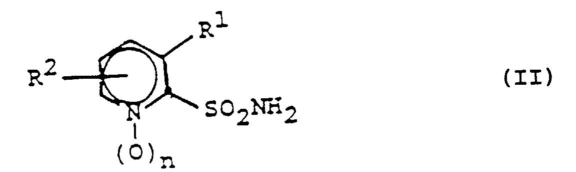 with a heterocyclic carbamate of the formula (III) 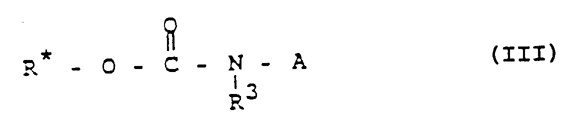 in which R* is phenyl or (C₁-C₄)alkyl, or
(b) reacting a pyridylsulfonylcarbamate of the formula (IV) 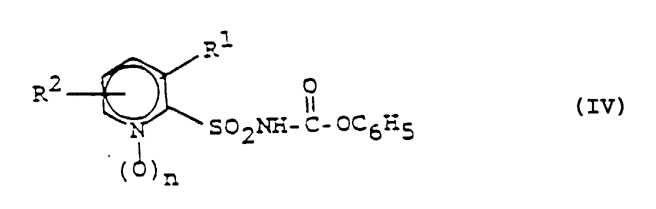 with an aminoheterocycle of the formula (V) 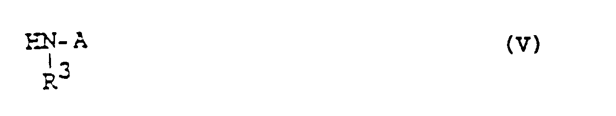 or
(c) reacting a sulfonyl isocyanate of the formula (VI) 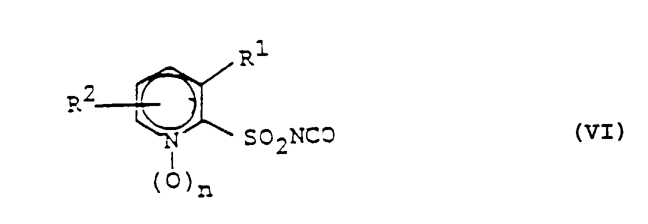 with an aminoheterocycle of the formula R³-NH-A (V) or
(d) first reacting an aminoheterocycle of the formula R³-NH-A (V) in a one-pot reaction with phosgene in the presence of a base and reacting the intermediate formed with a pyridinesulfonamide of the formula (II).

6. A herbicide or plant growth-regulating agent, which contains a compound of the formula (I) or its salts as defined by one or more of claims 1 to 4 and formulation auxiliaries.

7. A method of combating undesired plants or of regulating the growth of plants, wherein an amount of a compound of the formula (I) or its salts as defined by one or more of claims 1 to 4, which is herbicidally effective or effective for regulating the growth of plants, is applied to the plants, plant seed or the cultivated area.

8. A process for the preparation of a compound of the formula (II) 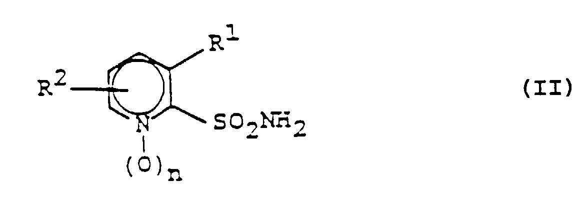 in which R¹, R² and n have the meaning defined in one or more of claims 1 to 4, which comprises converting a sulfochloride of the formula 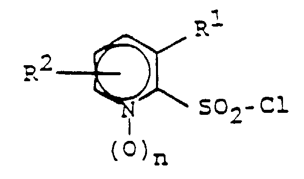 in which
R¹, R² and n have the meaning defined above, into a sulfonamide of the formula (II) by reaction with tert.-butylamine and removal of the tert.-butyl group under customary conditions, or by reaction with ammonia.

9. The process as claimed in claim 8, wherein the sulfochloride is prepared by reaction of a compound of the formula 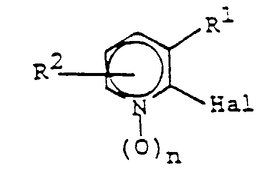 in which
Hal is fluorine, chlorine, bromine or iodine and R¹, R² and n have the meanings defined above, with an S-nucleophile and subsequent reaction of the resulting intermediate with sodium hypochlorite or chlorine.

## Revendications (Revendications pour l'(les) Etat(s) contractant(s) suivant(s): CH, DE, FR, GB, IT, LI)

1. Composés de formule (I) ou leurs sels 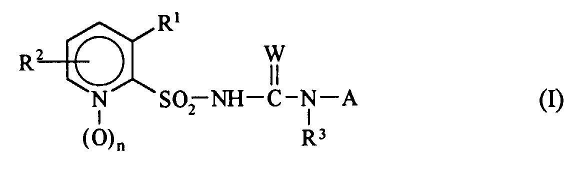 dans laquelle
R¹ est un reste -OSO₂NR⁴R⁵, -NR⁶R⁷ ou iodo,
R² représente un atome d'hydrogène ou un reste alkyle en C₁-C₄, halogénoalkyle en C₁-C₃, halogéno, NO₂, CN, alcoxy en C₁-C₃, halogénoalcoxy en C₁-C₃, alkylthio en C₁-C₃, (alcoxy en C₁-C₃)-(alkyle en C₁-C₃), (alcoxy en C₁-C₃)carbonyle, alkylsulfinyle en C₁-C₃, alkylsulfonyle en C₁-C₃, SO₂NR^{a}R^{b} ou C(O)NR^{a}R^{b},
R^{a}, R^{b} représentent indépendamment l'un de l'autre un atome d'hydrogène ou un reste alkyle en C₁-C₃, alcényle en C₃-C₄ ou propargyle, ou forment ensemble un reste -(CH₂)₄-, -(CH₂)₅- ou -CH₂CH₂OCH₂CH₂-,
R³ représente un atome d'hydrogène ou CH₃,
R⁴ est un atome d'hydrogène ou un reste alkyle en C₁-C₃, alcényle en C₃-C₄, alcoxy en C₁-C₃ ou alcynyle en C₃-C₄, et
R⁵ est un atome d'hydrogène ou un reste alkyle en C₁-C₃, alcényle en C₃-C₄ ou alcynyle en C₃-C₄, ou
R⁴ et R⁵ forment ensemble un reste -(CH₂)₄-, -(CH₂)₅- ou -CH₂CH₂OCH₂CH₂-,
R⁶ est un atome d'hydrogène, un reste alkyle en C₁-C₈ non substitué ou substitué par un ou plusieurs restes choisis dans le groupe constitué par les restes halogéno, alcoxy en C₁-C₄, alkylthio en C₁-C₄, alkylsulfinyle en C₁-C₄, alkylsulfonyle en C₁-C₄, (alcoxy en C₁-C₄)carbonyle et CN, un reste alcényle en C₃-C₆ non substitué ou substitué par un ou plusieurs atomes d'halogène, un reste alcynyle en C₃-C₆ non substitué ou substitué par un ou plusieurs atomes d'halogène, un reste alkylsulfonyle en C₁-C₄ non substitué ou substitué par un ou plusieurs atomes d'halogène, un reste phénylsulfonyle dont le reste phényle est non substitué ou substitué par un ou plusieurs restes choisis dans le groupe constitué par les restes halogéno, alkyle en C₁-C₄ et alcoxy en C₁-C₄, un reste alcoxy en C₁-C₄ ou un reste (alkyl en C₁-C₄)carbonyle non substitué ou substitué par un ou plusieurs atomes d'halogène,
R⁷ représente un reste alkylsulfonyle en C₁-C₄ non substitué ou substitué par un ou plusieurs atomes d'halogène, un reste phénylsulfonyle dont le reste phényle est non substitué ou substitué par un ou plusieurs restes choisis dans le groupe constitué par les restes halogéno, alkyle en C₁-C₄ et alcoxy en C₁-C₄, ou un reste di(alkyl en C₁-C₄)aminosulfonyle, ou bien
R⁶ et R⁷ forment ensemble une chaîne de formule -(CH₂)ₘ-SO₂-, cette chaîne pouvant encore être substituée par 1 à 4 restes alkyle en C₁-C₃, et m est 3 ou 4,
n est égal à 0 ou 1,
W est O ou S,
A représente un reste de formule 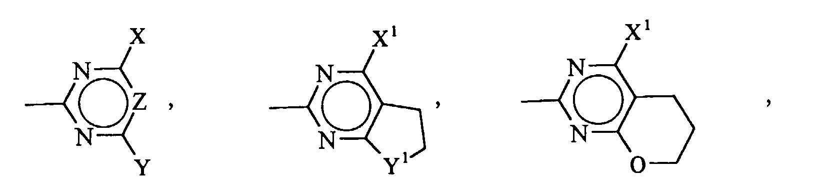 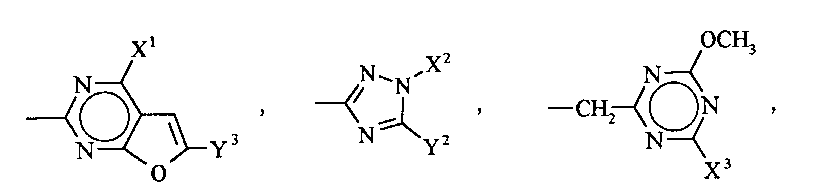 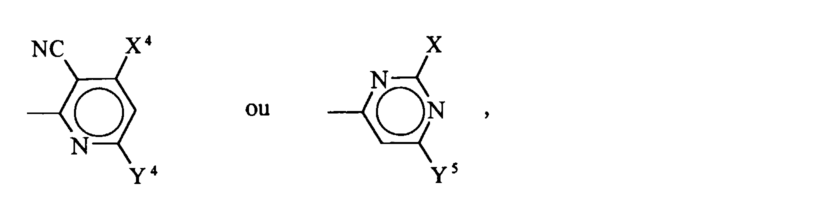
X est un atome d'hydrogène ou d'halogène ou un reste alkyle en C₁-C₃ ou alcoxy en C₁-C₃, ces deux derniers restes étant non substitués ou substitués une ou plusieurs fois par halogène ou une fois par un alcoxy en C₁-C₃,
Y est un atome d'hydrogène ou un reste alkyle en C₁-C₃, alcoxy en C₁-C₃ ou alkylthio en C₁-C₃, les restes alkylés précités étant non substitués ou substitués une ou plusieurs fois par halogène ou une ou deux fois par alcoxy en C₁-C₃ ou alkylthio en C₁-C₃, ou encore un reste de formule NR⁸R⁹ ou un reste cycloalkyle en C₃-C₆, alcényle en C₂-C₄, alcynyle en C₂-C₄, alcényloxy en C₃-C₄ ou alcynyloxy en C₃-C₄,
Z est CH ou N,
R⁸ et R⁹ représentent indépendamment l'un de l'autre un atome d'hydrogène ou un reste alkyle en C₁-C₃ ou alcényle en C₃-C₄,
X¹ est CH₃, OCH₃, OC₂H₅ ou OCF₂H,
Y¹ est -O- ou -CH₂-,
X² est CH₃, C₂H₅ ou CH₂CF₃
Y² est OCH₃, OC₂H₅, SCH₃, SC₂H₅, CH₃ ou C₂H₅,
X³ est CH₃ ou OCH₃,
Y³ est H Ou CH₃,
X⁴ est CH₃, OCH₃, OC₂H₅, CH₂OCH₃ ou Cl,
Y⁴ est CH₃, OCH₃, OC₂H₅ ou Cl,
Y⁵ est CH₃, C₂H₅, OCH₃ ou Cl.

2. Composés selon la revendication 1, caractérisés en ce que
R⁴ et R⁵ sont indépendamment l'un de l'autre un reste alkyle en C₁-C₃, allyle ou propargyle, ou
R⁴ et R⁵ forment ensemble un reste -(CH₂)₄-, -(CH₂)₅- ou -CH₂CH₂OCH₂CH₂-,
R⁶ représente un atome d'hydrogène ou un reste alkyle en C₁-C₄ non substitué ou substitué par un ou plusieurs atomes d'halogène ou par un reste choisi dans le groupe constitué par les restes alcoxy en C₁-C₃, alkylthio en C₁-C₃, alkylsulfonyle en C₁-C₃, (alcoxy en C₁-C₄)carbonyle et CN, un reste alcényle en C₃-C₄, un reste alcynyle en C₃-C₄, un reste alkylsulfonyle en C₁-C₄, un reste phénylsulfonyle ou phénylsulfonyle substitué par un à trois restes choisis dans le groupe constitué par les restes halogéno, alkyle en C₁-C₃ et alcoxy en C₁-C₃, un reste alcoxy en C₁-C₃ ou un reste (alkyl en C₁-C₄)carbonyle,
R⁷ représente un reste alkylsulfonyle en C₁-C₄, un reste phénylsulfonyle ou phénylsulfonyle substitué par 1 à 3 restes choisis dans le groupe constitué par les restes halogéno, alkyle en C₁-C₃ et alcoxy en C₁-C₃, ou un reste di(alkyl en C₁-C₄)aminosulfonyle, ou bien
R⁶ et R⁷ forment ensemble une chaîne de formule -(CH₂)ₘ-SO₂-, où m est 3 ou 4.

3. Composés selon la revendication 1 ou 2, caractérisés en ce que
W est un atome d'oxygène,
n est égal à 0 et
A est un reste de formule

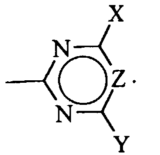

4. Composés selon l'une ou plusieurs des revendications 1 à 3, caractérisés en ce que R² est un atome d'hydrogène ou un reste alkyle en C₁-C₃, alcoxy en C₁-C₃, halogéno ou alkylthio en C₁-C₃, R⁴ et R⁵ sont indépendamment l'un de l'autre un reste alkyle en C₁-C₃, R⁶ est un atome d'hydrogène ou un reste alkyle en C₁-C₄ ou alkvlsulfonvle en C₁-C₃, R⁷ est un reste alkylsulfonyle en C₁-C₃ et A est un reste de formule 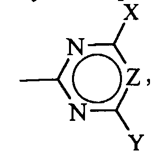 dans lequel Z est CH ou N, X est un atome d'halogène ou un reste alkyle en C₁-C₂, alcoxy en C₁-C₂, OCF₂H, CF₃ ou OCH₂CF₃ et Y est un reste alkyle en C₁-C₂, alcoxy en C₁-C₂ ou OCF₂H.

5. Procédé de préparation de composés de formule (I) définis selon la revendication 1 ou de leurs sels, caractérisé en ce que
(a) on fait réagir un composé de formule (II) 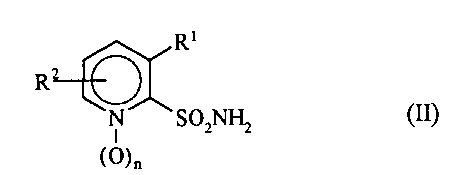 avec un carbamate hétérocyclique de formule (III) 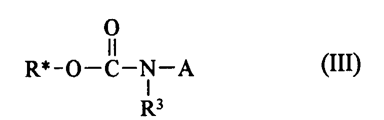 dans laquelle R* représente un reste phényle ou alkyle en C₁-C₄, ou
(b) on fait réagir un carbamate de pyridylsulfonyle de formule (IV) 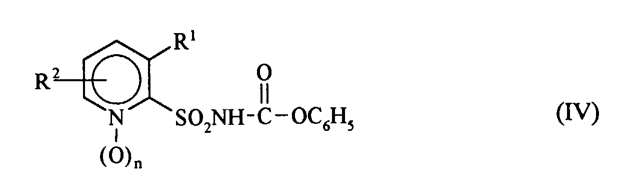 avec un aminohétérocycle de formule (V) 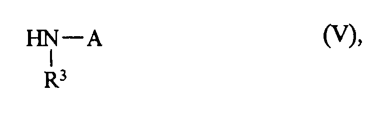 ou
(c) on fait réagir un sulfonylisocyanate de formule (VI) 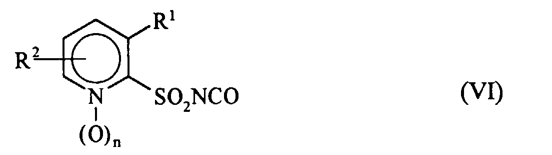 avec un aminohétérocycle de formule R³-NH-A (V), ou
(d) dans une réaction en un seul récipient, on fait d'abord réagir un aminohétérocycle de formule R³-NH-A (V) avec du phosgène en présence d'une base, et on fait réagir l'intermédiaire formé avec un pyridinesulfonamide de formule (II).

6. Compositions herbicides ou régulatrices de croissance des plantes, caractérisées en ce qu'elles contiennent des composés de formule (I) définis selon l'une ou plusieurs des revendications 1 à 4 ou leurs sels et des agents auxiliaires de formulation.

7. Procédé de lutte contre les plantes indésirables ou procédé de régulation de la croissance des plantes, caractérisé en ce que l'on applique sur les plantes, sur les semences des plantes ou sur la surface cultivée une quantité efficace comme herbicide ou une quantité efficace pour la régulation de la croissance des plantes de composés de formule (I) définis selon l'une ou plusieurs des revendications 1 à 4 ou de leurs sels.

8. Composés de formule (II) 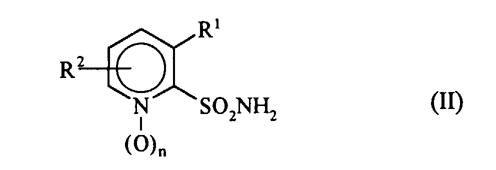 dans laquelle R¹, R² et n ont la signification indiquée dans l'une ou plusieurs des revendications 1 à 4.

9. Procédé de préparation d'un composé de formule (II) selon la revendication 8, caractérisé en ce que l'on transforme un sulfochlorure de formule 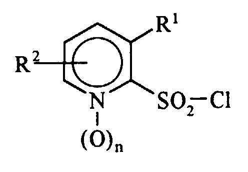 dans laquelle R¹, R² et n ont la signification définie ci-dessus, en un sulfonamide de formule (II) par réaction avec de la tert-butylamine et séparation du groupe tert-butyle dans les conditions habituelles, ou par réaction avec de l'ammoniac.

10. Procédé selon la revendication 9, caractérisé en ce que l'on prépare le sulfochlorure par réaction d'un composé de formule 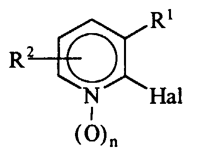 dans laquelle Hal représente un atome de fluor, de chlore, de brome ou d'iode, et R¹, R² et n ont les significations définies ci-dessus, avec un composé S-nucléophile, puis réaction du produit intermédiaire obtenu avec de l'hypochlorite de sodium ou du chlore.

## Revendications (Revendications pour l'(les) Etat(s) contractant(s) suivant(s): ES)

1. Utilisation comme herbicides ou régulateurs de croissance des plantes de composés de formule (I) ou de leurs sels 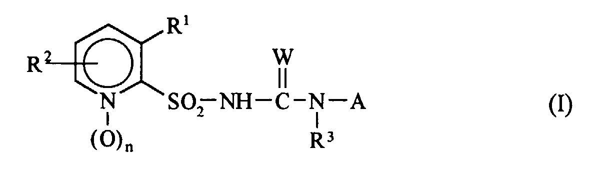 dans laquelle
R¹ est un reste -OSO₂NR⁴R⁵, -NR⁶R⁷ ou iodo,
R² représente un atome d'hydrogène ou un reste alkyle en C₁-C₄, halogénoalkyle en C₁-C₃, halogéno, NO₂, CN, alcoxy en C₁-C₃, halogénoalcoxy en C₁-C₃, alkylthio en C₁-C₃, (alcoxy en C₁-C₃)-(alkyle en C₁-C₃), (alcoxy en C₁-C₃)carbonyle, alkylsulfinyle en C₁-C₃, alkylsulfonyle en C₁-C₃, SO₂NR^{a}R^{b} ou C(O)NR^{a}R^{b},
R^{a}, R^{b} représentent indépendamment l'un de l'autre un atome d'hydrogène ou un reste alkyle en C₁-C₃, alcényle en C₃-C₄ ou propargyle, ou forment ensemble un reste -(CH₂)₄-, -(CH₂)₅- ou -CH₂CH₂OCH₂CH₂-,
R³ représente un atome d'hydrogène ou CH₃,
R⁴ est un atome d'hydrogène ou un reste alkyle en C₁-C₃, alcényle en C₃-C₄, alcoxy en C₁-C₃ ou alcynyle en C₃-C₄, et
R⁵ est un atome d'hydrogène ou un reste alkyle en C₁-C₃, alcényle en C₃-C₄ ou alcynyle en C₃-C₄, ou
R⁴ et R⁵ forment ensemble un reste -(CH₂)₄-, -(CH₂)₅- ou -CH₂CH₂OCH₂CH₂-,
R⁶ est un atome d'hydrogène, un reste alkyle en C₁-C₈ non substitué ou substitué par un ou plusieurs restes choisis dans le groupe constitué par les restes halogéno, alcoxy en C₁-C₄, alkylthio en C₁-C₄, alkylsulfinyle en C₁-C₄, alkylsulfonyle en C₁-C₄, (alcoxy en C₁-C₄)carbonyle et CN, un reste alcényle en C₃-C₆ non substitué ou substitué par un ou plusieurs atomes d'halogène, un reste alcynyle en C₃-C₆ non substitué ou substitué par un ou plusieurs atomes d'halogène, un reste alkylsulfonyle en C₁-C₄ non substitué ou substitué par un ou plusieurs atomes d'halogène, un reste phénylsulfonyle dont le reste phényle est non substitué ou substitué par un ou plusieurs restes choisis dans le groupe constitué par les restes halogéno, alkyle en C₁-C₄ et alcoxy en C₁-C₄, un reste alcoxy en C₁-C₄ ou un reste (alkyl en C₁-C₄)carbonyle non substitué ou substitué par un ou plusieurs atomes d'halogène,
R⁷ représente un reste alkylsulfonyle en C₁-C₄ non substitué ou substitué par un ou plusieurs atomes d'halogène, un reste phénylsulfonyle dont le reste phényle est non substitué ou substitué par un ou plusieurs restes choisis dans le groupe constitué par les restes halogéno, alkyle en C₁-C₄ et alcoxy en C₁-C₄, ou un reste di(alkyl en C₁-C₄)aminosulfonyle, ou bien
R⁶ et R⁷ forment ensemble une chaîne de formule -(CH₂)ₘ-SO₂-, cette chaîne pouvant encore être substituée par à 4 restes alkyle en C₁-C₃, et m est 3 ou 4,
n est égal à 0 ou 1,
W est O ou S,
A représente un reste de formule 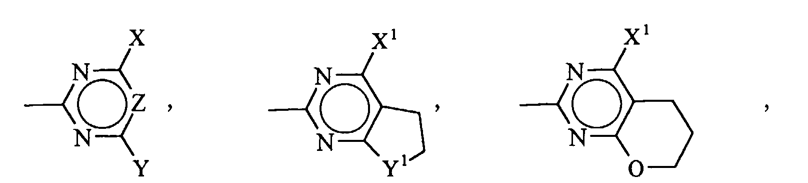 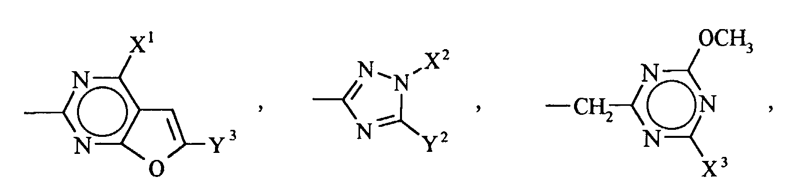 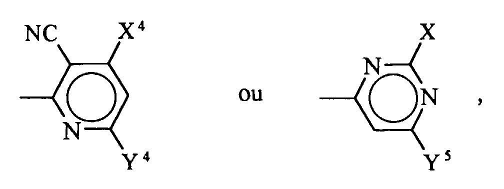
X est un atome d'hydrogène ou d'halogène ou un reste alkyle en C₁-C₃ ou alcoxy en C₁-C₃, ces deux derniers restes étant non substitués ou substitués une ou plusieurs fois par halogène ou une fois par un alcoxy en C₁-C₃,
Y est un atome d'hydrogène ou un reste alkyle en C₁-C₃, alcoxy en C₁-C₃ ou alkylthio en C₁-C₃, les restes alkylés précités étant non substitués ou substitués une ou plusieurs fois par halogène ou une ou deux fois par alcoxy en C₁-C₃ ou alkylthio en C₁-C₃, ou encore un reste de formule NR⁸R⁹ ou un reste cycloalkyle en C₃-C₆, alcényle en C₂-C₄, alcynyle en C₂-C₄, alcényloxy en C₃-C₄ ou alcynyloxy en C₃-C₄,
Z est CH ou N,
R⁸ et R⁹ représentent indépendamment l'un de l'autre un atome d'hydrogène ou un reste alkyle en C₁-C₃ ou alcényle en C₃-C₄,
X¹ est CH₃, OCH₃, OC₂H₅ ou OCF₂H,
Y¹ est -O- ou -CH₂-,
X² est CH₃, C₂H₅ ou CH₂CF₃
Y² est OCH₃, OC₂H₅, SCH₃, SC₂H₅, CH₃ ou C₂H₅,
X³ est CH₃ ou OCH₃,
Y³ est H ou CH₃,
X⁴ est CH₃, OCH₃, OC₂H₅, CH₂OCH₃ ou Cl,
Y⁴ est CH₃, OCH₃, OC₂H₅ ou Cl,
Y⁵ est CH₃, C₂H₅, OCH₃ ou Cl.

2. Utilisation selon la revendication 1, caractérisée en ce que
R⁴ et R⁵ sont indépendamment l'un de l'autre un reste alkyle en C₁-C₃, allyle ou propargyle, ou
R⁴ et R⁵ forment ensemble un reste -(CH₂)₄-, -(CH₂)₅- ou -CH₂CH₂OCH₂CH₂-,
R⁶ représente un atome d'hydrogène ou un reste alkyle en C₁-C₄ non substitué ou substitué par un ou plusieurs atomes d'halogène ou par un reste choisi dans le groupe constitué par les restes alcoxy en C₁-C₃, alkylthio en C₁-C₃, alkylsulfonyle en C₁-C₃, (alcoxy en C₁-C₄)carbonyle et CN, un reste alcényle en C₃-C₄, un reste alcynyle en C₃-C₄, un reste alkylsulfonyle en C₁-C₄, un reste phénylsulfonyle ou phénylsulfonyle substitué par un à trois restes choisis dans le groupe constitué par les restes halogéno, alkyle en C₁-C₃ et alcoxy en C₁-C₃, un reste alcoxy en C₁-C₃ ou un reste (alkyl en C₁-C₄)carbonyle,
R⁷ représente un reste alkylsulfonyle en C₁-C₄, un reste phénylsulfonyle ou phénylsulfonyle substitué par 1 à 3 restes choisis dans le groupe constitué par les restes halogéno, alkyle en C₁-C₃ et alcoxy en C₁-C₃, ou un reste di(alkyl en C₁-C₄)aminosulfonyle, ou bien
R⁶ et R⁷ forment ensemble une chaîne de formule -(CH₂)ₘ-SO₂-, où m est 3 ou 4,

3. Utilisation selon la revendication 1 ou 2, caractérisée en ce que
W est un atome d'oxygène,
n est égal à 0 et
A est un reste de formule

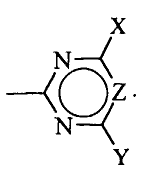

4. Utilisation selon l'une ou plusieurs des revendications 1 à 3, caractérisée en ce que R² est un atome d'hydrogène ou un reste alkyle en C₁-C₃, alcoxy en C₁-C₃, halogéno ou alkylthio en C₁-C₃, R⁴ et R⁵ sont indépendamment l'un de l'autre un reste alkyle en C₁-C₃, R⁶ est un atome d'hydrogène ou un reste alkyle en C₁-C₄ ou alkylsulfonyle en C₁-C₃, R⁷ est un reste alkvlsulfonvle en C₁-C₃ et A est un reste de formule 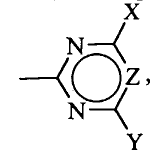 dans lequel Z est CH ou N, X est un atome d'halogène ou un reste alkyle en C₁-C₂, alcoxy en C₁-C₂, OCF₂H, CF₃ ou OCH₂CF₃ et Y est un reste alkyle en C₁-C₂, alcoxy en C₁-C₂ ou OCF₂H.

5. Procédé de préparation de composés de formule (I) définis selon la revendication 1 ou de leurs sels, caractérisé en ce que
(a) on fait réagir un composé de formule (II) 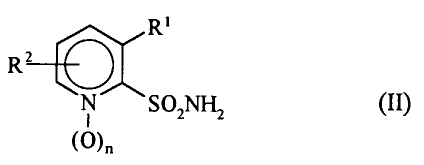 avec un carbamate hétérocyclique de formule (III) 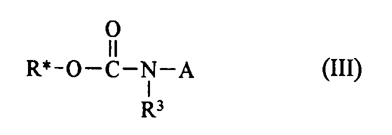 dans laquelle R* représente un reste phényle ou alkyle en C₁-C₄, ou
(b) on fait réagir un carbamate de pyridylsulfonyle de formule (IV) 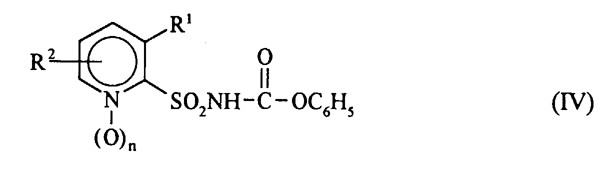 avec un aminohétérocycle de formule (V) 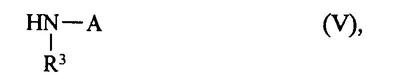 ou
(c) on fait réagir un sulfonylisocyanate de formule (VI) 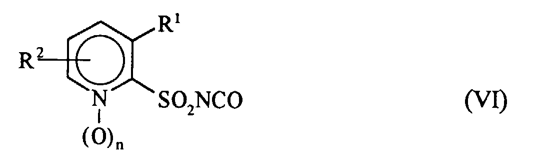 avec un aminohétérocycle de formule R³-NH-A (V), ou
(d) dans une réaction en un seul récipient, on fait d'abord réagir un aminohétérocycle de formule R³-NH-A (V) avec du phosgène en présence d'une base, et on fait réagir l'intermédiaire formé avec un pyridinesulfonamide de formule (II).

6. Compositions herbicides ou régulatrices de croissance des plantes, caractérisées en ce qu'elles contiennent des composés de formule (I) définis selon une ou plusieurs des revendications 1 à 4 ou leurs sels et des agents auxiliaires de formulation.

7. Procédé de lutte contre les plantes indésirables ou procédé de régulation de la croissance des plantes, caractérisé en ce que l'on applique sur les plantes, sur les semences des plantes ou sur la surface cultivée une quantité efficace comme herbicide ou une quantité efficace pour la régulation de la croissance des plantes de composés de formule (I) définis selon l'une ou plusieurs des revendications 1 à 4 ou de leurs sels.

8. Procédé de préparation d'un composé de formule (II) 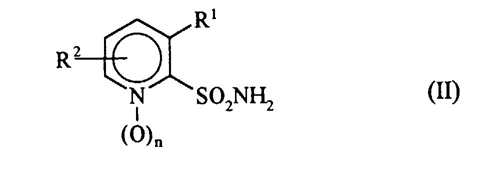 dans laquelle R¹, R² et n ont la signification indiquée dans l'une ou plusieurs des revendications 1 à 4, caractérisé en ce que l'on transforme un sulfochlorure de formule 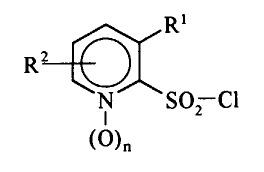 dans laquelle R¹, R² et n ont la signification définie ci-dessus, en un sulfonamide de formule (II) par réaction avec de la tert-butylamine et séparation du groupe tert-butyle dans les conditions habituelles, ou par réaction avec de l'ammoniac.

9. Procédé selon la revendication 8, caractérisé en ce que l'on prépare le sulfochlorure par réaction d'un composé de formule 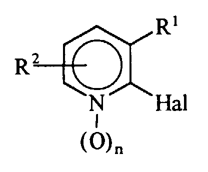 dans laquelle Hal représente un atome de fluor, de chlore, de brome ou d'iode, et R¹, R² et n ont les significations définies ci-dessus, avec un composé S-nucléophile, puis réaction du produit intermédiaire obtenu avec de l'hypochlorite de sodium ou du chlore.
